# EUROPEAN PATENT SPECIFICATION

(11) **EP 3 529 371 B1**
(45) Date of publication and mention of the grant of the patent: **22.06.2022**
(21) Application number: 17794256.2
(22) Date of filing: 19.10.2017
(51) Int. Cl.: C12Q 1/37, C07K 14/47, G01N 33/50, C07K 14/33, C12N 9/52

(54) **A FUNCTIONAL DETECTION ASSAY DEVOID OF ANTIBODIES FOR SEROTYPING OF BOTULINUM NEUROTOXINS**
FUNKTIONELLER DETEKTIONSTEST FREI VON ANTIKÖRPERN ZUR SEROTYPISIERUNG VON BOTULINUM-NEUROTOXINEN
DOSAGE DE DÉTECTION FONCTIONNELLE DÉPOURVU D'ANTICORPS, POUR LE SÉROTYPAGE DE NEUROTOXINES BOTULIQUES

(30) Priority: 21.10.2016 EP 16195083
(43) Date of publication of application: 28.08.2019
(73) Proprietor: Toxogen GmbH, 30625 Hannover (DE)
(72) Inventor: RUMMEL, Andreas, 30171 Hannover (DE); WEISEMANN, Jasmin, 30826 Garbsen (DE)
(74) Representative: Wilk, Thomas
(86) International application number: PCT/EP2017/076762
(87) International publication number: WO 2018/073370

(56) References cited:
- US-A1- 2003 143 651
- US-A1- 2006 063 222
- STEFAN SIKORRA ET AL: "Substrate Recognition Mechanism of VAMP/Synaptobrevin-cleaving Clostridial Neurotoxins", JOURNAL OF BIOLOGICAL CHEMISTRY, vol. 283, no. 30, 25 July 2008 (2008-07-25), pages 21145-21152, XP055441234, US ISSN: 0021-9258, DOI: 10.1074/jbc.M800610200
- STEFAN SIKORRA ET AL: "Substrate Recognition Mechanism of VAMP/Synaptobrevin-cleaving Clostridial Neurotoxins", JOURNAL OF BIOLOGICAL CHEMISTRY, vol. 283, no. 30, 25 July 2008 (2008-07-25), pages 21145-21152, XP055441234, US ISSN: 0021-9258, DOI: 10.1074/jbc.M800610200
- SHARMA SHASHI K ET AL: "Detection of type A, B, E, and F Clostridium botulinum neurotoxins in foods by using an amplified enzyme-linked immunosorbent assay with digoxigenin-labeled antibodies", APPLIED AND ENVIRONMENTAL MICROBIOLOGY, vol. 72, no. 2, February 2006 (2006-02), pages 1231-1238, ISSN: 0099-2240(print)

### SEQUENCE LISTING

<110> Toxogen GmbH
<120> A functional detection assay devoid of antibodies for serotyping of botulinum neurotoxins
<130> 757-8 PCT
<150> EP 16 195 083.7
   <151> 2016-10-21
<160> 76
<170> PatentIn version 3.5
<210> 1
   <211> 61
   <212> PRT
   <213> Artificial Sequence
<220>
   <223> H6tHAtev1S(146-206)LS D179KA199R
<400> 1
   Met Asp Glu Asn Leu Glu Gln Val Ser Gly Ile Ile Gly Asn Leu Arg
   1               5                   10                  15
   His Met Ala Leu Asp Met Gly Asn Glu Ile Asp Thr Gln Asn Arg Gln
               20                  25                  30
   Ile Lys Arg Ile Met Glu Lys Ala Asp Ser Asn Lys Thr Arg Ile Asp
           35                  40                  45
   Glu Ala Asn Gln Arg Arg Thr Lys Met Leu Gly Ser Gly
       50                  55                  60
<210> 2
   <211> 35
   <212> PRT
   <213> Artificial Sequence
<220>
   <223> H6tHAtev 1V(60-94)
<400> 2
   Leu Ser Glu Leu Asp Asp Arg Ala Asp Ala Leu Gln Ala Gly Ala Ser
   1               5                   10                  15
   Gln Phe Glu Thr Ser Ala Ala Lys Leu Lys Arg Lys Tyr Trp Trp Lys
               20                  25                  30
   Asn Leu Lys
           35
<210> 3
   <211> 35
   <212> PRT
   <213> Artificial Sequence
<220>
   <223> H6tHAtev 1V(60-94)L T79S
<400> 3
   Leu Ser Glu Leu Asp Asp Arg Ala Asp Ala Leu Gln Ala Gly Ala Ser
   1               5                   10                  15
   Gln Phe Glu Ser Ser Ala Ala Lys Leu Lys Arg Lys Tyr Trp Trp Lys
               20                  25                  30
   Asn Leu Lys
           35
<210> 4
   <211> 61
   <212> PRT
   <213> Artificial Sequence
<220>
   <223> H6tHA1S(146-206)LS D179KN196QQ197NR198K
<400> 4
   Met Asp Glu Asn Leu Glu Gln Val Ser Gly Ile Ile Gly Asn Leu Arg
   1               5                   10                  15
   His Met Ala Leu Asp Met Gly Asn Glu Ile Asp Thr Gln Asn Arg Gln
               20                  25                  30
   Ile Lys Arg Ile Met Glu Lys Ala Asp Ser Asn Lys Thr Arg Ile Asp
           35                  40                  45
   Glu Ala Gln Asn Lys Ala Thr Lys Met Leu Gly Ser Gly
       50                  55                  60
<210> 5
   <211> 75
   <212> PRT
   <213> Artificial Sequence
<220>
   <223> H6tHAtev1V(2-76)LS E55QK59R
<400> 5
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
   20 25 30
   Gln Thr Gln Ala Gln Val Asp Glu Val Val Asp Ile Met Arg Val Asn
   35 40 45
   Val Asp Lys Val Leu Gln Arg Asp Gln Arg Leu Ser Glu Leu Asp Asp
   50 55 60
   Arg Ala Asp Ala Leu Gln Ala Gly Ala Ser Gln
   65 70 75
<210> 6
   <211> 75
   <212> PRT
   <213> Artificial Sequence
<220>
   <223> H6tHAtev1V(2-76)LS L54AE55QK59R
<400> 6
   Ser Ala Thr Ala Ala Thr Ala Pro Pro Ala Ala Pro Ala Gly Glu Gly
   1 5 10 15
   Gly Pro Pro Ala Pro Pro Pro Asn Leu Thr Ser Asn Arg Arg Leu Gln
   20 25 30
   Gln Thr Gln Ala Gln Val Asp Glu Val Val Asp Ile Met Arg Val Asn
   35 40 45
   Val Asp Lys Val Ala Gln Arg Asp Gln Arg Leu Ser Glu Leu Asp Asp
   50 55 60
   Arg Ala Asp Ala Leu Gln Ala Gly Ala Ser Gln
   65 70 75
<210> 7
   <211> 58
   <212> PRT
   <213> Artificial Sequence
<220>
   <223> H6tHAtevlS(140-197)L
<400> 7
   Asp Ala Arg Glu Asn Glu Met Asp Glu Asn Leu Glu Gln Val Ser Gly
   1               5                   10                  15
   Ile Ile Gly Asn Leu Arg His Met Ala Leu Asp Met Gly Asn Glu Ile
               20                  25                  30
   Asp Thr Gln Asn Arg Gln Ile Asp Arg Ile Met Glu Lys Ala Asp Ser
           35                  40                  45
   Asn Lys Thr Arg Ile Asp Glu Ala Asn Gln
       50                  55
<210> 8
   <211> 196
   <212> PRT
   <213> Artificial Sequence
<220>
   <223> H6tHAtevlS(2-197)L
<400> 8
   Ala Glu Asp Ala Asp Met Arg Asn Glu Leu Glu Glu Met Gln Arg Arg
   1               5                   10                  15
   Ala Asp Gln Leu Ala Asp Glu Ser Leu Glu Ser Thr Arg Arg Met Leu
               20                  25                  30
   Gln Leu Val Glu Glu Ser Lys Asp Ala Gly Ile Arg Thr Leu Val Met
           35                  40                  45
   Leu Asp Glu Gln Gly Glu Gln Leu Asp Arg Val Glu Glu Gly Met Asn
       50                  55                  60
   His Ile Asn Gln Asp Met Lys Glu Ala Glu Lys Asn Leu Lys Asp Leu
   65                  70                  75                  80
   Gly Lys Cys Cys Gly Leu Tyr Ile Cys Pro Cys Asn Lys Leu Lys Ser
                   85                  90                  95
   Ser Asp Ala Tyr Lys Lys Ala Trp Gly Asn Asn Gln Asp Gly Val Val
               100                 105                 110
   Ala Ser Gln Pro Ala Arg Val Val Asp Glu Arg Glu Gln Met Ala Ile
           115                 120                 125
   Ser Gly Gly Phe Ile Arg Arg Val Thr Asn Asp Ala Arg Glu Asn Glu
       130                 135                 140
   Met Asp Glu Asn Leu Glu Gln Val Ser Gly Ile Ile Gly Asn Leu Arg
   145                 150                 155                 160
   His Met Ala Leu Asp Met Gly Asn Glu Ile Asp Thr Gln Asn Arg Gln
                   165                 170                 175
   Ile Asp Arg Ile Met Glu Lys Ala Asp Ser Asn Lys Thr Arg Ile Asp
               180                 185                 190
   Glu Ala Asn Gln
           195
<210> 9
   <211> 75
   <212> PRT
   <213> Artificial Sequence
<220>
   <223> H6tHAtev1V(2-76)LS M46IE55Q
<400> 9
   Ser Ala Thr Ala Ala Thr Ala Pro Pro Ala Ala Pro Ala Gly Glu Gly
   1 5 10 15
   Gly Pro Pro Ala Pro Pro Pro Asn Leu Thr Ser Asn Arg Arg Leu Gln
   20 25 30
   Gln Thr Gln Ala Gln Val Asp Glu Val Val Asp Ile Ile Arg Val Asn
   35 40 45
   Val Asp Lys Val Leu Gln Arg Asp Gln Lys Leu Ser Glu Leu Asp Asp
   50 55 60
   Arg Ala Asp Ala Leu Gln Ala Gly Ala Ser Gln
   65 70 75
<210> 10
   <211> 75
   <212> PRT
   <213> Artificial Sequence
<220>
   <223> H6tHAtev1V(2-76)LS M46IL54AK59R
<400> 10
   Ser Ala Thr Ala Ala Thr Ala Pro Pro Ala Ala Pro Ala Gly Glu Gly
   1 5 10 15
   Gly Pro Pro Ala Pro Pro Pro Asn Leu Thr Ser Asn Arg Arg Leu Gln
   20 25 30
   Gln Thr Gln Ala Gln Val Asp Glu Val Val Asp Ile Ile Arg Val Asn
   35 40 45
   Val Asp Lys Val Ala Glu Arg Asp Gln Arg Leu Ser Glu Leu Asp Asp
   50 55 60
   Arg Ala Asp Ala Leu Gln Ala Gly Ala Ser Gln
   65 70 75
<210> 11
   <211> 55
   <212> PRT
   <213> Artificial Sequence
<220>
   <223> H6tHAtevlV(40-94)LS M46IE55QK59RA72D
<400> 11
   Asp Glu Val Val Asp Ile Ile Arg Val Asn Val Asp Lys Val Leu Gln
   1               5                   10                  15
   Arg Asp Gln Arg Leu Ser Glu Leu Asp Asp Arg Ala Asp Ala Leu Gln
               20                  25                  30
   Asp Gly Ala Ser Gln Phe Glu Thr Ser Ala Ala Lys Leu Lys Arg Lys
           35                  40                  45
   Tyr Trp Trp Lys Asn Leu Lys
       50                  55
<210> 12
   <211> 55
   <212> PRT
   <213> Artificial Sequence
<220>
   <223> H6tHAtev1V(40-94)LS M46IL54AE55QK59RA72D
<400> 12
   Asp Glu Val Val Asp Ile Ile Arg Val Asn Val Asp Lys Val Ala Gln
   1               5                   10                  15
   Arg Asp Gln Arg Leu Ser Glu Leu Asp Asp Arg Ala Asp Ala Leu Gln
               20                  25                  30
   Asp Gly Ala Ser Gln Phe Glu Thr Ser Ala Ala Lys Leu Lys Arg Lys
           35                  40                  45
   Tyr Trp Trp Lys Asn Leu Lys
       50                  55
<210> 13
   <211> 61
   <212> PRT
   <213> Artificial Sequence
<220>
   <223> H6tHA1S(146-206)LS R180WI181EE183I
<400> 13
   Met Asp Glu Asn Leu Glu Gln Val Ser Gly Ile Ile Gly Asn Leu Arg
   1               5                   10                  15
   His Met Ala Leu Asp Met Gly Asn Glu Ile Asp Thr Gln Asn Arg Gln
               20                  25                  30
   Ile Asp Trp Glu Met Ile Lys Ala Asp Ser Asn Lys Thr Arg Ile Asp
           35                  40                  45
   Glu Ala Asn Gln Arg Ala Thr Lys Met Leu Gly Ser Gly
       50                  55                  60
<210> 14
   <211> 61
   <212> PRT
   <213> Artificial Sequence
<220>
   <223> H6tHA1S(146-206)LS D179K
<400> 14
   Met Asp Glu Asn Leu Glu Gln Val Ser Gly Ile Ile Gly Asn Leu Arg
   1               5                   10                  15
   His Met Ala Leu Asp Met Gly Asn Glu Ile Asp Thr Gln Asn Arg Gln
               20                  25                  30
   Ile Lys Arg Ile Met Glu Lys Ala Asp Ser Asn Lys Thr Arg Ile Asp
           35                  40                  45
   Glu Ala Asn Gln Arg Ala Thr Lys Met Leu Gly Ser Gly
       50                  55                  60
<210> 15
   <211> 61
   <212> PRT
   <213> Artificial Sequence
<220>
   <223> H6tHAtevlS(146-206)LS A199R
<400> 15
   Met Asp Glu Asn Leu Glu Gln Val Ser Gly Ile Ile Gly Asn Leu Arg
   1               5                   10                  15
   His Met Ala Leu Asp Met Gly Asn Glu Ile Asp Thr Gln Asn Arg Gln
               20                  25                  30
   Ile Asp Arg Ile Met Glu Lys Ala Asp Ser Asn Lys Thr Arg Ile Asp
   35 40 45
   Glu Ala Asn Gln Arg Arg Thr Lys Met Leu Gly Ser Gly
   50 55 60
<210> 16
   <211> 61
   <212> PRT
   <213> Artificial Sequence
<220>
   <223> H6tHAtev1S(146-206)LS N196QQ197NR198K
<400> 16
   Met Asp Glu Asn Leu Glu Gln Val Ser Gly Ile Ile Gly Asn Leu Arg
   1 5 10 15
   His Met Ala Leu Asp Met Gly Asn Glu Ile Asp Thr Gln Asn Arg Gln
   20 25 30
   Ile Asp Arg Ile Met Glu Lys Ala Asp Ser Asn Lys Thr Arg Ile Asp
   35 40 45
   Glu Ala Gln Asn Lys Ala Thr Lys Met Leu Gly Ser Gly
   50 55 60
<210> 17
   <211> 55
   <212> PRT
   <213> Artificial Sequence
<220>
   <223> H6tHAtev1V(40-94)LS M46IL54AE55QK59R
<400> 17
   Asp Glu Val Val Asp Ile Ile Arg Val Asn Val Asp Lys Val Ala Gln
   1 5 10 15
   Arg Asp Gln Arg Leu Ser Glu Leu Asp Asp Arg Ala Asp Ala Leu Gln
   20 25 30
   Ala Gly Ala Ser Gln Phe Glu Thr Ser Ala Ala Lys Leu Lys Arg Lys
   35 40 45
   Tyr Trp Trp Lys Asn Leu Lys
   50 55
<210> 18
   <211> 75
   <212> PRT
   <213> Artificial Sequence
<220>
   <223> H6tHAtev1V(2-76)LS K59R
<400> 18
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Val Asp Ile Met Arg Val Asn
           35                  40                  45
   Val Asp Lys Val Leu Glu Arg Asp Gln Arg Leu Ser Glu Leu Asp Asp
       50                  55                  60
   Arg Ala Asp Ala Leu Gln Ala Gly Ala Ser Gln
   65                  70                  75
<210> 19
   <211> 75
   <212> PRT
   <213> Artificial Sequence
<220>
   <223> H6tHAtev1V(2-76)LS M46I
<400> 19
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Val Asp Ile Ile Arg Val Asn
           35                  40                  45
   Val Asp Lys Val Leu Glu Arg Asp Gln Lys Leu Ser Glu Leu Asp Asp
       50                  55                  60
   Arg Ala Asp Ala Leu Gln Ala Gly Ala Ser Gln
   65                  70                  75
<210> 20
   <211> 75
   <212> PRT
   <213> Artificial Sequence
<220>
   <223> H6tHAtev1V(2-76)LS E55Q
<400> 20
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Val Asp Ile Met Arg Val Asn
           35                  40                  45
   Val Asp Lys Val Leu Gln Arg Asp Gln Lys Leu Ser Glu Leu Asp Asp
       50                  55                  60
   Arg Ala Asp Ala Leu Gln Ala Gly Ala Ser Gln
   65                  70                  75
<210> 21
   <211> 61
   <212> PRT
   <213> Artificial Sequence
<220>
   <223> H6tHAtev1S(146-206)LS
<400> 21
   Met Asp Glu Asn Leu Glu Gln Val Ser Gly Ile Ile Gly Asn Leu Arg
   1               5                   10                  15
   His Met Ala Leu Asp Met Gly Asn Glu Ile Asp Thr Gln Asn Arg Gln
               20                  25                  30
   Ile Asp Arg Ile Met Glu Lys Ala Asp Ser Asn Lys Thr Arg Ile Asp
           35                  40                  45
   Glu Ala Asn Gln Arg Ala Thr Lys Met Leu Gly Ser Gly
       50                  55                  60
<210> 22
   <211> 205
   <212> PRT
   <213> Artificial Sequence
<220>
   <223> H6tHAtev1S(2-206)L(S)
<400> 22
   Ala Glu Asp Ala Asp Met Arg Asn Glu Leu Glu Glu Met Gln Arg Arg
   1               5                   10                  15
   Ala Asp Gln Leu Ala Asp Glu Ser Leu Glu Ser Thr Arg Arg Met Leu
               20                  25                  30
   Gln Leu Val Glu Glu Ser Lys Asp Ala Gly Ile Arg Thr Leu Val Met
           35                  40                  45
   Leu Asp Glu Gln Gly Glu Gln Leu Asp Arg Val Glu Glu Gly Met Asn
       50                  55                  60
   His Ile Asn Gln Asp Met Lys Glu Ala Glu Lys Asn Leu Lys Asp Leu
   65                  70                  75                  80
   Gly Lys Cys Cys Gly Leu Tyr Ile Cys Pro Cys Asn Lys Leu Lys Ser
                   85                  90                  95
   Ser Asp Ala Tyr Lys Lys Ala Trp Gly Asn Asn Gln Asp Gly Val Val
               100                 105                 110
   Ala Ser Gln Pro Ala Arg Val Val Asp Glu Arg Glu Gln Met Ala Ile
           115                 120                 125
   Ser Gly Gly Phe Ile Arg Arg Val Thr Asn Asp Ala Arg Glu Asn Glu
       130                 135                 140
   Met Asp Glu Asn Leu Glu Gln Val Ser Gly Ile Ile Gly Asn Leu Arg
   145                 150                 155                 160
   His Met Ala Leu Asp Met Gly Asn Glu Ile Asp Thr Gln Asn Arg Gln
                   165                 170                 175
   Ile Asp Arg Ile Met Glu Lys Ala Asp Ser Asn Lys Thr Arg Ile Asp
               180                 185                 190
   Glu Ala Asn Gln Arg Ala Thr Lys Met Leu Gly Ser Gly
           195                 200                 205
<210> 23
   <211> 75
   <212> PRT
   <213> Artificial Sequence
<220>
   <223> H6tHA1V(2-76)LS
<400> 23
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Val Asp Ile Met Arg Val Asn
           35                  40                  45
   Val Asp Lys Val Leu Glu Arg Asp Gln Lys Leu Ser Glu Leu Asp Asp
       50                  55                  60
   Arg Ala Asp Ala Leu Gln Ala Gly Ala Ser Gln
   65                  70                  75
<210> 24
   <211> 93
   <212> PRT
   <213> Artificial Sequence
<220>
   <223> H6tHAtev1V(2-94)LS A72D
<400> 24
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Val Asp Ile Met Arg Val Asn
           35                  40                  45
   Val Asp Lys Val Leu Glu Arg Asp Gln Lys Leu Ser Glu Leu Asp Asp
       50                  55                  60
   Arg Ala Asp Ala Leu Gln Asp Gly Ala Ser Gln Phe Glu Thr Ser Ala
   65                  70                  75                  80
   Ala Lys Leu Lys Arg Lys Tyr Trp Trp Lys Asn Leu Lys
                   85                  90
<210> 25
   <211> 93
   <212> PRT
   <213> Artificial Sequence
<220>
   <223> H6tHAtev1V(2-94)LS M46I
<400> 25
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Val Asp Ile Ile Arg Val Asn
           35                  40                  45
   Val Asp Lys Val Leu Glu Arg Asp Gln Lys Leu Ser Glu Leu Asp Asp
       50                  55                  60
   Arg Ala Asp Ala Leu Gln Ala Gly Ala Ser Gln Phe Glu Thr Ser Ala
   65                  70                  75                  80
   Ala Lys Leu Lys Arg Lys Tyr Trp Trp Lys Asn Leu Lys
                   85                  90
<210> 26
   <211> 93
   <212> PRT
   <213> Artificial Sequence
<220>
   <223> H6tHAtev1V(2-94)LS K59R
<400> 26
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Val Asp Ile Met Arg Val Asn
           35                  40                  45
   Val Asp Lys Val Leu Glu Arg Asp Gln Arg Leu Ser Glu Leu Asp Asp
       50                  55                  60
   Arg Ala Asp Ala Leu Gln Ala Gly Ala Ser Gln Phe Glu Thr Ser Ala
   65                  70                  75                  80
   Ala Lys Leu Lys Arg Lys Tyr Trp Trp Lys Asn Leu Lys
                   85                  90
<210> 27
   <211> 93
   <212> PRT
   <213> Artificial Sequence
<220>
   <223> H6tHAtev1V(2-94)LS L54AK59R
<400> 27
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Val Asp Ile Met Arg Val Asn
           35                  40                  45
   Val Asp Lys Val Ala Glu Arg Asp Gln Arg Leu Ser Glu Leu Asp Asp
       50                  55                  60
   Arg Ala Asp Ala Leu Gln Ala Gly Ala Ser Gln Phe Glu Thr Ser Ala
   65                  70                  75                  80
   Ala Lys Leu Lys Arg Lys Tyr Trp Trp Lys Asn Leu Lys
                   85                  90
<210> 28
   <211> 93
   <212> PRT
   <213> Artificial Sequence
<220>
   <223> H6tHAtev1V(2-94)LS E55Q
<400> 28
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Val Asp Ile Met Arg Val Asn
           35                  40                  45
   Val Asp Lys Val Leu Gln Arg Asp Gln Lys Leu Ser Glu Leu Asp Asp
       50                  55                  60
   Arg Ala Asp Ala Leu Gln Ala Gly Ala Ser Gln Phe Glu Thr Ser Ala
   65                  70                  75                  80
   Ala Lys Leu Lys Arg Lys Tyr Trp Trp Lys Asn Leu Lys
                   85                  90
<210> 29
   <211> 93
   <212> PRT
   <213> Artificial Sequence
<220>
   <223> H6tHAtev1V(2-94)LS A69N
<400> 29
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Val Asp Ile Met Arg Val Asn
           35                  40                  45
   Val Asp Lys Val Leu Glu Arg Asp Gln Lys Leu Ser Glu Leu Asp Asp
       50                  55                  60
   Arg Ala Asp Asn Leu Gln Ala Gly Ala Ser Gln Phe Glu Thr Ser Ala
   65                  70                  75                  80
   Ala Lys Leu Lys Arg Lys Tyr Trp Trp Lys Asn Leu Lys
                   85                  90
<210> 30
   <211> 93
   <212> PRT
   <213> Artificial Sequence
<220>
   <223> H6tHAtev1V(2-94)LS
<400> 30
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Val Asp Ile Met Arg Val Asn
           35                  40                  45
   Val Asp Lys Val Leu Glu Arg Asp Gln Lys Leu Ser Glu Leu Asp Asp
       50                  55                  60
   Arg Ala Asp Ala Leu Gln Ala Gly Ala Ser Gln Phe Glu Thr Ser Ala
   65                  70                  75                  80
   Ala Lys Leu Lys Arg Lys Tyr Trp Trp Lys Asn Leu Lys
                       85                  90
<210> 31
   <211> 93
   <212> PRT
   <213> Artificial Sequence
<220>
   <223> H6tHAtev1V(2-94)LS T79S
<400> 31
   Ser Ala Thr Ala Ala Thr Ala Pro Pro Ala Ala Pro Ala Gly Glu Gly
   1 5 10 15
   Gly Pro Pro Ala Pro Pro Pro Asn Leu Thr Ser Asn Arg Arg Leu Gln
   20 25 30
   Gln Thr Gln Ala Gln Val Asp Glu Val Val Asp Ile Met Arg Val Asn
   35 40 45
   Val Asp Lys Val Leu Glu Arg Asp Gln Lys Leu Ser Glu Leu Asp Asp
   50 55 60
   Arg Ala Asp Ala Leu Gln Ala Gly Ala Ser Gln Phe Glu Ser Ser Ala
   65 70 75 80
   Ala Lys Leu Lys Arg Lys Tyr Trp Trp Lys Asn Leu Lys
   85 90
<210> 32
   <211> 1296
   <212> PRT
   <213> Clostridium botulinum
<400> 32
   Met Pro Phe Val Asn Lys Gln Phe Asn Tyr Lys Asp Pro Val Asn Gly
   1 5 10 15
   Val Asp Ile Ala Tyr Ile Lys Ile Pro Asn Ala Gly Gln Met Gln Pro
   20 25 30
   Val Lys Ala Phe Lys Ile His Asn Lys Ile Trp Val Ile Pro Glu Arg
   35 40 45
   Asp Thr Phe Thr Asn Pro Glu Glu Gly Asp Leu Asn Pro Pro Pro Glu
   50 55 60
   Ala Lys Gln Val Pro Val Ser Tyr Tyr Asp Ser Thr Tyr Leu Ser Thr
   65 70 75 80
   Asp Asn Glu Lys Asp Asn Tyr Leu Lys Gly Val Thr Lys Leu Phe Glu
                   85                  90                  95
   Arg Ile Tyr Ser Thr Asp Leu Gly Arg Met Leu Leu Thr Ser Ile Val
               100                 105                 110
   Arg Gly Ile Pro Phe Trp Gly Gly Ser Thr Ile Asp Thr Glu Leu Lys
           115                 120                 125
   Val Ile Asp Thr Asn Cys Ile Asn Val Ile Gln Pro Asp Gly Ser Tyr
       130                 135                 140
   Arg Ser Glu Glu Leu Asn Leu Val Ile Ile Gly Pro Ser Ala Asp Ile
   145                 150                 155                 160
   Ile Gln Phe Glu Cys Lys Ser Phe Gly His Glu Val Leu Asn Leu Thr
                   165                 170                 175
   Arg Asn Gly Tyr Gly Ser Thr Gln Tyr Ile Arg Phe Ser Pro Asp Phe
               180                 185                 190
   Thr Phe Gly Phe Glu Glu Ser Leu Glu Val Asp Thr Asn Pro Leu Leu
           195                 200                 205
   Gly Ala Gly Lys Phe Ala Thr Asp Pro Ala Val Thr Leu Ala His Glu
       210                 215                 220
   Leu Ile His Ala Gly His Arg Leu Tyr Gly Ile Ala Ile Asn Pro Asn
   225                 230                 235                 240
   Arg Val Phe Lys Val Asn Thr Asn Ala Tyr Tyr Glu Met Ser Gly Leu
                   245                 250                 255
   Glu Val Ser Phe Glu Glu Leu Arg Thr Phe Gly Gly His Asp Ala Lys
               260                 265                 270
   Phe Ile Asp Ser Leu Gln Glu Asn Glu Phe Arg Leu Tyr Tyr Tyr Asn
           275                 280                 285
   Lys Phe Lys Asp Ile Ala Ser Thr Leu Asn Lys Ala Lys Ser Ile Val
       290                 295                 300
   Gly Thr Thr Ala Ser Leu Gln Tyr Met Lys Asn Val Phe Lys Glu Lys
   305                 310                 315                 320
   Tyr Leu Leu Ser Glu Asp Thr Ser Gly Lys Phe Ser Val Asp Lys Leu
                   325                 330                 335
   Lys Phe Asp Lys Leu Tyr Lys Met Leu Thr Glu Ile Tyr Thr Glu Asp
               340                 345                 350
   Asn Phe Val Lys Phe Phe Lys Val Leu Asn Arg Lys Thr Tyr Leu Asn
           355                 360                 365
   Phe Asp Lys Ala Val Phe Lys Ile Asn Ile Val Pro Lys Val Asn Tyr
       370                 375                 380
   Thr Ile Tyr Asp Gly Phe Asn Leu Arg Asn Thr Asn Leu Ala Ala Asn
   385                 390                 395                 400
   Phe Asn Gly Gln Asn Thr Glu Ile Asn Asn Met Asn Phe Thr Lys Leu
                   405                 410                 415
   Lys Asn Phe Thr Gly Leu Phe Glu Phe Tyr Lys Leu Leu Cys Val Arg
               420                 425                 430
   Gly Ile Ile Thr Ser Lys Thr Lys Ser Leu Asp Lys Gly Tyr Asn Lys
           435                 440                 445
   Ala Leu Asn Asp Leu Cys Ile Lys Val Asn Asn Trp Asp Leu Phe Phe
       450                 455                 460
   Ser Pro Ser Glu Asp Asn Phe Thr Asn Asp Leu Asn Lys Gly Glu Glu
   465                 470                 475                 480
   Ile Thr Ser Asp Thr Asn Ile Glu Ala Ala Glu Glu Asn Ile Ser Leu
                   485                 490                 495
   Asp Leu Ile Gln Gln Tyr Tyr Leu Thr Phe Asn Phe Asp Asn Glu Pro
               500                 505                 510
   Glu Asn Ile Ser Ile Glu Asn Leu Ser Ser Asp Ile Ile Gly Gln Leu
           515                 520                 525
   Glu Leu Met Pro Asn Ile Glu Arg Phe Pro Asn Gly Lys Lys Tyr Glu
       530                 535                 540
   Leu Asp Lys Tyr Thr Met Phe His Tyr Leu Arg Ala Gln Glu Phe Glu
   545                 550                 555                 560
   His Gly Lys Ser Arg Ile Ala Leu Thr Asn Ser Val Asn Glu Ala Leu
                   565                 570                 575
   Leu Asn Pro Ser Arg Val Tyr Thr Phe Phe Ser Ser Asp Tyr Val Lys
   580 585 590
   Lys Val Asn Lys Ala Thr Glu Ala Ala Met Phe Leu Gly Trp Val Glu
   595 600 605
   Gln Leu Val Tyr Asp Phe Thr Asp Glu Thr Ser Glu Val Ser Thr Thr
   610 615 620
   Asp Lys Ile Ala Asp Ile Thr Ile Ile Ile Pro Tyr Ile Gly Pro Ala
   625 630 635 640
   Leu Asn Ile Gly Asn Met Leu Tyr Lys Asp Asp Phe Val Gly Ala Leu
   645 650 655
   Ile Phe Ser Gly Ala Val Ile Leu Leu Glu Phe Ile Pro Glu Ile Ala
   660 665 670
   Ile Pro Val Leu Gly Thr Phe Ala Leu Val Ser Tyr Ile Ala Asn Lys
   675 680 685
   Val Leu Thr Val Gln Thr Ile Asp Asn Ala Leu Ser Lys Arg Asn Glu
   690 695 700
   Lys Trp Asp Glu Val Tyr Lys Tyr Ile Val Thr Asn Trp Leu Ala Lys
   705 710 715 720
   Val Asn Thr Gln Ile Asp Leu Ile Arg Lys Lys Met Lys Glu Ala Leu
   725 730 735
   Glu Asn Gln Ala Glu Ala Thr Lys Ala Ile Ile Asn Tyr Gln Tyr Asn
   740 745 750
   Gln Tyr Thr Glu Glu Glu Lys Asn Asn Ile Asn Phe Asn Ile Asp Asp
   755 760 765
   Leu Ser Ser Lys Leu Asn Glu Ser Ile Asn Lys Ala Met Ile Asn Ile
   770 775 780
   Asn Lys Phe Leu Asn Gln Cys Ser Val Ser Tyr Leu Met Asn Ser Met
   785 790 795 800
   Ile Pro Tyr Gly Val Lys Arg Leu Glu Asp Phe Asp Ala Ser Leu Lys
   805 810 815
   Asp Ala Leu Leu Lys Tyr Ile Tyr Asp Asn Arg Gly Thr Leu Ile Gly
   820 825 830
   Gln Val Asp Arg Leu Lys Asp Lys Val Asn Asn Thr Leu Ser Thr Asp
   835 840 845
   Ile Pro Phe Gln Leu Ser Lys Tyr Val Asp Asn Gln Arg Leu Leu Ser
   850 855 860
   Thr Phe Thr Glu Tyr Ile Lys Asn Ile Ile Asn Thr Ser Ile Leu Asn
   865 870 875 880
   Leu Arg Tyr Glu Ser Asn His Leu Ile Asp Leu Ser Arg Tyr Ala Ser
   885 890 895
   Lys Ile Asn Ile Gly Ser Lys Val Asn Phe Asp Pro Ile Asp Lys Asn
   900 905 910
   Gln Ile Gln Leu Phe Asn Leu Glu Ser Ser Lys Ile Glu Val Ile Leu
   915 920 925
   Lys Asn Ala Ile Val Tyr Asn Ser Met Tyr Glu Asn Phe Ser Thr Ser
   930 935 940
   Phe Trp Ile Arg Ile Pro Lys Tyr Phe Asn Ser Ile Ser Leu Asn Asn
   945 950 955 960
   Glu Tyr Thr Ile Ile Asn Cys Met Glu Asn Asn Ser Gly Trp Lys Val
   965 970 975
   Ser Leu Asn Tyr Gly Glu Ile Ile Trp Thr Leu Gln Asp Thr Gln Glu
   980 985 990
   Ile Lys Gln Arg Val Val Phe Lys Tyr Ser Gln Met Ile Asn Ile Ser
   995 1000 1005
   Asp Tyr Ile Asn Arg Trp Ile Phe Val Thr Ile Thr Asn Asn Arg
   1010 1015 1020
   Leu Asn Asn Ser Lys Ile Tyr Ile Asn Gly Arg Leu Ile Asp Gln
   1025 1030 1035
   Lys Pro Ile Ser Asn Leu Gly Asn Ile His Ala Ser Asn Asn Ile
   1040 1045 1050
   Met Phe Lys Leu Asp Gly Cys Arg Asp Thr His Arg Tyr Ile Trp
   1055 1060 1065
   Ile Lys Tyr Phe Asn Leu Phe Asp Lys Glu Leu Asn Glu Lys Glu
   1070 1075 1080
   Ile Lys Asp Leu Tyr Asp Asn Gln Ser Asn Ser Gly Ile Leu Lys
       1085                1090                1095
   Asp Phe Trp Gly Asp Tyr Leu Gln Tyr Asp Lys Pro Tyr Tyr Met
       1100                1105                1110
   Leu Asn Leu Tyr Asp Pro Asn Lys Tyr Val Asp Val Asn Asn Val
       1115                1120                1125
   Gly Ile Arg Gly Tyr Met Tyr Leu Lys Gly Pro Arg Gly Ser Val
       1130                1135                1140
   Met Thr Thr Asn Ile Tyr Leu Asn Ser Ser Leu Tyr Arg Gly Thr
       1145                1150                1155
   Lys Phe Ile Ile Lys Lys Tyr Ala Ser Gly Asn Lys Asp Asn Ile
       1160                1165                1170
   Val Arg Asn Asn Asp Arg Val Tyr Ile Asn Val Val Val Lys Asn
       1175                1180                1185
   Lys Glu Tyr Arg Leu Ala Thr Asn Ala Ser Gln Ala Gly Val Glu
       1190                1195                1200
   Lys Ile Leu Ser Ala Leu Glu Ile Pro Asp Val Gly Asn Leu Ser
       1205                1210                1215
   Gln Val Val Val Met Lys Ser Lys Asn Asp Gln Gly Ile Thr Asn
       1220                1225                1230
   Lys Cys Lys Met Asn Leu Gln Asp Asn Asn Gly Asn Asp Ile Gly
       1235                1240                1245
   Phe Ile Gly Phe His Gln Phe Asn Asn Ile Ala Lys Leu Val Ala
       1250                1255                1260
   Ser Asn Trp Tyr Asn Arg Gln Ile Glu Arg Ser Ser Arg Thr Leu
       1265                1270                1275
   Gly Cys Ser Trp Glu Phe Ile Pro Val Asp Asp Gly Trp Gly Glu
       1280                1285                1290
   Arg Pro Leu
       1295
<210> 33
   <211> 1291
   <212> PRT
   <213> Clostridium botulinum
<400> 33
   Met Pro Val Thr Ile Asn Asn Phe Asn Tyr Asn Asp Pro Ile Asp Asn
   1               5                   10                  15
   Asn Asn Ile Ile Met Met Glu Pro Pro Phe Ala Arg Gly Thr Gly Arg
               20                  25                  30
   Tyr Tyr Lys Ala Phe Lys Ile Thr Asp Arg Ile Trp Ile Ile Pro Glu
           35                  40                  45
   Arg Tyr Thr Phe Gly Tyr Lys Pro Glu Asp Phe Asn Lys Ser Ser Gly
       50                  55                  60
   Ile Phe Asn Arg Asp Val Cys Glu Tyr Tyr Asp Pro Asp Tyr Leu Asn
   65                  70                  75                  80
   Thr Asn Asp Lys Lys Asn Ile Phe Leu Gln Thr Met Ile Lys Leu Phe
                   85                  90                  95
   Asn Arg Ile Lys Ser Lys Pro Leu Gly Glu Lys Leu Leu Glu Met Ile
               100                 105                 110
   Ile Asn Gly Ile Pro Tyr Leu Gly Asp Arg Arg Val Pro Leu Glu Glu
           115                 120                 125
   Phe Asn Thr Asn Ile Ala Ser Val Thr Val Asn Lys Leu Ile Ser Asn
       130                 135                 140
   Pro Gly Glu Val Glu Arg Lys Lys Gly Ile Phe Ala Asn Leu Ile Ile
   145                 150                 155                 160
   Phe Gly Pro Gly Pro Val Leu Asn Glu Asn Glu Thr Ile Asp Ile Gly
                   165                 170                 175
   Ile Gln Asn His Phe Ala Ser Arg Glu Gly Phe Gly Gly Ile Met Gln
               180                 185                 190
   Met Lys Phe Cys Pro Glu Tyr Val Ser Val Phe Asn Asn Val Gln Glu
           195                 200                 205
   Asn Lys Gly Ala Ser Ile Phe Asn Arg Arg Gly Tyr Phe Ser Asp Pro
       210                 215                 220
   Ala Leu Ile Leu Met His Glu Leu Ile His Val Leu His Gly Leu Tyr
   225                 230                 235                 240
   Gly Ile Lys Val Asp Asp Leu Pro Ile Val Pro Asn Glu Lys Lys Phe
   245 250 255
   Phe Met Gln Ser Thr Asp Ala Ile Gln Ala Glu Glu Leu Tyr Thr Phe
   260 265 270
   Gly Gly Gln Asp Pro Ser Ile Ile Thr Pro Ser Thr Asp Lys Ser Ile
   275 280 285
   Tyr Asp Lys Val Leu Gln Asn Phe Arg Gly Ile Val Asp Arg Leu Asn
   290 295 300
   Lys Val Leu Val Cys Ile Ser Asp Pro Asn Ile Asn Ile Asn Ile Tyr
   305 310 315 320
   Lys Asn Lys Phe Lys Asp Lys Tyr Lys Phe Val Glu Asp Ser Glu Gly
   325 330 335
   Lys Tyr Ser Ile Asp Val Glu Ser Phe Asp Lys Leu Tyr Lys Ser Leu
   340 345 350
   Met Phe Gly Phe Thr Glu Thr Asn Ile Ala Glu Asn Tyr Lys Ile Lys
   355 360 365
   Thr Arg Ala Ser Tyr Phe Ser Asp Ser Leu Pro Pro Val Lys Ile Lys
   370 375 380
   Asn Leu Leu Asp Asn Glu Ile Tyr Thr Ile Glu Glu Gly Phe Asn Ile
   385 390 395 400
   Ser Asp Lys Asp Met Glu Lys Glu Tyr Arg Gly Gln Asn Lys Ala Ile
   405 410 415
   Asn Lys Gln Ala Tyr Glu Glu Ile Ser Lys Glu His Leu Ala Val Tyr
   420 425 430
   Lys Ile Gln Met Cys Lys Ser Val Lys Ala Pro Gly Ile Cys Ile Asp
   435 440 445
   Val Asp Asn Glu Asp Leu Phe Phe Ile Ala Asp Lys Asn Ser Phe Ser
   450 455 460
   Asp Asp Leu Ser Lys Asn Glu Arg Ile Glu Tyr Asn Thr Gln Ser Asn
   465 470 475 480
   Tyr Ile Glu Asn Asp Phe Pro Ile Asn Glu Leu Ile Leu Asp Thr Asp
                   485                 490                 495
   Leu Ile Ser Lys Ile Glu Leu Pro Ser Glu Asn Thr Glu Ser Leu Thr
               500                 505                 510
   Asp Phe Asn Val Asp Val Pro Val Tyr Glu Lys Gln Pro Ala Ile Lys
           515                 520                 525
   Lys Ile Phe Thr Asp Glu Asn Thr Ile Phe Gln Tyr Leu Tyr Ser Gln
       530                 535                 540
   Thr Phe Pro Leu Asp Ile Arg Asp Ile Ser Leu Thr Ser Ser Phe Asp
   545                 550                 555                 560
   Asp Ala Leu Leu Phe Ser Asn Lys Val Tyr Ser Phe Phe Ser Met Asp
                   565                 570                 575
   Tyr Ile Lys Thr Ala Asn Lys Val Val Glu Ala Gly Leu Phe Ala Gly
               580                 585                 590
   Trp Val Lys Gln Ile Val Asn Asp Phe Val Ile Glu Ala Asn Lys Ser
           595                 600                 605
   Asn Thr Met Asp Lys Ile Ala Asp Ile Ser Leu Ile Val Pro Tyr Ile
       610                 615                 620
   Gly Leu Ala Leu Asn Val Gly Asn Glu Thr Ala Lys Gly Asn Phe Glu
   625                 630                 635                 640
   Asn Ala Phe Glu Ile Ala Gly Ala Ser Ile Leu Leu Glu Phe Ile Pro
                   645                 650                 655
   Glu Leu Leu Ile Pro Val Val Gly Ala Phe Leu Leu Glu Ser Tyr Ile
               660                 665                 670
   Asp Asn Lys Asn Lys Ile Ile Lys Thr Ile Asp Asn Ala Leu Thr Lys
           675                 680                 685
   Arg Asn Glu Lys Trp Ser Asp Met Tyr Gly Leu Ile Val Ala Gln Trp
       690                 695                 700
   Leu Ser Thr Val Asn Thr Gln Phe Tyr Thr Ile Lys Glu Gly Met Tyr
   705                 710                 715                 720
   Lys Ala Leu Asn Tyr Gln Ala Gln Ala Leu Glu Glu Ile Ile Lys Tyr
                   725                 730                 735
   Arg Tyr Asn Ile Tyr Ser Glu Lys Glu Lys Ser Asn Ile Asn Ile Asp
               740                 745                 750
   Phe Asn Asp Ile Asn Ser Lys Leu Asn Glu Gly Ile Asn Gln Ala Ile
           755                 760                 765
   Asp Asn Ile Asn Asn Phe Ile Asn Gly Cys Ser Val Ser Tyr Leu Met
       770                 775                 780
   Lys Lys Met Ile Pro Leu Ala Val Glu Lys Leu Leu Asp Phe Asp Asn
   785                 790                 795                 800
   Thr Leu Lys Lys Asn Leu Leu Asn Tyr Ile Asp Glu Asn Lys Leu Tyr
                   805                 810                 815
   Leu Ile Gly Ser Ala Glu Tyr Glu Lys Ser Lys Val Asn Lys Tyr Leu
               820                 825                 830
   Lys Thr Ile Met Pro Phe Asp Leu Ser Ile Tyr Thr Asn Asp Thr Ile
           835                 840                 845
   Leu Ile Glu Met Phe Asn Lys Tyr Asn Ser Glu Ile Leu Asn Asn Ile
       850                 855                 860
   Ile Leu Asn Leu Arg Tyr Lys Asp Asn Asn Leu Ile Asp Leu Ser Gly
   865                 870                 875                 880
   Tyr Gly Ala Lys Val Glu Val Tyr Asp Gly Val Glu Leu Asn Asp Lys
                   885                 890                 895
   Asn Gln Phe Lys Leu Thr Ser Ser Ala Asn Ser Lys Ile Arg Val Thr
               900                 905                 910
   Gln Asn Gln Asn Ile Ile Phe Asn Ser Val Phe Leu Asp Phe Ser Val
           915                 920                 925
   Ser Phe Trp Ile Arg Ile Pro Lys Tyr Lys Asn Asp Gly Ile Gln Asn
       930                 935                 940
   Tyr Ile His Asn Glu Tyr Thr Ile Ile Asn Cys Met Lys Asn Asn Ser
   945                 950                 955                 960
   Gly Trp Lys Ile Ser Ile Arg Gly Asn Arg Ile Ile Trp Thr Leu Ile
                   965                 970                 975
   Asp Ile Asn Gly Lys Thr Lys Ser Val Phe Phe Glu Tyr Asn Ile Arg
               980                 985                 990
   Glu Asp Ile Ser Glu Tyr Ile Asn Arg Trp Phe Phe Val Thr Ile Thr
   995 1000 1005
   Asn Asn Leu Asn Asn Ala Lys Ile Tyr Ile Asn Gly Lys Leu Glu
   1010 1015 1020
   Ser Asn Thr Asp Ile Lys Asp Ile Arg Glu Val Ile Ala Asn Gly
   1025 1030 1035
   Glu Ile Ile Phe Lys Leu Asp Gly Asp Ile Asp Arg Thr Gln Phe
   1040 1045 1050
   Ile Trp Met Lys Tyr Phe Ser Ile Phe Asn Thr Glu Leu Ser Gln
   1055 1060 1065
   Ser Asn Ile Glu Glu Arg Tyr Lys Ile Gln Ser Tyr Ser Glu Tyr
   1070 1075 1080
   Leu Lys Asp Phe Trp Gly Asn Pro Leu Met Tyr Asn Lys Glu Tyr
   1085 1090 1095
   Tyr Met Phe Asn Ala Gly Asn Lys Asn Ser Tyr Ile Lys Leu Lys
   1100 1105 1110
   Lys Asp Ser Pro Val Gly Glu Ile Leu Thr Arg Ser Lys Tyr Asn
   1115 1120 1125
   Gln Asn Ser Lys Tyr Ile Asn Tyr Arg Asp Leu Tyr Ile Gly Glu
   1130 1135 1140
   Lys Phe Ile Ile Arg Arg Lys Ser Asn Ser Gln Ser Ile Asn Asp
   1145 1150 1155
   Asp Ile Val Arg Lys Glu Asp Tyr Ile Tyr Leu Asp Phe Phe Asn
   1160 1165 1170
   Leu Asn Gln Glu Trp Arg Val Tyr Thr Tyr Lys Tyr Phe Lys Lys
   1175 1180 1185
   Glu Glu Glu Lys Leu Phe Leu Ala Pro Ile Ser Asp Ser Asp Glu
   1190 1195 1200
   Phe Tyr Asn Thr Ile Gln Ile Lys Glu Tyr Asp Glu Gln Pro Thr
   1205 1210 1215
   Tyr Ser Cys Gln Leu Leu Phe Lys Lys Asp Glu Glu Ser Thr Asp
   1220 1225 1230
   Glu Ile  Gly Leu Ile Gly Ile  His Arg Phe Tyr Glu  Ser Gly Ile
       1235                 1240                 1245
   Val Phe  Glu Glu Tyr Lys Asp  Tyr Phe Cys Ile Ser  Lys Trp Tyr
       1250                 1255                 1260
   Leu Lys  Glu Val Lys Arg Lys  Pro Tyr Asn Leu Lys  Leu Gly Cys
       1265                 1270                 1275
   Asn Trp  Gln Phe Ile Pro Lys  Asp Glu Gly Trp Thr  Glu
       1280                 1285                 1290
<210> 34
   <211> 1291
   <212> PRT
   <213> Clostridium botulinum
<400> 34
   Met Pro Ile Thr Ile Asn Asn Phe Asn Tyr Ser Asp Pro Val Asp Asn
   1               5                   10                  15
   Lys Asn Ile Leu Tyr Leu Asp Thr His Leu Asn Thr Leu Ala Asn Glu
               20                  25                  30
   Pro Glu Lys Ala Phe Arg Ile Thr Gly Asn Ile Trp Val Ile Pro Asp
           35                  40                  45
   Arg Phe Ser Arg Asn Ser Asn Pro Asn Leu Asn Lys Pro Pro Arg Val
       50                  55                  60
   Thr Ser Pro Lys Ser Gly Tyr Tyr Asp Pro Asn Tyr Leu Ser Thr Asp
   65                  70                  75                  80
   Ser Asp Lys Asp Pro Phe Leu Lys Glu Ile Ile Lys Leu Phe Lys Arg
                   85                  90                  95
   Ile Asn Ser Arg Glu Ile Gly Glu Glu Leu Ile Tyr Arg Leu Ser Thr
               100                 105                 110
   Asp Ile Pro Phe Pro Gly Asn Asn Asn Thr Pro Ile Asn Thr Phe Asp
           115                 120                 125
   Phe Asp Val Asp Phe Asn Ser Val Asp Val Lys Thr Arg Gln Gly Asn
       130                 135                 140
   Asn Trp Val Lys Thr Gly Ser Ile Asn Pro Ser Val Ile Ile Thr Gly
   145                 150                 155                 160
   Pro Arg Glu Asn Ile Ile Asp Pro Glu Thr Ser Thr Phe Lys Leu Thr
   165 170 175
   Asn Asn Thr Phe Ala Ala Gln Glu Gly Phe Gly Ala Leu Ser Ile Ile
   180 185 190
   Ser Ile Ser Pro Arg Phe Met Leu Thr Tyr Ser Asn Ala Thr Asn Asp
   195 200 205
   Val Gly Glu Gly Arg Phe Ser Lys Ser Glu Phe Cys Met Asp Pro Ile
   210 215 220
   Leu Ile Leu Met His Glu Leu Asn His Ala Met His Asn Leu Tyr Gly
   225 230 235 240
   Ile Ala Ile Pro Asn Asp Gln Thr Ile Ser Ser Val Thr Ser Asn Ile
   245 250 255
   Phe Tyr Ser Gln Tyr Asn Val Lys Leu Glu Tyr Ala Glu Ile Tyr Ala
   260 265 270
   Phe Gly Gly Pro Thr Ile Asp Leu Ile Pro Lys Ser Ala Arg Lys Tyr
   275 280 285
   Phe Glu Glu Lys Ala Leu Asp Tyr Tyr Arg Ser Ile Ala Lys Arg Leu
   290 295 300
   Asn Ser Ile Thr Thr Ala Asn Pro Ser Ser Phe Asn Lys Tyr Ile Gly
   305 310 315 320
   Glu Tyr Lys Gln Lys Leu Ile Arg Lys Tyr Arg Phe Val Val Glu Ser
   325 330 335
   Ser Gly Glu Val Thr Val Asn Arg Asn Lys Phe Val Glu Leu Tyr Asn
   340 345 350
   Glu Leu Thr Gln Ile Phe Thr Glu Phe Asn Tyr Ala Lys Ile Tyr Asn
   355 360 365
   Val Gln Asn Arg Lys Ile Tyr Leu Ser Asn Val Tyr Thr Pro Val Thr
   370 375 380
   Ala Asn Ile Leu Asp Asp Asn Val Tyr Asp Ile Gln Asn Gly Phe Asn
   385 390 395 400
   Ile Pro Lys Ser Asn Leu Asn Val Leu Phe Met Gly Gln Asn Leu Ser
   405 410 415
   Arg Asn Pro Ala Leu Arg Lys Val Asn Pro Glu Asn Met Leu Tyr Leu
   420 425 430
   Phe Thr Lys Phe Cys His Lys Ala Ile Asp Gly Arg Ser Leu Tyr Asn
   435 440 445
   Lys Thr Leu Asp Cys Arg Glu Leu Leu Val Lys Asn Thr Asp Leu Pro
   450 455 460
   Phe Ile Gly Asp Ile Ser Asp Val Lys Thr Asp Ile Phe Leu Arg Lys
   465 470 475 480
   Asp Ile Asn Glu Glu Thr Glu Val Ile Tyr Tyr Pro Asp Asn Val Ser
   485 490 495
   Val Asp Gln Val Ile Leu Ser Lys Asn Thr Ser Glu His Gly Gln Leu
   500 505 510
   Asp Leu Leu Tyr Pro Ser Ile Asp Ser Glu Ser Glu Ile Leu Pro Gly
   515 520 525
   Glu Asn Gln Val Phe Tyr Asp Asn Arg Thr Gln Asn Val Asp Tyr Leu
   530 535 540
   Asn Ser Tyr Tyr Tyr Leu Glu Ser Gln Lys Leu Ser Asp Asn Val Glu
   545 550 555 560
   Asp Phe Thr Phe Thr Arg Ser Ile Glu Glu Ala Leu Asp Asn Ser Ala
   565 570 575
   Lys Val Tyr Thr Tyr Phe Pro Thr Leu Ala Asn Lys Val Asn Ala Gly
   580 585 590
   Val Gln Gly Gly Leu Phe Leu Met Trp Ala Asn Asp Val Val Glu Asp
   595 600 605
   Phe Thr Thr Asn Ile Leu Arg Lys Asp Thr Leu Asp Lys Ile Ser Asp
   610 615 620
   Val Ser Ala Ile Ile Pro Tyr Ile Gly Pro Ala Leu Asn Ile Ser Asn
   625 630 635 640
   Ser Val Arg Arg Gly Asn Phe Thr Glu Ala Phe Ala Val Thr Gly Val
   645 650 655
   Thr Ile Leu Leu Glu Ala Phe Pro Glu Phe Thr Ile Pro Ala Leu Gly
               660                 665                 670
   Ala Phe Val Ile Tyr Ser Lys Val Gln Glu Arg Asn Glu Ile Ile Lys
           675                 680                 685
   Thr Ile Asp Asn Cys Leu Glu Gln Arg Ile Lys Arg Trp Lys Asp Ser
       690                 695                 700
   Tyr Glu Trp Met Met Gly Thr Trp Leu Ser Arg Ile Ile Thr Gln Phe
   705                 710                 715                 720
   Asn Asn Ile Ser Tyr Gln Met Tyr Asp Ser Leu Asn Tyr Gln Ala Gly
                   725                 730                 735
   Ala Ile Lys Ala Lys Ile Asp Leu Glu Tyr Lys Lys Tyr Ser Gly Ser
               740                 745                 750
   Asp Lys Glu Asn Ile Lys Ser Gln Val Glu Asn Leu Lys Asn Ser Leu
           755                 760                 765
   Asp Val Lys Ile Ser Glu Ala Met Asn Asn Ile Asn Lys Phe Ile Arg
       770                 775                 780
   Glu Cys Ser Val Thr Tyr Leu Phe Lys Asn Met Leu Pro Lys Val Ile
   785                 790                 795                 800
   Asp Glu Leu Asn Glu Phe Asp Arg Asn Thr Lys Ala Lys Leu Ile Asn
                   805                 810                 815
   Leu Ile Asp Ser His Asn Ile Ile Leu Val Gly Glu Val Asp Lys Leu
               820                 825                 830
   Lys Ala Lys Val Asn Asn Ser Phe Gln Asn Thr Ile Pro Phe Asn Ile
           835                 840                 845
   Phe Ser Tyr Thr Asn Asn Ser Leu Leu Lys Asp Ile Ile Asn Glu Tyr
       850                 855                 860
   Phe Asn Asn Ile Asn Asp Ser Lys Ile Leu Ser Leu Gln Asn Arg Lys
   865                 870                 875                 880
   Asn Thr Leu Val Asp Thr Ser Gly Tyr Asn Ala Glu Val Ser Glu Glu
                   885                 890                 895
   Gly Asp Val Gln Leu Asn Pro Ile Phe Pro Phe Asp Phe Lys Leu Gly
               900                 905                 910
   Ser Ser Gly Glu Asp Arg Gly Lys Val Ile Val Thr Gln Asn Glu Asn
           915                 920                 925
   Ile Val Tyr Asn Ser Met Tyr Glu Ser Phe Ser Ile Ser Phe Trp Ile
       930                 935                 940
   Arg Ile Asn Lys Trp Val Ser Asn Leu Pro Gly Tyr Thr Ile Ile Asp
   945                 950                 955                 960
   Ser Val Lys Asn Asn Ser Gly Trp Ser Ile Gly Ile Ile Ser Asn Phe
                   965                 970                 975
   Leu Val Phe Thr Leu Lys Gln Asn Glu Asp Ser Glu Gln Ser Ile Asn
               980                 985                 990
   Phe Ser Tyr Asp Ile Ser Asn Asn  Ala Pro Gly Tyr Asn  Lys Trp Phe
           995                 1000                1005
   Phe Val  Thr Val Thr Asn Asn  Met Met Gly Asn Met  Lys Ile Tyr
       1010                 1015                 1020
   Ile Asn  Gly Lys Leu Ile Asp  Thr Ile Lys Val Lys  Glu Leu Thr
       1025                 1030                 1035
   Gly Ile  Asn Phe Ser Lys Thr  Ile Thr Phe Glu Ile  Asn Lys Ile
       1040                 1045                 1050
   Pro Asp  Thr Gly Leu Ile Thr  Ser Asp Ser Asp Asn  Ile Asn Met
       1055                 1060                 1065
   Trp Ile  Arg Asp Phe Tyr Ile  Phe Ala Lys Glu Leu  Asp Gly Lys
       1070                 1075                 1080
   Asp Ile  Asn Ile Leu Phe Asn  Ser Leu Gln Tyr Thr  Asn Val Val
       1085                 1090                 1095
   Lys Asp  Tyr Trp Gly Asn Asp  Leu Arg Tyr Asn Lys  Glu Tyr Tyr
       1100                 1105                 1110
   Met Val  Asn Ile Asp Tyr Leu  Asn Arg Tyr Met Tyr  Ala Asn Ser
       1115                 1120                 1125
   Arg Gln  Ile Val Phe Asn Thr  Arg Arg Asn Asn Asn  Asp Phe Asn
       1130                 1135                 1140
   Glu Gly  Tyr Lys Ile Ile Ile  Lys Arg Ile Arg Gly  Asn Thr Asn
       1145                 1150                 1155
   Asp Thr  Arg Val Arg Gly Gly  Asp Ile Leu Tyr Phe  Asp Met Thr
       1160                 1165                 1170
   Ile Asn  Asn Lys Ala Tyr Asn  Leu Phe Met Lys Asn  Glu Thr Met
       1175                 1180                 1185
   Tyr Ala  Asp Asn His Ser Thr  Glu Asp Ile Tyr Ala  Ile Gly Leu
       1190                 1195                 1200
   Arg Glu  Gln Thr Lys Asp Ile  Asn Asp Asn Ile Ile  Phe Gln Ile
       1205                 1210                 1215
   Gln Pro  Met Asn Asn Thr Tyr  Tyr Tyr Ala Ser Gln  Ile Phe Lys
       1220                 1225                 1230
   Ser Asn  Phe Asn Gly Glu Asn  Ile Ser Gly Ile Cys  Ser Ile Gly
       1235                 1240                 1245
   Thr Tyr  Arg Phe Arg Leu Gly  Gly Asp Trp Tyr Arg  His Asn Tyr
       1250                 1255                 1260
   Leu Val  Pro Thr Val Lys Gln  Gly Asn Tyr Ala Ser  Leu Leu Glu
       1265                 1270                 1275
   Ser Thr  Ser Thr His Trp Gly  Phe Val Pro Val Ser  Glu
       1280                 1285                 1290
<210> 35
   <211> 1276
   <212> PRT
   <213> Clostridium botulinum
<400> 35
   Met Thr Trp Pro Val Lys Asp Phe Asn Tyr Ser Asp Pro Val Asn Asp
   1               5                   10                  15
   Asn Asp Ile Leu Tyr Leu Arg Ile Pro Gln Asn Lys Leu Ile Thr Thr
               20                  25                  30
   Pro Val Lys Ala Phe Met Ile Thr Gln Asn Ile Trp Val Ile Pro Glu
           35                  40                  45
   Arg Phe Ser Ser Asp Thr Asn Pro Ser Leu Ser Lys Pro Pro Arg Pro
       50                  55                  60
   Thr Ser Lys Tyr Gln Ser Tyr Tyr Asp Pro Ser Tyr Leu Ser Thr Asp
   65                  70                  75                  80
   Glu Gln Lys Asp Thr Phe Leu Lys Gly Ile Ile Lys Leu Phe Lys Arg
                   85                  90                  95
   Ile Asn Glu Arg Asp Ile Gly Lys Lys Leu Ile Asn Tyr Leu Val Val
               100                 105                 110
   Gly Ser Pro Phe Met Gly Asp Ser Ser Thr Pro Glu Asp Thr Phe Asp
           115                 120                 125
   Phe Thr Arg His Thr Thr Asn Ile Ala Val Glu Lys Phe Glu Asn Gly
       130                 135                 140
   Ser Trp Lys Val Thr Asn Ile Ile Thr Pro Ser Val Leu Ile Phe Gly
   145                 150                 155                 160
   Pro Leu Pro Asn Ile Leu Asp Tyr Thr Ala Ser Leu Thr Leu Gln Gly
                   165                 170                 175
   Gln Gln Ser Asn Pro Ser Phe Glu Gly Phe Gly Thr Leu Ser Ile Leu
               180                 185                 190
   Lys Val Ala Pro Glu Phe Leu Leu Thr Phe Ser Asp Val Thr Ser Asn
           195                 200                 205
   Gln Ser Ser Ala Val Leu Gly Lys Ser Ile Phe Cys Met Asp Pro Val
       210                 215                 220
   Ile Ala Leu Met His Glu Leu Thr His Ser Leu His Gln Leu Tyr Gly
   225                 230                 235                 240
   Ile Asn Ile Pro Ser Asp Lys Arg Ile Arg Pro Gln Val Ser Glu Gly
                   245                 250                 255
   Phe Phe Ser Gln Asp Gly Pro Asn Val Gln Phe Glu Glu Leu Tyr Thr
               260                 265                 270
   Phe Gly Gly Leu Asp Val Glu Ile Ile Pro Gln Ile Glu Arg Ser Gln
           275                 280                 285
   Leu Arg Glu Lys Ala Leu Gly His Tyr Lys Asp Ile Ala Lys Arg Leu
       290                 295                 300
   Asn Asn Ile Asn Lys Thr Ile Pro Ser Ser Trp Ile Ser Asn Ile Asp
   305                 310                 315                 320
   Lys Tyr Lys Lys Ile Phe Ser Glu Lys Tyr Asn Phe Asp Lys Asp Asn
   325 330 335
   Thr Gly Asn Phe Val Val Asn Ile Asp Lys Phe Asn Ser Leu Tyr Ser
   340 345 350
   Asp Leu Thr Asn Val Met Ser Glu Val Val Tyr Ser Ser Gln Tyr Asn
   355 360 365
   Val Lys Asn Arg Thr His Tyr Phe Ser Arg His Tyr Leu Pro Val Phe
   370 375 380
   Ala Asn Ile Leu Asp Asp Asn Ile Tyr Thr Ile Arg Asp Gly Phe Asn
   385 390 395 400
   Leu Thr Asn Lys Gly Phe Asn Ile Glu Asn Ser Gly Gln Asn Ile Glu
   405 410 415
   Arg Asn Pro Ala Leu Gln Lys Leu Ser Ser Glu Ser Val Val Asp Leu
   420 425 430
   Phe Thr Lys Val Cys Leu Arg Leu Thr Lys Asn Ser Arg Asp Asp Ser
   435 440 445
   Thr Cys Ile Lys Val Lys Asn Asn Arg Leu Pro Tyr Val Ala Asp Lys
   450 455 460
   Asp Ser Ile Ser Gln Glu Ile Phe Glu Asn Lys Ile Ile Thr Asp Glu
   465 470 475 480
   Thr Asn Val Gln Asn Tyr Ser Asp Lys Phe Ser Leu Asp Glu Ser Ile
   485 490 495
   Leu Asp Gly Gln Val Pro Ile Asn Pro Glu Ile Val Asp Pro Leu Leu
   500 505 510
   Pro Asn Val Asn Met Glu Pro Leu Asn Leu Pro Gly Glu Glu Ile Val
   515 520 525
   Phe Tyr Asp Asp Ile Thr Lys Tyr Val Asp Tyr Leu Asn Ser Tyr Tyr
   530 535 540
   Tyr Leu Glu Ser Gln Lys Leu Ser Asn Asn Val Glu Asn Ile Thr Leu
   545 550 555 560
   Thr Thr Ser Val Glu Glu Ala Leu Gly Tyr Ser Asn Lys Ile Tyr Thr
   565 570 575
   Phe Leu Pro Ser Leu Ala Glu Lys Val Asn Lys Gly Val Gln Ala Gly
   580 585 590
   Leu Phe Leu Asn Trp Ala Asn Glu Val Val Glu Asp Phe Thr Thr Asn
   595 600 605
   Ile Met Lys Lys Asp Thr Leu Asp Lys Ile Ser Asp Val Ser Val Ile
   610 615 620
   Ile Pro Tyr Ile Gly Pro Ala Leu Asn Ile Gly Asn Ser Ala Leu Arg
   625 630 635 640
   Gly Asn Phe Asn Gln Ala Phe Ala Thr Ala Gly Val Ala Phe Leu Leu
   645 650 655
   Glu Gly Phe Pro Glu Phe Thr Ile Pro Ala Leu Gly Val Phe Thr Phe
   660 665 670
   Tyr Ser Ser Ile Gln Glu Arg Glu Lys Ile Ile Lys Thr Ile Glu Asn
   675 680 685
   Cys Leu Glu Gln Arg Val Lys Arg Trp Lys Asp Ser Tyr Gln Trp Met
   690 695 700
   Val Ser Asn Trp Leu Ser Arg Ile Thr Thr Gln Phe Asn His Ile Asn
   705 710 715 720
   Tyr Gln Met Tyr Asp Ser Leu Ser Tyr Gln Ala Asp Ala Ile Lys Ala
   725 730 735
   Lys Ile Asp Leu Glu Tyr Lys Lys Tyr Ser Gly Ser Asp Lys Glu Asn
   740 745 750
   Ile Lys Ser Gln Val Glu Asn Leu Lys Asn Ser Leu Asp Val Lys Ile
   755 760 765
   Ser Glu Ala Met Asn Asn Ile Asn Lys Phe Ile Arg Glu Cys Ser Val
   770 775 780
   Thr Tyr Leu Phe Lys Asn Met Leu Pro Lys Val Ile Asp Glu Leu Asn
   785 790 795 800
   Lys Phe Asp Leu Arg Thr Lys Thr Glu Leu Ile Asn Leu Ile Asp Ser
   805 810 815
   His Asn Ile Ile Leu Val Gly Glu Val Asp Arg Leu Lys Ala Lys Val
   820 825 830
   Asn Glu Ser Phe Glu Asn Thr Met Pro Phe Asn Ile Phe Ser Tyr Thr
   835 840 845
   Asn Asn Ser Leu Leu Lys Asp Ile Ile Asn Glu Tyr Phe Asn Ser Ile
   850 855 860
   Asn Asp Ser Lys Ile Leu Ser Leu Gln Asn Lys Lys Asn Ala Leu Val
   865 870 875 880
   Asp Thr Ser Gly Tyr Asn Ala Glu Val Arg Val Gly Asp Asn Val Gln
   885 890 895
   Leu Asn Thr Ile Tyr Thr Asn Asp Phe Lys Leu Ser Ser Ser Gly Asp
   900 905 910
   Lys Ile Ile Val Asn Leu Asn Asn Asn Ile Leu Tyr Ser Ala Ile Tyr
   915 920 925
   Glu Asn Ser Ser Val Ser Phe Trp Ile Lys Ile Ser Lys Asp Leu Thr
   930 935 940
   Asn Ser His Asn Glu Tyr Thr Ile Ile Asn Ser Ile Glu Gln Asn Ser
   945 950 955 960
   Gly Trp Lys Leu Cys Ile Arg Asn Gly Asn Ile Glu Trp Ile Leu Gln
   965 970 975
   Asp Val Asn Arg Lys Tyr Lys Ser Leu Ile Phe Asp Tyr Ser Glu Ser
   980 985 990
   Leu Ser His Thr Gly Tyr Thr Asn Lys Trp Phe Phe Val Thr Ile Thr
   995 1000 1005
   Asn Asn Ile Met Gly Tyr Met Lys Leu Tyr Ile Asn Gly Glu Leu
   1010 1015 1020
   Lys Gln Ser Gln Lys Ile Glu Asp Leu Asp Glu Val Lys Leu Asp
   1025 1030 1035
   Lys Thr Ile Val Phe Gly Ile Asp Glu Asn Ile Asp Glu Asn Gln
   1040 1045 1050
   Met Leu Trp Ile Arg Asp Phe Asn Ile Phe Ser Lys Glu Leu Ser
   1055 1060 1065
   Asn Glu Asp Ile Asn Ile Val Tyr Glu Gly Gln Ile Leu Arg Asn
   1070 1075 1080
   Val Ile Lys Asp Tyr Trp Gly Asn Pro Leu Lys Phe Asp Thr Glu
   1085 1090 1095
   Tyr Tyr Ile Ile Asn Asp Asn Tyr Ile Asp Arg Tyr Ile Ala Pro
   1100 1105 1110
   Glu Ser Asn Val Leu Val Leu Val Gln Tyr Pro Asp Arg Ser Lys
   1115 1120 1125
   Leu Tyr Thr Gly Asn Pro Ile Thr Ile Lys Ser Val Ser Asp Lys
   1130 1135 1140
   Asn Pro Tyr Ser Arg Ile Leu Asn Gly Asp Asn Ile Ile Leu His
   1145 1150 1155
   Met Leu Tyr Asn Ser Arg Lys Tyr Met Ile Ile Arg Asp Thr Asp
   1160 1165 1170
   Thr Ile Tyr Ala Thr Gln Gly Gly Glu Cys Ser Gln Asn Cys Val
   1175 1180 1185
   Tyr Ala Leu Lys Leu Gln Ser Asn Leu Gly Asn Tyr Gly Ile Gly
   1190 1195 1200
   Ile Phe Ser Ile Lys Asn Ile Val Ser Lys Asn Lys Tyr Cys Ser
   1205 1210 1215
   Gln Ile Phe Ser Ser Phe Arg Glu Asn Thr Met Leu Leu Ala Asp
   1220 1225 1230
   Ile Tyr Lys Pro Trp Arg Phe Ser Phe Lys Asn Ala Tyr Thr Pro
   1235 1240 1245
   Val Ala Val Thr Asn Tyr Glu Thr Lys Leu Leu Ser Thr Ser Ser
   1250 1255 1260
   Phe Trp Lys Phe Ile Ser Arg Asp Pro Gly Trp Val Glu
   1265 1270 1275
<210> 36
   <211> 1251
   <212> PRT
   <213> Clostridium botulinum
<400> 36
   Met Pro Lys Ile Asn Ser Phe Asn Tyr Asn Asp Pro Val Asn Asp Arg
   1 5 10 15
   Thr Ile Leu Tyr Ile Lys Pro Gly Gly Cys Gln Glu Phe Tyr Lys Ser
               20                  25                  30
   Phe Asn Ile Met Lys Asn Ile Trp Ile Ile Pro Glu Arg Asn Val Ile
           35                  40                  45
   Gly Thr Thr Pro Gln Asp Phe His Pro Pro Thr Ser Leu Lys Asn Gly
       50                  55                  60
   Asp Ser Ser Tyr Tyr Asp Pro Asn Tyr Leu Gln Ser Asp Glu Glu Lys
   65                  70                  75                  80
   Asp Arg Phe Leu Lys Ile Val Thr Lys Ile Phe Asn Arg Ile Asn Asn
                   85                  90                  95
   Asn Leu Ser Gly Gly Ile Leu Leu Glu Glu Leu Ser Lys Ala Asn Pro
               100                 105                 110
   Tyr Leu Gly Asn Asp Asn Thr Pro Asp Asn Gln Phe His Ile Gly Asp
           115                 120                 125
   Ala Ser Ala Val Glu Ile Lys Phe Ser Asn Gly Ser Gln Asp Ile Leu
       130                 135                 140
   Leu Pro Asn Val Ile Ile Met Gly Ala Glu Pro Asp Leu Phe Glu Thr
   145                 150                 155                 160
   Asn Ser Ser Asn Ile Ser Leu Arg Asn Asn Tyr Met Pro Ser Asn His
                   165                 170                 175
   Arg Phe Gly Ser Ile Ala Ile Val Thr Phe Ser Pro Glu Tyr Ser Phe
               180                 185                 190
   Arg Phe Asn Asp Asn Cys Met Asn Glu Phe Ile Gln Asp Pro Ala Leu
           195                 200                 205
   Thr Leu Met His Glu Leu Ile His Ser Leu His Gly Leu Tyr Gly Ala
       210                 215                 220
   Lys Gly Ile Thr Thr Lys Tyr Thr Ile Thr Gln Lys Gln Asn Pro Leu
   225                 230                 235                 240
   Ile Thr Asn Ile Arg Gly Thr Asn Ile Glu Glu Phe Leu Thr Phe Gly
                   245                 250                 255
   Gly Thr Asp Leu Asn Ile Ile Thr Ser Ala Gln Ser Asn Asp Ile Tyr
   260 265 270
   Thr Asn Leu Leu Ala Asp Tyr Lys Lys Ile Ala Ser Lys Leu Ser Lys
   275 280 285
   Val Gln Val Ser Asn Pro Leu Leu Asn Pro Tyr Lys Asp Val Phe Glu
   290 295 300
   Ala Lys Tyr Gly Leu Asp Lys Asp Ala Ser Gly Ile Tyr Ser Val Asn
   305 310 315 320
   Ile Asn Lys Phe Asn Asp Ile Phe Lys Lys Leu Tyr Ser Phe Thr Glu
   325 330 335
   Phe Asp Leu Arg Thr Lys Phe Gln Val Lys Cys Arg Gln Thr Tyr Ile
   340 345 350
   Gly Gln Tyr Lys Tyr Phe Lys Leu Ser Asn Leu Leu Asn Asp Ser Ile
   355 360 365
   Tyr Asn Ile Ser Glu Gly Tyr Asn Ile Asn Asn Leu Lys Val Asn Phe
   370 375 380
   Arg Gly Gln Asn Ala Asn Leu Asn Pro Arg Ile Ile Thr Pro Ile Thr
   385 390 395 400
   Gly Arg Gly Leu Val Lys Lys Ile Ile Arg Phe Cys Lys Asn Ile Val
   405 410 415
   Ser Val Lys Gly Ile Arg Lys Ser Ile Cys Ile Glu Ile Asn Asn Gly
   420 425 430
   Glu Leu Phe Phe Val Ala Ser Glu Asn Ser Tyr Asn Asp Asp Asn Ile
   435 440 445
   Asn Thr Pro Lys Glu Ile Asp Asp Thr Val Thr Ser Asn Asn Asn Tyr
   450 455 460
   Glu Asn Asp Leu Asp Gln Val Ile Leu Asn Phe Asn Ser Glu Ser Ala
   465 470 475 480
   Pro Gly Leu Ser Asp Glu Lys Leu Asn Leu Thr Ile Gln Asn Asp Ala
   485 490 495
   Tyr Ile Pro Lys Tyr Asp Ser Asn Gly Thr Ser Asp Ile Glu Gln His
   500 505 510
   Asp Val Asn Glu Leu Asn Val Phe Phe Tyr Leu Asp Ala Gln Lys Val
   515 520 525
   Pro Glu Gly Glu Asn Asn Val Asn Leu Thr Ser Ser Ile Asp Thr Ala
   530 535 540
   Leu Leu Glu Gln Pro Lys Ile Tyr Thr Phe Phe Ser Ser Glu Phe Ile
   545 550 555 560
   Asn Asn Val Asn Lys Pro Val Gln Ala Ala Leu Phe Val Ser Trp Ile
   565 570 575
   Gln Gln Val Leu Val Asp Phe Thr Thr Glu Ala Asn Gln Lys Ser Thr
   580 585 590
   Val Asp Lys Ile Ala Asp Ile Ser Ile Val Val Pro Tyr Ile Gly Leu
   595 600 605
   Ala Leu Asn Ile Gly Asn Glu Ala Gln Lys Gly Asn Phe Lys Asp Ala
   610 615 620
   Leu Glu Leu Leu Gly Ala Gly Ile Leu Leu Glu Phe Glu Pro Glu Leu
   625 630 635 640
   Leu Ile Pro Thr Ile Leu Val Phe Thr Ile Lys Ser Phe Leu Gly Ser
   645 650 655
   Ser Asp Asn Lys Asn Lys Val Ile Lys Ala Ile Asn Asn Ala Leu Lys
   660 665 670
   Glu Arg Asp Glu Lys Trp Lys Glu Val Tyr Ser Phe Ile Val Ser Asn
   675 680 685
   Trp Met Thr Lys Ile Asn Thr Gln Phe Asn Lys Arg Lys Glu Gln Met
   690 695 700
   Tyr Gln Ala Leu Gln Asn Gln Val Asn Ala Ile Lys Thr Ile Ile Glu
   705 710 715 720
   Ser Lys Tyr Asn Ser Tyr Thr Leu Glu Glu Lys Asn Glu Leu Thr Asn
   725 730 735
   Lys Tyr Asp Ile Lys Gln Ile Glu Asn Glu Leu Asn Gln Lys Val Ser
   740 745 750
   Ile Ala Met Asn Asn Ile Asp Arg Phe Leu Thr Glu Ser Ser Ile Ser
   755 760 765
   Tyr Leu Met Lys Ile Ile Asn Glu Val Lys Ile Asn Lys Leu Arg Glu
       770                 775                 780
   Tyr Asp Glu Asn Val Lys Thr Tyr Leu Leu Asn Tyr Ile Ile Gln His
   785                 790                 795                 800
   Gly Ser Ile Leu Gly Glu Ser Gln Gln Glu Leu Asn Ser Met Val Thr
                   805                 810                 815
   Asp Thr Leu Asn Asn Ser Ile Pro Phe Lys Leu Ser Ser Tyr Thr Asp
               820                 825                 830
   Asp Lys Ile Leu Ile Ser Tyr Phe Asn Lys Phe Phe Lys Arg Ile Lys
           835                 840                 845
   Ser Ser Ser Val Leu Asn Met Arg Tyr Lys Asn Asp Lys Tyr Val Asp
       850                 855                 860
   Thr Ser Gly Tyr Asp Ser Asn Ile Asn Ile Asn Gly Asp Val Tyr Lys
   865                 870                 875                 880
   Tyr Pro Thr Asn Lys Asn Gln Phe Gly Ile Tyr Asn Asp Lys Leu Ser
                   885                 890                 895
   Glu Val Asn Ile Ser Gln Asn Asp Tyr Ile Ile Tyr Asp Asn Lys Tyr
               900                 905                 910
   Lys Asn Phe Ser Ile Ser Phe Trp Val Arg Ile Pro Asn Tyr Asp Asn
           915                 920                 925
   Lys Ile Val Asn Val Asn Asn Glu Tyr Thr Ile Ile Asn Cys Met Arg
       930                 935                 940
   Asp Asn Asn Ser Gly Trp Lys Val Ser Leu Asn His Asn Glu Ile Ile
   945                 950                 955                 960
   Trp Thr Phe Glu Asp Asn Arg Gly Ile Asn Gln Lys Leu Ala Phe Asn
                   965                 970                 975
   Tyr Gly Asn Ala Asn Gly Ile Ser Asp Tyr Ile Asn Lys Trp Ile Phe
               980                 985                 990
   Val Thr Ile Thr Asn Asp Arg Leu  Gly Asp Ser Lys Leu  Tyr Ile Asn
           995                 1000                 1005
   Gly Asn  Leu Ile Asp Gln Lys  Ser Ile Leu Asn Leu  Gly Asn Ile
       1010                 1015                 1020
   His Val Ser Asp Asn Ile Leu Phe Lys Ile Val Asn Cys Ser Tyr
   1025 1030 1035
   Thr Arg Tyr Ile Gly Ile Arg Tyr Phe Asn Ile Phe Asp Lys Glu
   1040 1045 1050
   Leu Asp Glu Thr Glu Ile Gln Thr Leu Tyr Ser Asn Glu Pro Asn
   1055 1060 1065
   Thr Asn Ile Leu Lys Asp Phe Trp Gly Asn Tyr Leu Leu Tyr Asp
   1070 1075 1080
   Lys Glu Tyr Tyr Leu Leu Asn Val Leu Lys Pro Asn Asn Phe Ile
   1085 1090 1095
   Asp Arg Arg Lys Asp Ser Thr Leu Ser Ile Asn Asn Ile Arg Ser
   1100 1105 1110
   Thr Ile Leu Leu Ala Asn Arg Leu Tyr Ser Gly Ile Lys Val Lys
   1115 1120 1125
   Ile Gln Arg Val Asn Asn Ser Ser Thr Asn Asp Asn Leu Val Arg
   1130 1135 1140
   Lys Asn Asp Gln Val Tyr Ile Asn Phe Val Ala Ser Lys Thr His
   1145 1150 1155
   Leu Phe Pro Leu Tyr Ala Asp Thr Ala Thr Thr Asn Lys Glu Lys
   1160 1165 1170
   Thr Ile Lys Ile Ser Ser Ser Gly Asn Arg Phe Asn Gln Val Val
   1175 1180 1185
   Val Met Asn Ser Val Gly Asn Cys Thr Met Asn Phe Lys Asn Asn
   1190 1195 1200
   Asn Gly Asn Asn Ile Gly Leu Leu Gly Phe Lys Ala Asp Thr Val
   1205 1210 1215
   Val Ala Ser Thr Trp Tyr Tyr Thr His Met Arg Asp His Thr Asn
   1220 1225 1230
   Ser Asn Gly Cys Phe Trp Asn Phe Ile Ser Glu Glu His Gly Trp
   1235 1240 1245
   Gln Glu Lys
   1250
<210> 37
   <211> 1278
   <212> PRT
   <213> Clostridium botulinum
<400> 37
   Met Pro Val Val Ile Asn Ser Phe Asn Tyr Asn Asp Pro Val Asn Asp
   1               5                   10                  15
   Asp Thr Ile Leu Tyr Met Gln Ile Pro Tyr Glu Glu Lys Ser Lys Lys
               20                  25                  30
   Tyr Tyr Lys Ala Phe Glu Ile Met Arg Asn Val Trp Ile Ile Pro Glu
           35                  40                  45
   Arg Asn Thr Ile Gly Thr Asp Pro Ser Asp Phe Asp Pro Pro Ala Ser
       50                  55                  60
   Leu Glu Asn Gly Ser Ser Ala Tyr Tyr Asp Pro Asn Tyr Leu Thr Thr
   65                  70                  75                  80
   Asp Ala Glu Lys Asp Arg Tyr Leu Lys Thr Thr Ile Lys Leu Phe Lys
                   85                  90                  95
   Arg Ile Asn Ser Asn Pro Ala Gly Glu Val Leu Leu Gln Glu Ile Ser
               100                 105                 110
   Tyr Ala Lys Pro Tyr Leu Gly Asn Glu His Thr Pro Ile Asn Glu Phe
           115                 120                 125
   His Pro Val Thr Arg Thr Thr Ser Val Asn Ile Lys Ser Ser Thr Asn
       130                 135                 140
   Val Lys Ser Ser Ile Ile Leu Asn Leu Leu Val Leu Gly Ala Gly Pro
   145                 150                 155                 160
   Asp Ile Phe Glu Asn Ser Ser Tyr Pro Val Arg Lys Leu Met Asp Ser
                   165                 170                 175
   Gly Gly Val Tyr Asp Pro Ser Asn Asp Gly Phe Gly Ser Ile Asn Ile
               180                 185                 190
   Val Thr Phe Ser Pro Glu Tyr Glu Tyr Thr Phe Asn Asp Ile Ser Gly
           195                 200                 205
   Gly Tyr Asn Ser Ser Thr Glu Ser Phe Ile Ala Asp Pro Ala Ile Ser
       210                 215                 220
   Leu Ala His Glu Leu Ile His Ala Leu His Gly Leu Tyr Gly Ala Arg
   225                 230                 235                 240
   Gly Val Thr Tyr Lys Glu Thr Ile Lys Val Lys Gln Ala Pro Leu Met
                   245                 250                 255
   Ile Ala Glu Lys Pro Ile Arg Leu Glu Glu Phe Leu Thr Phe Gly Gly
               260                 265                 270
   Gln Asp Leu Asn Ile Ile Thr Ser Ala Met Lys Glu Lys Ile Tyr Asn
           275                 280                 285
   Asn Leu Leu Ala Asn Tyr Glu Lys Ile Ala Thr Arg Leu Ser Arg Val
       290                 295                 300
   Asn Ser Ala Pro Pro Glu Tyr Asp Ile Asn Glu Tyr Lys Asp Tyr Phe
   305                 310                 315                 320
   Gln Trp Lys Tyr Gly Leu Asp Lys Asn Ala Asp Gly Ser Tyr Thr Val
                   325                 330                 335
   Asn Glu Asn Lys Phe Asn Glu Ile Tyr Lys Lys Leu Tyr Ser Phe Thr
               340                 345                 350
   Glu Ile Asp Leu Ala Asn Lys Phe Lys Val Lys Cys Arg Asn Thr Tyr
           355                 360                 365
   Phe Ile Lys Tyr Gly Phe Leu Lys Val Pro Asn Leu Leu Asp Asp Asp
       370                 375                 380
   Ile Tyr Thr Val Ser Glu Gly Phe Asn Ile Gly Asn Leu Ala Val Asn
   385                 390                 395                 400
   Asn Arg Gly Gln Asn Ile Lys Leu Asn Pro Lys Ile Ile Asp Ser Ile
                   405                 410                 415
   Pro Asp Lys Gly Leu Val Glu Lys Ile Val Lys Phe Cys Lys Ser Val
               420                 425                 430
   Ile Pro Arg Lys Gly Thr Lys Ala Pro Pro Arg Leu Cys Ile Arg Val
           435                 440                 445
   Asn Asn Arg Glu Leu Phe Phe Val Ala Ser Glu Ser Ser Tyr Asn Glu
       450                 455                 460
   Asn Asp Ile Asn Thr Pro Lys Glu Ile Asp Asp Thr Thr Asn Leu Asn
   465                 470                 475                 480
   Asn Asn Tyr Arg Asn Asn Leu Asp Glu Val Ile Leu Asp Tyr Asn Ser
                   485                 490                 495
   Glu Thr Ile Pro Gln Ile Ser Asn Gln Thr Leu Asn Thr Leu Val Gln
               500                 505                 510
   Asp Asp Ser Tyr Val Pro Arg Tyr Asp Ser Asn Gly Thr Ser Glu Ile
           515                 520                 525
   Glu Glu His Asn Val Val Asp Leu Asn Val Phe Phe Tyr Leu His Ala
       530                 535                 540
   Gln Lys Val Pro Glu Gly Glu Thr Asn Ile Ser Leu Thr Ser Ser Ile
   545                 550                 555                 560
   Asp Thr Ala Leu Ser Glu Glu Ser Gln Val Tyr Thr Phe Phe Ser Ser
                   565                 570                 575
   Glu Phe Ile Asn Thr Ile Asn Lys Pro Val His Ala Ala Leu Phe Ile
               580                 585                 590
   Ser Trp Ile Asn Gln Val Ile Arg Asp Phe Thr Thr Glu Ala Thr Gln
           595                 600                 605
   Lys Ser Thr Phe Asp Lys Ile Ala Asp Ile Ser Leu Val Val Pro Tyr
       610                 615                 620
   Val Gly Leu Ala Leu Asn Ile Gly Asn Glu Val Gln Lys Glu Asn Phe
   625                 630                 635                 640
   Lys Glu Ala Phe Glu Leu Leu Gly Ala Gly Ile Leu Leu Glu Phe Val
                   645                 650                 655
   Pro Glu Leu Leu Ile Pro Thr Ile Leu Val Phe Thr Ile Lys Ser Phe
               660                 665                 670
   Ile Gly Ser Ser Glu Asn Lys Asn Lys Ile Ile Lys Ala Ile Asn Asn
           675                 680                 685
   Ser Leu Met Glu Arg Glu Thr Lys Trp Lys Glu Ile Tyr Ser Trp Ile
       690                 695                 700
   Val Ser Asn Trp Leu Thr Arg Ile Asn Thr Gln Phe Asn Lys Arg Lys
   705                 710                 715                 720
   Glu Gln Met Tyr Gln Ala Leu Gln Asn Gln Val Asp Ala Ile Lys Thr
   725 730 735
   Val Ile Glu Tyr Lys Tyr Asn Asn Tyr Thr Ser Asp Glu Arg Asn Arg
   740 745 750
   Leu Glu Ser Glu Tyr Asn Ile Asn Asn Ile Arg Glu Glu Leu Asn Lys
   755 760 765
   Lys Val Ser Leu Ala Met Glu Asn Ile Glu Arg Phe Ile Thr Glu Ser
   770 775 780
   Ser Ile Phe Tyr Leu Met Lys Leu Ile Asn Glu Ala Lys Val Ser Lys
   785 790 795 800
   Leu Arg Glu Tyr Asp Glu Gly Val Lys Glu Tyr Leu Leu Asp Tyr Ile
   805 810 815
   Ser Glu His Arg Ser Ile Leu Gly Asn Ser Val Gln Glu Leu Asn Asp
   820 825 830
   Leu Val Thr Ser Thr Leu Asn Asn Ser Ile Pro Phe Glu Leu Ser Ser
   835 840 845
   Tyr Thr Asn Asp Lys Ile Leu Ile Leu Tyr Phe Asn Lys Leu Tyr Lys
   850 855 860
   Lys Ile Lys Asp Asn Ser Ile Leu Asp Met Arg Tyr Glu Asn Asn Lys
   865 870 875 880
   Phe Ile Asp Ile Ser Gly Tyr Gly Ser Asn Ile Ser Ile Asn Gly Asp
   885 890 895
   Val Tyr Ile Tyr Ser Thr Asn Arg Asn Gln Phe Gly Ile Tyr Ser Ser
   900 905 910
   Lys Pro Ser Glu Val Asn Ile Ala Gln Asn Asn Asp Ile Ile Tyr Asn
   915 920 925
   Gly Arg Tyr Gln Asn Phe Ser Ile Ser Phe Trp Val Arg Ile Pro Lys
   930 935 940
   Tyr Phe Asn Lys Val Asn Leu Asn Asn Glu Tyr Thr Ile Ile Asp Cys
   945 950 955 960
   Ile Arg Asn Asn Asn Ser Gly Trp Lys Ile Ser Leu Asn Tyr Asn Lys
   965 970 975
   Ile Ile Trp Thr Leu Gln Asp Thr Ala Gly Asn Asn Gln Lys Leu Val
               980                 985                 990
   Phe Asn Tyr Thr Gln Met Ile Ser  Ile Ser Asp Tyr Ile  Asn Lys Trp
           995                 1000                 1005
   Ile Phe  Val Thr Ile Thr Asn  Asn Arg Leu Gly Asn  Ser Arg Ile
       1010                 1015                 1020
   Tyr Ile  Asn Gly Asn Leu Ile  Asp Glu Lys Ser Ile  Ser Asn Leu
       1025                 1030                 1035
   Gly Asp  Ile His Val Ser Asp  Asn Ile Leu Phe Lys  Ile Val Gly
       1040                 1045                 1050
   Cys Asn  Asp Thr Arg Tyr Val  Gly Ile Arg Tyr Phe  Lys Val Phe
       1055                 1060                 1065
   Asp Thr  Glu Leu Gly Lys Thr  Glu Ile Glu Thr Leu  Tyr Ser Asp
       1070                 1075                 1080
   Glu Pro  Asp Pro Ser Ile Leu  Lys Asp Phe Trp Gly  Asn Tyr Leu
       1085                 1090                 1095
   Leu Tyr  Asn Lys Arg Tyr Tyr  Leu Leu Asn Leu Leu  Arg Thr Asp
       1100                 1105                 1110
   Lys Ser  Ile Thr Gln Asn Ser  Asn Phe Leu Asn Ile  Asn Gln Gln
       1115                 1120                 1125
   Arg Gly  Val Tyr Gln Lys Pro  Asn Ile Phe Ser Asn  Thr Arg Leu
       1130                 1135                 1140
   Tyr Thr  Gly Val Glu Val Ile  Ile Arg Lys Asn Gly  Ser Thr Asp
       1145                 1150                 1155
   Ile Ser  Asn Thr Asp Asn Phe  Val Arg Lys Asn Asp  Leu Ala Tyr
       1160                 1165                 1170
   Ile Asn  Val Val Asp Arg Asp  Val Glu Tyr Arg Leu  Tyr Ala Asp
       1175                 1180                 1185
   Ile Ser  Ile Ala Lys Pro Glu  Lys Ile Ile Lys Leu  Ile Arg Thr
       1190                 1195                 1200
   Ser Asn  Ser Asn Asn Ser Leu  Gly Gln Ile Ile Val  Met Asp Ser
   1205 1210 1215
   Ile Gly Asn Asn Cys Thr Met Asn Phe Gln Asn Asn Asn Gly Gly
   1220 1225 1230
   Asn Ile Gly Leu Leu Gly Phe His Ser Asn Asn Leu Val Ala Ser
   1235 1240 1245
   Ser Trp Tyr Tyr Asn Asn Ile Arg Lys Asn Thr Ser Ser Asn Gly
   1250 1255 1260
   Cys Phe Trp Ser Phe Ile Ser Lys Glu His Gly Trp Gln Glu Asn
   1265 1270 1275
<210> 38
   <211> 1277
   <212> PRT
   <213> Clostridium botulinum
<400> 38
   Met Pro Val Glu Ile Asn Ser Phe Asn Tyr Asp Asp Leu Val Asn Asp
   1               5                   10                  15
   Asn Thr Ile Leu Tyr Ile Arg Pro Pro Tyr Tyr Glu Arg Ser Asn Thr
               20                  25                  30
   Tyr Phe Lys Ala Phe Asn Ile Met Glu Asn Val Trp Ile Ile Pro Glu
           35                  40                  45
   Arg Tyr Arg Leu Gly Ile Glu Ala Ser Lys Phe Asp Pro Pro Asp Ser
       50                  55                  60
   Leu Lys Ala Gly Ser Asp Gly Tyr Phe Asp Pro Asn Tyr Leu Ser Thr
   65                  70                  75                  80
   Asn Thr Glu Lys Asn Arg Tyr Leu Gln Ile Met Ile Lys Leu Phe Lys
                   85                  90                  95
   Arg Ile Asn Ser Asn Glu Ala Gly Lys Ile Leu Leu Asn Gln Ile Lys
               100                 105                 110
   Asp Ala Ile Pro Tyr Leu Gly Asn Ser Tyr Thr Ala Glu Asp Gln Phe
           115                 120                 125
   Thr Thr Asn Asn Arg Thr Ile Ser Phe Asn Val Arg Leu Ala Asn Gly
       130                 135                 140
   Thr Ile Glu Gln Glu Met Ala Asn Leu Ile Ile Trp Gly Pro Gly Pro
   145 150 155 160
   Asp Leu Thr Thr Asn Arg Thr Gly Gly Thr Thr Tyr Thr Pro Ala Gln
   165 170 175
   Ser Leu Glu Ala Ile Pro Tyr Lys Glu Gly Phe Gly Ser Ile Met Thr
   180 185 190
   Ile Glu Phe Ser Pro Glu Tyr Ala Thr Ala Phe Asn Asp Ile Ser Leu
   195 200 205
   Thr Ser His Ala Pro Ser Leu Phe Ile Lys Asp Pro Ala Leu Ile Leu
   210 215 220
   Met His Glu Leu Ile His Val Leu His Gly Leu Tyr Gly Thr Tyr Thr
   225 230 235 240
   Thr Gly Phe Lys Ile Lys Pro Asn Ile Thr Glu Pro Tyr Met Glu Val
   245 250 255
   Thr Lys Pro Ile Thr Ser Gly Glu Phe Leu Thr Phe Gly Gly Asn Asp
   260 265 270
   Val Asn Lys Ile Pro Gln Leu Ile Gln Ser Gln Leu Arg Ser Lys Val
   275 280 285
   Leu Asp Asp Tyr Glu Lys Ile Ala Ser Arg Leu Asn Lys Val Asn Arg
   290 295 300
   Ala Thr Ala Glu Ile Asn Ile Asp Lys Phe Lys Tyr Ser Tyr Gln Leu
   305 310 315 320
   Lys Tyr Gln Phe Val Lys Asp Ser Asn Gly Val Tyr Ser Val Asp Leu
   325 330 335
   Asp Lys Phe Asn Lys Leu Tyr Asp Lys Ile Tyr Ser Phe Thr Glu Phe
   340 345 350
   Asn Leu Ala His Glu Phe Lys Ile Lys Thr Arg Asn Ser Tyr Leu Ala
   355 360 365
   Lys Asn Phe Gly Pro Phe Tyr Leu Pro Asn Leu Leu Asp Asn Ser Ile
   370 375 380
   Tyr Asn Glu Ala Asp Gly Phe Asn Ile Gly Asp Leu Ser Val Asn Tyr
   385 390 395 400
   Lys Gly Gln Val Ile Gly Ser Asp Ile Asp Ser Ile Lys Lys Leu Glu
                   405                 410                 415
   Gly Gln Gly Val Val Ser Arg Val Val Arg Leu Cys Leu Asn Ser Ser
               420                 425                 430
   Phe Lys Lys Asn Thr Lys Lys Pro Leu Cys Ile Thr Val Asn Asn Gly
           435                 440                 445
   Asp Leu Phe Phe Ile Ala Ser Glu Asp Ser Tyr Gly Glu Asp Thr Ile
       450                 455                 460
   Asn Thr Pro Lys Glu Ile Asp Asp Thr Thr Thr Leu Val Pro Ser Phe
   465                 470                 475                 480
   Lys Asn Ile Leu Asp Lys Val Ile Leu Asp Phe Asn Lys Gln Val Thr
                   485                 490                 495
   Pro Gln Ile Pro Asn Arg Arg Ile Arg Thr Asp Ile Gln Glu Asp Asn
               500                 505                 510
   Tyr Ile Pro Glu Tyr Asp Ser Asn Gly Thr Ser Glu Ile Glu Glu Tyr
           515                 520                 525
   Asn Val Val Asp Leu Asn Ala Phe Phe Tyr Leu His Ala Gln Lys Val
       530                 535                 540
   Pro Glu Gly Glu Thr Asn Ile Ser Leu Thr Ser Ser Ile Asp Thr Ala
   545                 550                 555                 560
   Leu Ser Glu Glu Ser Lys Val Tyr Thr Phe Phe Ser Ser Glu Phe Ile
                   565                 570                 575
   Asp Thr Ile Asn Glu Pro Val Asn Ala Ala Leu Phe Ile Asp Trp Ile
               580                 585                 590
   Ser Lys Val Ile Arg Asp Phe Thr Thr Glu Ala Thr Gln Lys Ser Thr
           595                 600                 605
   Val Asp Lys Ile Ala Asp Ile Ser Leu Ile Val Pro Tyr Val Gly Leu
       610                 615                 620
   Ala Leu Asn Ile Val Asn Glu Thr Glu Lys Gly Asn Phe Lys Glu Ala
   625                 630                 635                 640
   Phe Glu Leu Leu Gly Ala Gly Ile Leu Leu Glu Phe Val Pro Glu Leu
                   645                 650                 655
   Ala Ile Pro Val Ile Leu Val Phe Thr Ile Lys Ser Tyr Ile Asp Ser
   660 665 670
   Tyr Glu Asn Lys Asn Lys Ile Ile Lys Ala Ile Asn Asn Ser Leu Ile
   675 680 685
   Glu Arg Glu Ala Lys Trp Lys Glu Ile Tyr Ser Trp Ile Val Ser Asn
   690 695 700
   Trp Leu Thr Arg Ile Asn Thr Gln Phe Asn Lys Arg Lys Glu Gln Met
   705 710 715 720
   Tyr Gln Ala Leu Gln Asn Gln Val Asp Ala Ile Lys Thr Ala Ile Glu
   725 730 735
   Tyr Lys Tyr Asn Asn Tyr Thr Ser Asp Glu Lys Asn Arg Leu Glu Ser
   740 745 750
   Glu Tyr Asn Ile Asn Asn Ile Glu Glu Glu Leu Asn Lys Lys Val Ser
   755 760 765
   Leu Ala Met Lys Asn Ile Glu Arg Phe Ile Thr Glu Ser Ser Ile Ser
   770 775 780
   Tyr Leu Met Lys Leu Ile Asn Glu Ala Glu Val Gly Lys Leu Lys Glu
   785 790 795 800
   Tyr Asp Lys Arg Val Lys Arg His Leu Leu Glu Tyr Ile Phe Asp Tyr
   805 810 815
   Arg Leu Ile Leu Gly Glu Gln Gly Gly Glu Leu Ile Asp Leu Val Thr
   820 825 830
   Ser Thr Leu Asn Thr Ser Ile Pro Phe Glu Leu Ser Ser Tyr Thr Asn
   835 840 845
   Asp Lys Ile Leu Ile Ile Tyr Phe Asn Arg Leu Tyr Lys Lys Ile Lys
   850 855 860
   Asp Ser Ser Ile Leu Asp Met Arg Tyr Glu Asn Asn Lys Phe Ile Asp
   865 870 875 880
   Ile Ser Gly Tyr Gly Ser Asn Ile Ser Ile Asn Gly Asn Val Tyr Ile
   885 890 895
   Tyr Ser Thr Asn Arg Asn Gln Phe Gly Ile Tyr Asp Asp Arg Leu Ser
   900 905 910
   Glu Val Asn Ile Ala Gln Asn Asn Asp Ile Ile Tyr Asn Ser Arg Tyr
   915 920 925
   Gln Asn Phe Ser Ile Ser Phe Trp Val Arg Ile Pro Lys His Tyr Arg
   930 935 940
   Pro Met Asn His Asn Arg Glu Tyr Thr Ile Ile Asn Cys Met Gly Asn
   945 950 955 960
   Asn Asn Ser Gly Trp Lys Ile Ser Leu Arg Thr Thr Gly Asp Cys Glu
   965 970 975
   Ile Ile Trp Thr Leu Gln Asp Thr Ser Gly Asn Lys Lys Lys Leu Ile
   980 985 990
   Phe Arg Tyr Ser Gln Leu Gly Gly Ile Ser Asp Tyr Ile Asn Lys Trp
   995 1000 1005
   Ile Phe Val Thr Ile Thr Asn Asn Arg Leu Gly Asn Ser Arg Ile
   1010 1015 1020
   Tyr Ile Asn Gly Asn Leu Ile Val Glu Lys Ser Ile Ser Asn Leu
   1025 1030 1035
   Gly Asp Ile His Val Ser Asp Asn Ile Leu Phe Lys Ile Val Gly
   1040 1045 1050
   Cys Asp Asp Lys Met Tyr Val Gly Ile Arg Tyr Phe Lys Val Phe
   1055 1060 1065
   Asn Thr Glu Leu Asp Lys Thr Glu Ile Glu Ile Leu Tyr Ser Asn
   1070 1075 1080
   Glu Pro Asp Pro Ser Ile Leu Lys Asp Tyr Trp Gly Asn Tyr Leu
   1085 1090 1095
   Leu Tyr Asn Lys Lys Tyr Tyr Leu Leu Asn Leu Leu Arg Asn Asp
   1100 1105 1110
   Lys Tyr Ile Thr Arg Asn Ser Asp Ile Leu Asn Ile Ser His Gln
   1115 1120 1125
   Arg Gly Val Thr Lys Asp Leu Phe Ile Phe Ser Asn Tyr Lys Leu
   1130 1135 1140
   Tyr Glu Gly Val Glu Val Ile Ile Arg Lys Asn Gly Pro Ile Asp
   1145 1150 1155
   Ile Ser Asn Thr Asp Asn Phe Val Arg Lys Asn Asp Leu Ala Tyr
   1160 1165 1170
   Ile Asn Val Val Asp His Gly Val Glu Tyr Arg Leu Tyr Ala Asp
   1175 1180 1185
   Ile Ser Ile Thr Lys Pro Glu Lys Ile Ile Lys Leu Ile Arg Arg
   1190 1195 1200
   Ser Asn Pro Asp Asp Ser Leu Gly Gln Ile Ile Val Met Asp Ser
   1205 1210 1215
   Ile Gly Asn Asn Cys Thr Met Asn Phe Gln Asn Asn Asn Gly Gly
   1220 1225 1230
   Asn Ile Gly Leu Leu Gly Phe His Ser Asp Asn Leu Val Ala Ser
   1235 1240 1245
   Ser Trp Tyr Tyr Asn Asn Ile Arg Arg Asn Thr Ser Ser Asn Gly
   1250 1255 1260
   Cys Phe Trp Ser Phe Ile Ser Lys Glu His Gly Trp Gln Glu
   1265 1270 1275
<210> 39
   <211> 1297
   <212> PRT
   <213> Clostridium botulinum
<220>
   <221> misc_feature
   <222> (7)..(7)
   <223> Xaa can be any naturally occurring amino acid
<400> 39
   Met Pro Val Asn Ile Lys Xaa Phe Asn Tyr Asn Asp Pro Ile Asn Asn
   1 5 10 15
   Asp Asp Ile Ile Met Met Glu Pro Phe Asn Asp Pro Gly Pro Gly Thr
   20 25 30
   Tyr Tyr Lys Ala Phe Arg Ile Ile Asp Arg Ile Trp Ile Val Pro Glu
   35 40 45
   Arg Phe Thr Tyr Gly Phe Gln Pro Asp Gln Phe Asn Ala Ser Thr Gly
   50 55 60
   Val Phe Ser Lys Asp Val Tyr Glu Tyr Tyr Asp Pro Thr Tyr Leu Lys
   65                  70                  75                  80
   Thr Asp Ala Glu Lys Asp Lys Phe Leu Lys Thr Met Ile Lys Leu Phe
                   85                  90                  95
   Asn Arg Ile Asn Ser Lys Pro Ser Gly Gln Arg Leu Leu Asp Met Ile
               100                 105                 110
   Val Asp Ala Ile Pro Tyr Leu Gly Asn Ala Ser Thr Pro Pro Asp Lys
           115                 120                 125
   Phe Ala Ala Asn Val Ala Asn Val Ser Ile Asn Lys Lys Ile Ile Gln
       130                 135                 140
   Pro Gly Ala Glu Asp Gln Ile Lys Gly Leu Met Thr Asn Leu Ile Ile
   145                 150                 155                 160
   Phe Gly Pro Gly Pro Val Leu Ser Asp Asn Phe Thr Asp Ser Met Ile
                   165                 170                 175
   Met Asn Gly His Ser Pro Ile Ser Glu Gly Phe Gly Ala Arg Met Met
               180                 185                 190
   Ile Arg Phe Cys Pro Ser Cys Leu Asn Val Phe Asn Asn Val Gln Glu
           195                 200                 205
   Asn Lys Asp Thr Ser Ile Phe Ser Arg Arg Ala Tyr Phe Ala Asp Pro
       210                 215                 220
   Ala Leu Thr Leu Met His Glu Leu Ile His Val Leu His Gly Leu Tyr
   225                 230                 235                 240
   Gly Ile Lys Ile Ser Asn Leu Pro Ile Thr Pro Asn Thr Lys Glu Phe
                   245                 250                 255
   Phe Met Gln His Ser Asp Pro Val Gln Ala Glu Glu Leu Tyr Thr Phe
               260                 265                 270
   Gly Gly His Asp Pro Ser Val Ile Ser Pro Ser Thr Asp Met Asn Ile
           275                 280                 285
   Tyr Asn Lys Ala Leu Gln Asn Phe Gln Asp Ile Ala Asn Arg Leu Asn
       290                 295                 300
   Ile Val Ser Ser Ala Gln Gly Ser Gly Ile Asp Ile Ser Leu Tyr Lys
   305                 310                 315                 320
   Gln Ile Tyr Lys Asn Lys Tyr Asp Phe Val Glu Asp Pro Asn Gly Lys
   325 330 335
   Tyr Ser Val Asp Lys Asp Lys Phe Asp Lys Leu Tyr Lys Ala Leu Met
   340 345 350
   Phe Gly Phe Thr Glu Thr Asn Leu Ala Gly Glu Tyr Gly Ile Lys Thr
   355 360 365
   Arg Tyr Ser Tyr Phe Ser Glu Tyr Leu Pro Pro Ile Lys Thr Glu Lys
   370 375 380
   Leu Leu Asp Asn Thr Ile Tyr Thr Gln Asn Glu Gly Phe Asn Ile Ala
   385 390 395 400
   Ser Lys Asn Leu Lys Thr Glu Phe Asn Gly Gln Asn Lys Ala Val Asn
   405 410 415
   Lys Glu Ala Tyr Glu Glu Ile Ser Leu Glu His Leu Val Ile Tyr Arg
   420 425 430
   Ile Ala Met Cys Lys Pro Val Met Tyr Lys Asn Thr Gly Lys Ser Glu
   435 440 445
   Gln Cys Ile Ile Val Asn Asn Glu Asp Leu Phe Phe Ile Ala Asn Lys
   450 455 460
   Asp Ser Phe Ser Lys Asp Leu Ala Lys Ala Glu Thr Ile Ala Tyr Asn
   465 470 475 480
   Thr Gln Asn Asn Thr Ile Glu Asn Asn Phe Ser Ile Asp Gln Leu Ile
   485 490 495
   Leu Asp Asn Asp Leu Ser Ser Gly Ile Asp Leu Pro Asn Glu Asn Thr
   500 505 510
   Glu Pro Phe Thr Asn Phe Asp Asp Ile Asp Ile Pro Val Tyr Ile Lys
   515 520 525
   Gln Ser Ala Leu Lys Lys Ile Phe Val Asp Gly Asp Ser Leu Phe Glu
   530 535 540
   Tyr Leu His Ala Gln Thr Phe Pro Ser Asn Ile Glu Asn Leu Gln Leu
   545 550 555 560
   Thr Asn Ser Leu Asn Asp Ala Leu Arg Asn Asn Asn Lys Val Tyr Thr
   565 570 575
   Phe Phe Ser Thr Asn Leu Val Glu Lys Ala Asn Thr Val Val Gly Ala
   580 585 590
   Ser Leu Phe Val Asn Trp Val Lys Gly Val Ile Asp Asp Phe Thr Ser
   595 600 605
   Glu Ser Thr Gln Lys Ser Thr Ile Asp Lys Val Ser Asp Val Ser Ile
   610 615 620
   Ile Ile Pro Tyr Ile Gly Pro Ala Leu Asn Val Gly Asn Glu Thr Ala
   625 630 635 640
   Lys Glu Asn Phe Lys Asn Ala Phe Glu Ile Gly Gly Ala Ala Ile Leu
   645 650 655
   Met Glu Phe Ile Pro Glu Leu Ile Val Pro Ile Val Gly Phe Phe Thr
   660 665 670
   Leu Glu Ser Tyr Val Gly Asn Lys Gly His Ile Ile Met Thr Ile Ser
   675 680 685
   Asn Ala Leu Lys Lys Arg Asp Gln Lys Trp Thr Asp Met Tyr Gly Leu
   690 695 700
   Ile Val Ser Gln Trp Leu Ser Thr Val Asn Thr Gln Phe Tyr Thr Ile
   705 710 715 720
   Lys Glu Arg Met Tyr Asn Ala Leu Asn Asn Gln Ser Gln Ala Ile Glu
   725 730 735
   Lys Ile Ile Glu Asp Gln Tyr Asn Arg Tyr Ser Glu Glu Asp Lys Met
   740 745 750
   Asn Ile Asn Ile Asp Phe Asn Asp Ile Asp Phe Lys Leu Asn Gln Ser
   755 760 765
   Ile Asn Leu Ala Ile Asn Asn Ile Asp Asp Phe Ile Asn Gln Cys Ser
   770 775 780
   Ile Ser Tyr Leu Met Asn Arg Met Ile Pro Leu Ala Val Lys Lys Leu
   785 790 795 800
   Lys Asp Phe Asp Asp Asn Leu Lys Arg Asp Leu Leu Glu Tyr Ile Asp
   805 810 815
   Thr Asn Glu Leu Tyr Leu Leu Asp Glu Val Asn Ile Leu Lys Ser Lys
   820 825 830
   Val Asn Arg His Leu Lys Asp Ser Ile Pro Phe Asp Leu Ser Leu Tyr
   835 840 845
   Thr Lys Asp Thr Ile Leu Ile Gln Val Phe Asn Asn Tyr Ile Ser Asn
   850 855 860
   Ile Ser Ser Asn Ala Ile Leu Ser Leu Ser Tyr Arg Gly Gly Arg Leu
   865 870 875 880
   Ile Asp Ser Ser Gly Tyr Gly Ala Thr Met Asn Val Gly Ser Asp Val
   885 890 895
   Ile Phe Asn Asp Ile Gly Asn Gly Gln Phe Lys Leu Asn Asn Ser Glu
   900 905 910
   Asn Ser Asn Ile Thr Ala His Gln Ser Lys Phe Val Val Tyr Asp Ser
   915 920 925
   Met Phe Asp Asn Phe Ser Ile Asn Phe Trp Val Arg Thr Pro Lys Tyr
   930 935 940
   Asn Asn Asn Asp Ile Gln Thr Tyr Leu Gln Asn Glu Tyr Thr Ile Ile
   945 950 955 960
   Ser Cys Ile Lys Asn Asp Ser Gly Trp Lys Val Ser Ile Lys Gly Asn
   965 970 975
   Arg Ile Ile Trp Thr Leu Ile Asp Val Asn Ala Lys Ser Lys Ser Ile
   980 985 990
   Phe Phe Glu Tyr Ser Ile Lys Asp Asn Ile Ser Asp Tyr Ile Asn Lys
   995 1000 1005
   Trp Phe Ser Ile Thr Ile Thr Asn Asp Arg Leu Gly Asn Ala Asn
   1010 1015 1020
   Ile Tyr Ile Asn Gly Ser Leu Lys Lys Ser Glu Lys Ile Leu Asn
   1025 1030 1035
   Leu Asp Arg Ile Asn Ser Ser Asn Asp Ile Asp Phe Lys Leu Ile
   1040 1045 1050
   Asn Cys Thr Asp Thr Thr Lys Phe Val Trp Ile Lys Asp Phe Asn
   1055 1060 1065
   Ile Phe  Gly Arg Glu Leu Asn  Ala Thr Glu Val Ser  Ser Leu Tyr
       1070                 1075                 1080
   Trp Ile  Gln Ser Ser Thr Asn  Thr Leu Lys Asp Phe  Trp Gly Asn
       1085                 1090                 1095
   Pro Leu  Arg Tyr Asp Thr Gln  Tyr Tyr Leu Phe Asn  Gln Gly Met
       1100                 1105                 1110
   Gln Asn  Ile Tyr Ile Lys Tyr  Phe Ser Lys Ala Ser  Met Gly Glu
       1115                 1120                 1125
   Thr Ala  Pro Arg Thr Asn Phe  Asn Asn Ala Ala Ile  Asn Tyr Gln
       1130                 1135                 1140
   Asn Leu  Tyr Leu Gly Leu Arg  Phe Ile Ile Lys Lys  Ala Ser Asn
       1145                 1150                 1155
   Ser Arg  Asn Ile Asn Asn Asp  Asn Ile Val Arg Glu  Gly Asp Tyr
       1160                 1165                 1170
   Ile Tyr  Leu Asn Ile Asp Asn  Ile Ser Asp Glu Ser  Tyr Arg Val
       1175                 1180                 1185
   Tyr Val  Leu Val Asn Ser Lys  Glu Ile Gln Thr Gln  Leu Phe Leu
       1190                 1195                 1200
   Ala Pro  Ile Asn Asp Asp Pro  Thr Phe Tyr Asp Val  Leu Gln Ile
       1205                 1210                 1215
   Lys Lys  Tyr Tyr Glu Lys Thr  Thr Tyr Asn Cys Gln  Ile Leu Cys
       1220                 1225                 1230
   Glu Lys  Asp Thr Lys Thr Phe  Gly Leu Phe Gly Ile  Gly Lys Phe
       1235                 1240                 1245
   Val Lys  Asp Tyr Gly Tyr Val  Trp Asp Thr Tyr Asp  Asn Tyr Phe
       1250                 1255                 1260
   Cys Ile  Ser Gln Trp Tyr Leu  Arg Arg Ile Ser Glu  Asn Ile Asn
       1265                 1270                 1275
   Lys Leu  Arg Leu Gly Cys Asn  Trp Gln Phe Ile Pro  Val Asp Glu
       1280                 1285                 1290
   Gly Trp  Thr Glu
       1295
<210> 40
   <211> 1288
   <212> PRT
   <213> Clostridium botulinum
<400> 40
   Met Pro Val Val Ile Asn Ser Phe Asn Tyr Asp Asp Pro Val Asn Asp
   1               5                   10                  15
   Asn Thr Ile Ile Tyr Ile Arg Pro Pro Tyr Tyr Glu Thr Ser Asn Thr
               20                  25                  30
   Tyr Phe Lys Ala Phe Gln Ile Met Asp Asn Val Trp Ile Ile Pro Glu
           35                  40                  45
   Arg Tyr Arg Leu Gly Ile Asp Pro Ser Leu Phe Asn Pro Pro Val Ser
       50                  55                  60
   Leu Lys Ala Gly Ser Asp Gly Tyr Phe Asp Pro Asn Tyr Leu Ser Thr
   65                  70                  75                  80
   Asn Thr Glu Lys Asn Lys Tyr Leu Gln Ile Met Ile Lys Leu Phe Lys
                   85                  90                  95
   Arg Ile Asn Ser Lys Pro Ala Gly Gln Ile Leu Leu Glu Glu Ile Lys
               100                 105                 110
   Asn Ala Ile Pro Tyr Leu Gly Asn Ser Tyr Thr Gln Glu Glu Gln Phe
           115                 120                 125
   Thr Thr Asn Asn Arg Thr Val Ser Phe Asn Val Lys Leu Ala Asn Gly
       130                 135                 140
   Asn Ile Val Gln Gln Met Ala Asn Leu Ile Ile Trp Gly Pro Gly Pro
   145                 150                 155                 160
   Asp Leu Thr Thr Asn Lys Thr Gly Gly Ile Ile Tyr Ser Pro Tyr Gln
                   165                 170                 175
   Ser Met Glu Ala Thr Pro Tyr Lys Asp Gly Phe Gly Ser Ile Met Thr
               180                 185                 190
   Val Glu Phe Ser Pro Glu Tyr Ala Thr Ala Phe Asn Asp Ile Ser Ile
           195                 200                 205
   Ala Ser His Ser Pro Ser Leu Phe Ile Lys Asp Pro Ala Leu Ile Leu
       210                 215                 220
   Met His Glu Leu Ile His Val Leu His Gly Leu Tyr Gly Thr Tyr Ile
   225                 230                 235                 240
   Thr Glu Tyr Lys Ile Thr Pro Asn Val Val Gln Ser Tyr Met Lys Val
                   245                 250                 255
   Thr Lys Pro Ile Thr Ser Ala Glu Phe Leu Thr Phe Gly Gly Arg Asp
               260                 265                 270
   Arg Asn Ile Val Pro Gln Ser Ile Gln Ser Gln Leu Tyr Asn Lys Val
           275                 280                 285
   Leu Ser Asp Tyr Lys Arg Ile Ala Ser Arg Leu Asn Lys Val Asn Thr
       290                 295                 300
   Ala Thr Ala Leu Ile Asn Ile Asp Glu Phe Lys Asn Leu Tyr Glu Trp
   305                 310                 315                 320
   Lys Tyr Gln Phe Ala Lys Asp Ser Asn Gly Val Tyr Ser Val Asp Leu
                   325                 330                 335
   Asn Lys Phe Glu Gln Leu Tyr Lys Lys Ile Tyr Ser Phe Thr Glu Phe
               340                 345                 350
   Asn Leu Ala Tyr Glu Phe Lys Ile Lys Thr Arg Leu Gly Tyr Leu Ala
           355                 360                 365
   Glu Asn Phe Gly Pro Phe Tyr Leu Pro Asn Leu Leu Asp Asp Ser Ile
       370                 375                 380
   Tyr Thr Glu Val Asp Gly Phe Asn Ile Gly Ala Leu Ser Ile Asn Tyr
   385                 390                 395                 400
   Gln Gly Gln Asn Ile Gly Ser Asp Ile Asn Ser Ile Lys Lys Leu Gln
                   405                 410                 415
   Gly Gln Gly Val Val Ser Arg Val Val Arg Leu Cys Ser Asn Ser Asn
               420                 425                 430
   Thr Lys Asn Ser Leu Cys Ile Thr Val Asn Asn Arg Asp Leu Phe Phe
           435                 440                 445
   Ile Ala Ser Gln Glu Ser Tyr Gly Glu Asn Thr Ile Asn Thr Tyr Lys
       450                 455                 460
   Glu Ile Asp Asp Thr Thr Thr Leu Asp Pro Ser Phe Glu Asp Ile Leu
   465 470 475 480
   Asp Lys Val Ile Leu Asn Phe Asn Glu Gln Val Ile Pro Gln Met Pro
   485 490 495
   Asn Arg Asn Val Ser Thr Asp Ile Gln Lys Asp Asn Tyr Ile Pro Lys
   500 505 510
   Tyr Asp Tyr Asn Arg Thr Asp Ile Ile Asp Ser Tyr Glu Val Gly Arg
   515 520 525
   Asn Tyr Asn Thr Phe Phe Tyr Leu Asn Ala Gln Lys Phe Ser Pro Asn
   530 535 540
   Glu Ser Asn Ile Thr Leu Thr Ser Ser Phe Asp Thr Gly Leu Leu Glu
   545 550 555 560
   Gly Ser Lys Val Tyr Thr Phe Phe Ser Ser Asp Phe Ile Asn Asn Ile
   565 570 575
   Asn Lys Pro Val Gln Ala Leu Leu Phe Ile Glu Trp Val Lys Gln Val
   580 585 590
   Ile Arg Asp Phe Thr Thr Glu Ala Thr Lys Thr Ser Thr Val Asp Lys
   595 600 605
   Leu Lys Asp Ile Ser Leu Val Val Pro Tyr Ile Gly Leu Ala Leu Asn
   610 615 620
   Ile Gly Asp Glu Ile Tyr Lys Gln His Phe Ala Glu Ala Val Glu Leu
   625 630 635 640
   Val Gly Ala Gly Leu Leu Leu Glu Phe Ser Pro Glu Phe Leu Ile Pro
   645 650 655
   Thr Leu Leu Ile Phe Thr Ile Lys Gly Tyr Leu Thr Gly Ser Ile Arg
   660 665 670
   Asp Lys Asp Lys Ile Ile Lys Thr Leu Asp Asn Ala Leu Asn Val Arg
   675 680 685
   Asp Gln Lys Trp Lys Glu Leu Tyr Arg Trp Val Val Ser Lys Trp Leu
   690 695 700
   Thr Thr Ile Asn Thr Gln Phe Asn Lys Arg Lys Glu Gln Met Tyr Lys
   705 710 715 720
   Ala Leu Lys Asn Gln Ala Thr Ala Ile Lys Lys Ile Ile Glu Asn Lys
                   725                 730                 735
   Tyr Asn Asn Tyr Thr Thr Asp Glu Lys Ser Lys Ile Asp Ser Ser Tyr
               740                 745                 750
   Asn Ile Asn Glu Ile Glu Arg Thr Leu Asn Glu Lys Ile Asn Leu Ala
           755                 760                 765
   Met Lys Asn Ile Glu Gln Phe Ile Thr Glu Ser Ser Ile Ala Tyr Leu
       770                 775                 780
   Ile Asn Ile Ile Asn Asn Glu Thr Ile Gln Lys Leu Lys Ser Tyr Asp
   785                 790                 795                 800
   Asp Leu Val Arg Arg Tyr Leu Leu Gly Tyr Ile Arg Asn His Ser Ser
                   805                 810                 815
   Ile Leu Gly Asn Ser Val Glu Glu Leu Asn Ser Lys Val Asn Asn His
               820                 825                 830
   Leu Asp Asn Gly Ile Pro Phe Glu Leu Ser Ser Tyr Thr Asn Asp Ser
           835                 840                 845
   Leu Leu Ile Arg Tyr Phe Asn Lys Asn Tyr Gly Glu Leu Lys Tyr Asn
       850                 855                 860
   Cys Ile Leu Asn Ile Lys Tyr Glu Met Asp Arg Asp Lys Leu Val Asp
   865                 870                 875                 880
   Ser Ser Gly Tyr Arg Ser Arg Ile Asn Ile Gly Thr Gly Val Lys Phe
                   885                 890                 895
   Ser Glu Ile Asp Lys Asn Gln Val Gln Leu Ser Asn Leu Glu Ser Ser
               900                 905                 910
   Lys Ile Glu Val Ile Leu Asn Asn Gly Val Ile Tyr Asn Ser Met Tyr
           915                 920                 925
   Glu Asn Phe Ser Thr Ser Phe Trp Ile Arg Ile Pro Lys Tyr Phe Arg
       930                 935                 940
   Asn Ile Asn Asn Glu Tyr Lys Ile Ile Ser Cys Met Gln Asn Asn Ser
   945                 950                 955                 960
   Gly Trp Glu Val Ser Leu Asn Phe Ser Asn Met Asn Ser Lys Ile Ile
                   965                 970                 975
   Trp Thr Leu Gln Asp Thr Glu Gly Ile Lys Lys Thr Val Val Phe Gln
               980                 985                 990
   Tyr Thr Gln Asn Ile Asn Ile Ser  Asp Tyr Ile Asn Arg  Trp Ile Phe
           995                 1000                1005
   Val Thr  Ile Thr Asn Asn Arg  Leu Ser Asn Ser Lys  Ile Tyr Ile
       1010                 1015                 1020
   Asn Gly  Arg Leu Ile Asn Glu  Glu Ser Ile Ser Asp  Leu Gly Asn
       1025                 1030                 1035
   Ile His  Ala Ser Asn Asn Ile  Met Phe Lys Leu Asp  Gly Cys Arg
       1040                 1045                 1050
   Asp Pro  His Arg Tyr Ile Trp  Ile Lys Tyr Phe Asn  Leu Phe Asp
       1055                 1060                 1065
   Lys Glu  Leu Asn Lys Lys Glu  Ile Lys Asp Leu Tyr  Asp Asn Gln
       1070                 1075                 1080
   Ser Asn  Ser Gly Ile Leu Lys  Asp Phe Trp Gly Asp  Tyr Leu Gln
       1085                 1090                 1095
   Tyr Asp  Lys Pro Tyr Tyr Met  Leu Asn Leu Tyr Asp  Pro Asn Lys
       1100                 1105                 1110
   Tyr Leu  Asp Val Asn Asn Val  Gly Ile Arg Gly Tyr  Met Tyr Leu
       1115                 1120                 1125
   Lys Gly  Pro Arg Gly Arg Ile  Val Thr Thr Asn Ile  Tyr Leu Asn
       1130                 1135                 1140
   Ser Thr  Leu Tyr Met Gly Thr  Lys Phe Ile Ile Lys  Lys Tyr Ala
       1145                 1150                 1155
   Ser Gly  Asn Lys Asp Asn Ile  Val Arg Asn Asn Asp  Arg Val Tyr
       1160                 1165                 1170
   Ile Asn  Val Val Val Lys Asn  Lys Glu Tyr Arg Leu  Ala Thr Asn
       1175                 1180                 1185
   Ala Ser  Gln Ala Gly Val Glu  Lys Ile Leu Ser Ala  Val Glu Ile
       1190                 1195                 1200
   Pro Asp  Val Gly Asn Leu Ser  Gln Val Val Val Met  Lys Ser Glu
       1205                 1210                 1215
   Asn Asp  Gln Gly Ile Arg Asn  Lys Cys Lys Met Asn  Leu Gln Asp
       1220                 1225                 1230
   Asn Asn  Gly Asn Asp Ile Gly  Phe Ile Gly Phe His  Gln Phe Asn
       1235                 1240                 1245
   Asn Ile  Ala Lys Leu Val Ala  Ser Asn Trp Tyr Asn  Arg Gln Ile
       1250                 1255                 1260
   Gly Lys  Ala Ser Arg Thr Phe  Gly Cys Ser Trp Glu  Phe Ile Pro
       1265                 1270                 1275
   Val Asp  Asp Gly Trp Gly Glu  Ser Ser Leu
       1280                 1285
<210> 41
   <211> 1315
   <212> PRT
   <213> Clostridium botulinum
<400> 41
   Met Pro Ile Thr Ile Asn Asn Phe Arg Tyr Ser Asp Pro Val Asn Asn
   1               5                   10                  15
   Asp Thr Ile Ile Met Met Glu Pro Pro Tyr Cys Lys Gly Leu Asp Ile
               20                  25                  30
   Tyr Tyr Lys Ala Phe Lys Ile Thr Asp Arg Ile Trp Ile Val Pro Glu
           35                  40                  45
   Arg Tyr Glu Phe Gly Thr Lys Pro Glu Asp Phe Asn Pro Pro Ser Ser
       50                  55                  60
   Leu Ile Glu Gly Ala Ser Glu Tyr Tyr Asp Pro Asn Tyr Leu Arg Thr
   65                  70                  75                  80
   Asp Ser Asp Lys Asp Arg Phe Leu Gln Thr Met Val Lys Leu Phe Asn
                   85                  90                  95
   Arg Ile Lys Asn Asn Val Ala Gly Glu Ala Leu Leu Asp Lys Ile Ile
               100                 105                 110
   Asn Ala Ile Pro Tyr Leu Gly Asn Ser Tyr Ser Leu Leu Asp Lys Phe
           115                 120                 125
   Asp Thr Asn Ser Asn Ser Val Ser Phe Asn Leu Leu Glu Gln Asp Pro
       130                 135                 140
   Ser Gly Ala Thr Thr Lys Ser Ala Met Leu Thr Asn Leu Ile Ile Phe
   145                 150                 155                 160
   Gly Pro Gly Pro Val Leu Asn Lys Asn Glu Val Arg Gly Ile Val Leu
                   165                 170                 175
   Arg Val Asp Asn Lys Asn Tyr Phe Pro Cys Arg Asp Gly Phe Gly Ser
               180                 185                 190
   Ile Met Gln Met Ala Phe Cys Pro Glu Tyr Val Pro Thr Phe Asp Asn
           195                 200                 205
   Val Ile Glu Asn Ile Thr Ser Leu Thr Ile Gly Lys Ser Lys Tyr Phe
       210                 215                 220
   Gln Asp Pro Ala Leu Leu Leu Met His Glu Leu Ile His Val Leu His
   225                 230                 235                 240
   Gly Leu Tyr Gly Met Gln Val Ser Ser His Glu Ile Ile Pro Ser Lys
                   245                 250                 255
   Gln Glu Ile Tyr Met Gln His Thr Tyr Pro Ile Ser Ala Glu Glu Leu
               260                 265                 270
   Phe Thr Phe Gly Gly Gln Asp Ala Asn Leu Ile Ser Ile Asp Ile Lys
           275                 280                 285
   Asn Asp Leu Tyr Glu Lys Thr Leu Asn Asp Tyr Lys Ala Ile Ala Asn
       290                 295                 300
   Lys Leu Ser Gln Val Thr Ser Cys Asn Asp Pro Asn Ile Asp Ile Asp
   305                 310                 315                 320
   Ser Tyr Lys Gln Ile Tyr Gln Gln Lys Tyr Gln Phe Asp Lys Asp Ser
                   325                 330                 335
   Asn Gly Gln Tyr Ile Val Asn Glu Asp Lys Phe Gln Ile Leu Tyr Asn
               340                 345                 350
   Ser Ile Met Tyr Gly Phe Thr Glu Ile Glu Leu Gly Lys Lys Phe Asn
           355                 360                 365
   Ile Lys Thr Arg Leu Ser Tyr Phe Ser Met Asn His Asp Pro Val Lys
       370                 375                 380
   Ile Pro Asn Leu Leu Asp Asp Thr Ile Tyr Asn Asp Thr Glu Gly Phe
   385 390 395 400
   Asn Ile Glu Ser Lys Asp Leu Lys Ser Glu Tyr Lys Gly Gln Asn Met
   405 410 415
   Arg Val Asn Thr Asn Ala Phe Arg Asn Val Asp Gly Ser Gly Leu Val
   420 425 430
   Ser Lys Leu Ile Gly Leu Cys Lys Lys Ile Ile Pro Pro Thr Asn Ile
   435 440 445
   Arg Glu Asn Leu Tyr Asn Arg Thr Ala Ser Leu Thr Asp Leu Gly Gly
   450 455 460
   Glu Leu Cys Ile Lys Ile Lys Asn Glu Asp Leu Thr Phe Ile Ala Glu
   465 470 475 480
   Lys Asn Ser Phe Ser Glu Glu Pro Phe Gln Asp Glu Ile Val Ser Tyr
   485 490 495
   Asn Thr Lys Asn Lys Pro Leu Asn Phe Asn Tyr Ser Leu Asp Lys Ile
   500 505 510
   Ile Val Asp Tyr Asn Leu Gln Ser Lys Ile Thr Leu Pro Asn Asp Arg
   515 520 525
   Thr Thr Pro Val Thr Lys Gly Ile Pro Tyr Ala Pro Glu Tyr Lys Ser
   530 535 540
   Asn Ala Ala Ser Thr Ile Glu Ile His Asn Ile Asp Asp Asn Thr Ile
   545 550 555 560
   Tyr Gln Tyr Leu Tyr Ala Gln Lys Ser Pro Thr Thr Leu Gln Arg Ile
   565 570 575
   Thr Met Thr Asn Ser Val Asp Asp Ala Leu Ile Asn Ser Thr Lys Ile
   580 585 590
   Tyr Ser Tyr Phe Pro Ser Val Ile Ser Lys Val Asn Gln Gly Ala Gln
   595 600 605
   Gly Ile Leu Phe Leu Gln Trp Val Arg Asp Ile Ile Asp Asp Phe Thr
   610 615 620
   Asn Glu Ser Ser Gln Lys Thr Thr Ile Asp Lys Ile Ser Asp Val Ser
   625 630 635 640
   Thr Ile Val Pro Tyr Ile Gly Pro Ala Leu Asn Ile Val Lys Gln Gly
   645 650 655
   Tyr Glu Gly Asn Phe Ile Gly Ala Leu Glu Thr Thr Gly Val Val Leu
   660 665 670
   Leu Leu Glu Tyr Ile Pro Glu Ile Thr Leu Pro Val Ile Ala Ala Leu
   675 680 685
   Ser Ile Ala Glu Ser Ser Thr Gln Lys Glu Lys Ile Ile Lys Thr Ile
   690 695 700
   Asp Asn Phe Leu Glu Lys Arg Tyr Glu Lys Trp Ile Glu Val Tyr Lys
   705 710 715 720
   Leu Val Lys Ala Lys Trp Leu Gly Thr Val Asn Thr Gln Phe Gln Lys
   725 730 735
   Arg Ser Tyr Gln Met Tyr Arg Ser Leu Glu Tyr Gln Val Asp Ala Ile
   740 745 750
   Lys Lys Ile Ile Asp Tyr Glu Tyr Lys Ile Tyr Ser Gly Pro Asp Lys
   755 760 765
   Glu Gln Ile Ala Asp Glu Ile Asn Asn Leu Lys Asn Lys Leu Glu Glu
   770 775 780
   Lys Ala Asn Lys Ala Met Ile Asn Ile Asn Ile Phe Met Arg Glu Ser
   785 790 795 800
   Ser Arg Ser Phe Leu Val Asn Gln Met Ile Asn Glu Ala Lys Lys Gln
   805 810 815
   Leu Leu Glu Phe Asp Thr Gln Ser Lys Asn Ile Leu Met Gln Tyr Ile
   820 825 830
   Lys Ala Asn Ser Lys Phe Ile Gly Ile Thr Glu Leu Lys Lys Leu Glu
   835 840 845
   Ser Lys Ile Asn Lys Val Phe Ser Thr Pro Ile Pro Phe Ser Tyr Ser
   850 855 860
   Lys Asn Leu Asp Cys Trp Val Asp Asn Glu Glu Asp Ile Asp Val Ile
   865 870 875 880
   Leu Lys Lys Ser Thr Ile Leu Asn Leu Asp Ile Asn Asn Asp Ile Ile
   885 890 895
   Ser Asp Ile Ser Gly Phe Asn Ser Ser Val Ile Thr Tyr Pro Asp Ala
   900 905 910
   Gln Leu Val Pro Gly Ile Asn Gly Lys Ala Ile His Leu Val Asn Asn
   915 920 925
   Glu Ser Ser Glu Val Ile Val His Lys Ala Met Asp Ile Glu Tyr Asn
   930 935 940
   Asp Met Phe Asn Asn Phe Thr Val Ser Phe Trp Leu Arg Val Pro Lys
   945 950 955 960
   Val Ser Ala Ser His Leu Glu Gln Tyr Gly Thr Asn Glu Tyr Ser Ile
   965 970 975
   Ile Ser Ser Met Lys Lys His Ser Leu Ser Ile Gly Ser Gly Trp Ser
   980 985 990
   Val Ser Leu Lys Gly Asn Asn Leu Ile Trp Thr Leu Lys Asp Ser Ala
   995 1000 1005
   Gly Glu Val Arg Gln Ile Thr Phe Arg Asp Leu Pro Asp Lys Phe
   1010 1015 1020
   Asn Ala Tyr Leu Ala Asn Lys Trp Val Phe Ile Thr Ile Thr Asn
   1025 1030 1035
   Asp Arg Leu Ser Ser Ala Asn Leu Tyr Ile Asn Gly Val Leu Met
   1040 1045 1050
   Gly Ser Ala Glu Ile Thr Gly Leu Gly Ala Ile Arg Glu Asp Asn
   1055 1060 1065
   Asn Ile Thr Leu Lys Leu Asp Arg Cys Asn Asn Asn Asn Gln Tyr
   1070 1075 1080
   Val Ser Ile Asp Lys Phe Arg Ile Phe Cys Lys Ala Leu Asn Pro
   1085 1090 1095
   Lys Glu Ile Glu Lys Leu Tyr Thr Ser Tyr Leu Ser Ile Thr Phe
   1100 1105 1110
   Leu Arg Asp Phe Trp Gly Asn Pro Leu Arg Tyr Asp Thr Glu Tyr
   1115 1120 1125
   Tyr Leu Ile Pro Val Ala Ser Ser Ser Lys Asp Val Gln Leu Lys
   1130 1135 1140
   Asn Ile Thr Asp Tyr Met Tyr Leu Thr Asn Ala Pro Ser Tyr Thr
   1145 1150 1155
   Asn Gly Lys Leu Asn Ile Tyr Tyr Arg Arg Leu Tyr Asn Gly Leu
   1160 1165 1170
   Lys Phe Ile Ile Lys Arg Tyr Thr Pro Asn Asn Glu Ile Asp Ser
   1175 1180 1185
   Phe Val Lys Ser Gly Asp Phe Ile Lys Leu Tyr Val Ser Tyr Asn
   1190 1195 1200
   Asn Asn Glu His Ile Val Gly Tyr Pro Lys Asp Gly Asn Ala Phe
   1205 1210 1215
   Asn Asn Leu Asp Arg Ile Leu Arg Val Gly Tyr Asn Ala Pro Gly
   1220 1225 1230
   Ile Pro Leu Tyr Lys Lys Met Glu Ala Val Lys Leu Arg Asp Leu
   1235 1240 1245
   Lys Thr Tyr Ser Val Gln Leu Lys Leu Tyr Asp Asp Lys Asn Ala
   1250 1255 1260
   Ser Leu Gly Leu Val Gly Thr His Asn Gly Gln Ile Gly Asn Asp
   1265 1270 1275
   Pro Asn Arg Asp Ile Leu Ile Ala Ser Asn Trp Tyr Phe Asn His
   1280 1285 1290
   Leu Lys Asp Lys Ile Leu Gly Cys Asp Trp Tyr Phe Val Pro Thr
   1295 1300 1305
   Asp Glu Gly Trp Thr Asn Asp
   1310 1315
<210> 42
   <211> 1306
   <212> PRT
   <213> Clostridium botulinum
<400> 42
   Met Lys Leu Glu Ile Asn Lys Phe Asn Tyr Asn Asp Pro Ile Asp Gly
   1 5 10 15
   Ile Asn Val Ile Thr Met Arg Pro Pro Arg His Ser Asp Lys Ile Asn
   20 25 30
   Lys Gly Lys Gly Pro Phe Lys Ala Phe Gln Val Ile Lys Asn Ile Trp
           35                  40                  45
   Ile Val Pro Glu Arg Tyr Asn Phe Thr Asn Asn Thr Asn Asp Leu Asn
       50                  55                  60
   Ile Pro Ser Glu Pro Ile Met Glu Ala Asp Ala Ile Tyr Asn Pro Asn
   65                  70                  75                  80
   Tyr Leu Asn Thr Pro Ser Glu Lys Asp Glu Phe Leu Gln Gly Val Ile
                   85                  90                  95
   Lys Val Leu Glu Arg Ile Lys Ser Lys Pro Glu Gly Glu Lys Leu Leu
               100                 105                 110
   Glu Leu Ile Ser Ser Ser Ile Pro Leu Pro Leu Val Ser Asn Gly Ala
           115                 120                 125
   Leu Thr Leu Ser Asp Asn Glu Thr Ile Ala Tyr Gln Glu Asn Asn Asn
       130                 135                 140
   Ile Val Ser Asn Leu Gln Ala Asn Leu Val Ile Tyr Gly Pro Gly Pro
   145                 150                 155                 160
   Asp Ile Ala Asn Asn Ala Thr Tyr Gly Leu Tyr Ser Thr Pro Ile Ser
                   165                 170                 175
   Asn Gly Glu Gly Thr Leu Ser Glu Val Ser Phe Ser Pro Phe Tyr Leu
               180                 185                 190
   Lys Pro Phe Asp Glu Ser Tyr Gly Asn Tyr Arg Ser Leu Val Asn Ile
           195                 200                 205
   Val Asn Lys Phe Val Lys Arg Glu Phe Ala Pro Asp Pro Ala Ser Thr
       210                 215                 220
   Leu Met His Glu Leu Val His Val Thr His Asn Leu Tyr Gly Ile Ser
   225                 230                 235                 240
   Asn Arg Asn Phe Tyr Tyr Asn Phe Asp Thr Gly Lys Ile Glu Thr Ser
                   245                 250                 255
   Arg Gln Gln Asn Ser Leu Ile Phe Glu Glu Leu Leu Thr Phe Gly Gly
               260                 265                 270
   Ile Asp Ser Lys Ala Ile Ser Ser Leu Ile Ile Lys Lys Ile Ile Glu
   275 280 285
   Thr Ala Lys Asn Asn Tyr Thr Thr Leu Ile Ser Glu Arg Leu Asn Thr
   290 295 300
   Val Thr Val Glu Asn Asp Leu Leu Lys Tyr Ile Lys Asn Lys Ile Pro
   305 310 315 320
   Val Gln Gly Arg Leu Gly Asn Phe Lys Leu Asp Thr Ala Glu Phe Glu
   325 330 335
   Lys Lys Leu Asn Thr Ile Leu Phe Val Leu Asn Glu Ser Asn Leu Ala
   340 345 350
   Gln Arg Phe Ser Ile Leu Val Arg Lys His Tyr Leu Lys Glu Arg Pro
   355 360 365
   Ile Asp Pro Ile Tyr Val Asn Ile Leu Asp Asp Asn Ser Tyr Ser Thr
   370 375 380
   Leu Glu Gly Phe Asn Ile Ser Ser Gln Gly Ser Asn Asp Phe Gln Gly
   385 390 395 400
   Gln Leu Leu Glu Ser Ser Tyr Phe Glu Lys Ile Glu Ser Asn Ala Leu
   405 410 415
   Arg Ala Phe Ile Lys Ile Cys Pro Arg Asn Gly Leu Leu Tyr Asn Ala
   420 425 430
   Ile Tyr Arg Asn Ser Lys Asn Tyr Leu Asn Asn Ile Asp Leu Glu Asp
   435 440 445
   Lys Lys Thr Thr Ser Lys Thr Asn Val Ser Tyr Pro Cys Ser Leu Leu
   450 455 460
   Asn Gly Cys Ile Glu Val Glu Asn Lys Asp Leu Phe Leu Ile Ser Asn
   465 470 475 480
   Lys Asp Ser Leu Asn Asp Ile Asn Leu Ser Glu Glu Lys Ile Lys Pro
   485 490 495
   Glu Thr Thr Val Phe Phe Lys Asp Lys Leu Pro Pro Gln Asp Ile Thr
   500 505 510
   Leu Ser Asn Tyr Asp Phe Thr Glu Ala Asn Ser Ile Pro Ser Ile Ser
   515 520 525
   Gln Gln Asn Ile Leu Glu Arg Asn Glu Glu Leu Tyr Glu Pro Ile Arg
   530 535 540
   Asn Ser Leu Phe Glu Ile Lys Thr Ile Tyr Val Asp Lys Leu Thr Thr
   545 550 555 560
   Phe His Phe Leu Glu Ala Gln Asn Ile Asp Glu Ser Ile Asp Ser Ser
   565 570 575
   Lys Ile Arg Val Glu Leu Thr Asp Ser Val Asp Glu Ala Leu Ser Asn
   580 585 590
   Pro Asn Lys Val Tyr Ser Pro Phe Lys Asn Met Ser Asn Thr Ile Asn
   595 600 605
   Ser Ile Glu Thr Gly Ile Thr Ser Thr Tyr Ile Phe Tyr Gln Trp Leu
   610 615 620
   Arg Ser Ile Val Lys Asp Phe Ser Asp Glu Thr Gly Lys Ile Asp Val
   625 630 635 640
   Ile Asp Lys Ser Ser Asp Thr Leu Ala Ile Val Pro Tyr Ile Gly Pro
   645 650 655
   Leu Leu Asn Ile Gly Asn Asp Ile Arg His Gly Asp Phe Val Gly Ala
   660 665 670
   Ile Glu Leu Ala Gly Ile Thr Ala Leu Leu Glu Tyr Val Pro Glu Phe
   675 680 685
   Thr Ile Pro Ile Leu Val Gly Leu Glu Val Ile Gly Gly Glu Leu Ala
   690 695 700
   Arg Glu Gln Val Glu Ala Ile Val Asn Asn Ala Leu Asp Lys Arg Asp
   705 710 715 720
   Gln Lys Trp Ala Glu Val Tyr Asn Ile Thr Lys Ala Gln Trp Trp Gly
   725 730 735
   Thr Ile His Leu Gln Ile Asn Thr Arg Leu Ala His Thr Tyr Lys Ala
   740 745 750
   Leu Ser Arg Gln Ala Asn Ala Ile Lys Met Asn Met Glu Phe Gln Leu
   755 760 765
   Ala Asn Tyr Lys Gly Asn Ile Asp Asp Lys Ala Lys Ile Lys Asn Ala
   770 775 780
   Ile Ser Glu Thr Glu Ile Leu Leu Asn Lys Ser Val Glu Gln Ala Met
   785                 790                 795                 800
   Lys Asn Thr Glu Lys Phe Met Ile Lys Leu Ser Asn Ser Tyr Leu Thr
                   805                 810                 815
   Lys Glu Met Ile Pro Lys Val Gln Asp Asn Leu Lys Asn Phe Asp Leu
               820                 825                 830
   Glu Thr Lys Lys Thr Leu Asp Lys Phe Ile Lys Glu Lys Glu Asp Ile
           835                 840                 845
   Leu Gly Thr Asn Leu Ser Ser Ser Leu Arg Arg Lys Val Ser Ile Arg
       850                 855                 860
   Leu Asn Lys Asn Ile Ala Phe Asp Ile Asn Asp Ile Pro Phe Ser Glu
   865                 870                 875                 880
   Phe Asp Asp Leu Ile Asn Gln Tyr Lys Asn Glu Ile Glu Asp Tyr Glu
                   885                 890                 895
   Val Leu Asn Leu Gly Ala Glu Asp Gly Lys Ile Lys Asp Leu Ser Gly
               900                 905                 910
   Thr Thr Ser Asp Ile Asn Ile Gly Ser Asp Ile Glu Leu Ala Asp Gly
           915                 920                 925
   Arg Glu Asn Lys Ala Ile Lys Ile Lys Gly Ser Glu Asn Ser Thr Ile
       930                 935                 940
   Lys Ile Ala Met Asn Lys Tyr Leu Arg Phe Ser Ala Thr Asp Asn Phe
   945                 950                 955                 960
   Ser Ile Ser Phe Trp Ile Lys His Pro Lys Pro Thr Asn Leu Leu Asn
                   965                 970                 975
   Asn Gly Ile Glu Tyr Thr Leu Val Glu Asn Phe Asn Gln Arg Gly Trp
               980                 985                 990
   Lys Ile Ser Ile Gln Asp Ser Lys  Leu Ile Trp Tyr Leu  Arg Asp His
           995                 1000                1005
   Asn Asn  Ser Ile Lys Ile Val  Thr Pro Asp Tyr Ile  Ala Phe Asn
       1010                1015                1020
   Gly Trp  Asn Leu Ile Thr Ile  Thr Asn Asn Arg Ser  Lys Gly Ser
       1025                1030                1035
   Ile Val  Tyr Val Asn Gly Ser  Lys Ile Glu Glu Lys  Asp Ile Ser
       1040                 1045                 1050
   Ser Ile  Trp Asn Thr Glu Val  Asp Asp Pro Ile Ile  Phe Arg Leu
       1055                 1060                 1065
   Lys Asn  Asn Arg Asp Thr Gln  Ala Phe Thr Leu Leu  Asp Gln Phe
       1070                 1075                 1080
   Ser Ile  Tyr Arg Lys Glu Leu  Asn Gln Asn Glu Val  Val Lys Leu
       1085                 1090                 1095
   Tyr Asn  Tyr Tyr Phe Asn Ser  Asn Tyr Ile Arg Asp  Ile Trp Gly
       1100                 1105                 1110
   Asn Pro  Leu Gln Tyr Asn Lys  Lys Tyr Tyr Leu Gln  Thr Gln Asp
       1115                 1120                 1125
   Lys Pro  Gly Lys Gly Leu Ile  Arg Glu Tyr Trp Ser  Ser Phe Gly
       1130                 1135                 1140
   Tyr Asp  Tyr Val Ile Leu Ser  Asp Ser Lys Thr Ile  Thr Phe Pro
       1145                 1150                 1155
   Asn Asn  Ile Arg Tyr Gly Ala  Leu Tyr Asn Gly Ser  Lys Val Leu
       1160                 1165                 1170
   Ile Lys  Asn Ser Lys Lys Leu  Asp Gly Leu Val Arg  Asn Lys Asp
       1175                 1180                 1185
   Phe Ile  Gln Leu Glu Ile Asp  Gly Tyr Asn Met Gly  Ile Ser Ala
       1190                 1195                 1200
   Asp Arg  Phe Asn Glu Asp Thr  Asn Tyr Ile Gly Thr  Thr Tyr Gly
       1205                 1210                 1215
   Thr Thr  His Asp Leu Thr Thr  Asp Phe Glu Ile Ile  Gln Arg Gln
       1220                 1225                 1230
   Glu Lys  Tyr Arg Asn Tyr Cys  Gln Leu Lys Thr Pro  Tyr Asn Ile
       1235                 1240                 1245
   Phe His  Lys Ser Gly Leu Met  Ser Thr Glu Thr Ser  Lys Pro Thr
       1250                 1255                 1260
   Phe His  Asp Tyr Arg Asp Trp  Val Tyr Ser Ser Ala  Trp Tyr Phe
   1265 1270 1275
   Gln Asn Tyr Glu Asn Leu Asn Leu Arg Lys His Thr Lys Thr Asn
   1280 1285 1290
   Trp Tyr Phe Ile Pro Lys Asp Glu Gly Trp Asp Glu Asp
   1295 1300 1305
<210> 43
   <211> 35
   <212> PRT
   <213> Artificial Sequence
<220>
   <223> H6tHAtev 1V(60-94)L A67R
<400> 43
   Leu Ser Glu Leu Asp Asp Arg Arg Asp Ala Leu Gln Ala Gly Ala Ser
   1 5 10 15
   Gln Phe Glu Thr Ser Ala Ala Lys Leu Lys Arg Lys Tyr Trp Trp Lys
   20 25 30
   Asn Leu Lys
   35
<210> 44
   <211> 35
   <212> PRT
   <213> Artificial Sequence
<220>
   <223> H6tHAtev 1V(60-94)L A67RT79S
<400> 44
   Leu Ser Glu Leu Asp Asp Arg Arg Asp Ala Leu Gln Ala Gly Ala Ser
   1 5 10 15
   Gln Phe Glu Ser Ser Ala Ala Lys Leu Lys Arg Lys Tyr Trp Trp Lys
   20 25 30
   Asn Leu Lys
   35
<210> 45
   <211> 75
   <212> PRT
   <213> Artificial Sequence
<220>
   <223> H6tHAtev1V(2-76)LS E55QK59RA67R
<210> 46
   <211> 75
   <212> PRT
   <213> Artificial Sequence
<220>
   <223> H6tHAtev1V(2-76)LS L54AE55QK59RA67R
<400> 46
   Ser Ala Thr Ala Ala Thr Ala Pro Pro Ala Ala Pro Ala Gly Glu Gly
   1 5 10 15
   Gly Pro Pro Ala Pro Pro Pro Asn Leu Thr Ser Asn Arg Arg Leu Gln
   20 25 30
   Gln Thr Gln Ala Gln Val Asp Glu Val Val Asp Ile Met Arg Val Asn
   35 40 45
   Val Asp Lys Val Ala Gln Arg Asp Gln Arg Leu Ser Glu Leu Asp Asp
   50 55 60
   Arg Arg Asp Ala Leu Gln Ala Gly Ala Ser Gln
   65 70 75
<210> 47
   <211> 75
   <212> PRT
   <213> Artificial Sequence
<220>
   <223> H6tHAtev1V(2-76)LS L54EE55LK59RA67R
<400> 47
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Val Asp Ile Met Arg Val Asn
           35                  40                  45
   Val Asp Lys Val Glu Leu Arg Asp Gln Arg Leu Ser Glu Leu Asp Asp
       50                  55                  60
   Arg Arg Asp Ala Leu Gln Ala Gly Ala Ser Gln
   65                  70                  75
<210> 48
   <211> 74
   <212> PRT
   <213> Artificial Sequence
<220>
   <223> H6tHAtev1V(2-76)LS Q34AK59RA67R
<400> 48
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Ala Thr Gln Ala Gln Val Asp Glu Val Val Asp Ile Met Arg Val Asn
           35                  40                  45
   Val Asp Lys Val Leu Glu Arg Asp Gln Arg Leu Ser Glu Leu Asp Asp
       50                  55                  60
   Arg Arg Asp Ala Leu Gln Ala Gly Ala Ser
   65                  70
<210> 49
   <211> 75
   <212> PRT
   <213> Artificial Sequence
<220>
   <223> H6tHAtev1V(2-76)LS V43AK59RA67R
<400> 49
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Ala Asp Ile Met Arg Val Asn
           35                  40                  45
   Val Asp Lys Val Leu Glu Arg Asp Gln Arg Leu Ser Glu Leu Asp Asp
       50                  55                  60
   Arg Arg Asp Ala Leu Gln Ala Gly Ala Ser Gln
   65                  70                  75
<210> 50
   <211> 75
   <212> PRT
   <213> Artificial Sequence
<220>
   <223> H6tHAtev1V(2-76)LS M46IE55QA67R
<400> 50
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Val Asp Ile Ile Arg Val Asn
           35                  40                  45
   Val Asp Lys Val Leu Gln Arg Asp Gln Lys Leu Ser Glu Leu Asp Asp
       50                  55                  60
   Arg Arg Asp Ala Leu Gln Ala Gly Ala Ser Gln
   65                  70                  75
<210> 51
   <211> 75
   <212> PRT
   <213> Artificial Sequence
<220>
   <223> H6tHAtev1V(2-76)LS M46IL54EE55LA67R
<400> 51
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Val Asp Ile Ile Arg Val Asn
           35                  40                  45
   Val Asp Lys Val Glu Leu Arg Asp Gln Lys Leu Ser Glu Leu Asp Asp
       50                  55                  60
   Arg Arg Asp Ala Leu Gln Ala Gly Ala Ser Gln
   65                  70                  75
<210> 52
   <211> 75
   <212> PRT
   <213> Artificial Sequence
<220>
   <223> H6tHAtev1V(2-76)LS Q34AM46IA67R
<400> 52
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Ala Thr Gln Ala Gln Val Asp Glu Val Val Asp Ile Ile Arg Val Asn
           35                  40                  45
   Val Asp Lys Val Leu Glu Arg Asp Gln Lys Leu Ser Glu Leu Asp Asp
       50                  55                  60
   Arg Arg Asp Ala Leu Gln Ala Gly Ala Ser Gln
   65                  70                  75
<210> 53
   <211> 75
   <212> PRT
   <213> Artificial Sequence
<220>
   <223> H6tHAtev1V(2-76)LS V43AM46IA67R
<400> 53
   Ser Ala Thr Ala Ala Thr Ala Pro Pro Ala Ala Pro Ala Gly Glu Gly
   1 5 10 15
   Gly Pro Pro Ala Pro Pro Pro Asn Leu Thr Ser Asn Arg Arg Leu Gln
   20 25 30
   Gln Thr Gln Ala Gln Val Asp Glu Val Ala Asp Ile Ile Arg Val Asn
   35 40 45
   Val Asp Lys Val Leu Glu Arg Asp Gln Lys Leu Ser Glu Leu Asp Asp
   50 55 60
   Arg Arg Asp Ala Leu Gln Ala Gly Ala Ser Gln
   65 70 75
<210> 54
   <211> 75
   <212> PRT
   <213> Artificial Sequence
<220>
   <223> H6tHAtev1V(2-76)LS M46IL54AK59RA67R
<400> 54
   Ser Ala Thr Ala Ala Thr Ala Pro Pro Ala Ala Pro Ala Gly Glu Gly
   1 5 10 15
   Gly Pro Pro Ala Pro Pro Pro Asn Leu Thr Ser Asn Arg Arg Leu Gln
   20 25 30
   Gln Thr Gln Ala Gln Val Asp Glu Val Val Asp Ile Ile Arg Val Asn
   35 40 45
   Val Asp Lys Val Ala Glu Arg Asp Gln Arg Leu Ser Glu Leu Asp Asp
   50 55 60
   Arg Arg Asp Ala Leu Gln Ala Gly Ala Ser Gln
   65 70 75
<210> 55
   <211> 75
   <212> PRT
   <213> Artificial Sequence
<220>
   <223> H6tHAtev1V(2-76)LS M46IK59RA67R
<400> 55
   Ser Ala Thr Ala Ala Thr Ala Pro Pro Ala Ala Pro Ala Gly Glu Gly
   1 5 10 15
   Gly Pro Pro Ala Pro Pro Pro Asn Leu Thr Ser Asn Arg Arg Leu Gln
   20 25 30
   Gln Thr Gln Ala Gln Val Asp Glu Val Val Asp Ile Ile Arg Val Asn
   35 40 45
   Val Asp Lys Val Leu Glu Arg Asp Gln Arg Leu Ser Glu Leu Asp Asp
   50 55 60
   Arg Arg Asp Ala Leu Gln Ala Gly Ala Ser Gln
   65 70 75
<210> 56
   <211> 55
   <212> PRT
   <213> Artificial Sequence
<220>
   <223> H6tHAtev1V(40-94)LS M46IE55QK59RA67RA72D
<400> 56
   Asp Glu Val Val Asp Ile Ile Arg Val Asn Val Asp Lys Val Leu Gln
   1 5 10 15
   Arg Asp Gln Arg Leu Ser Glu Leu Asp Asp Arg Arg Asp Ala Leu Gln
   20 25 30
   Asp Gly Ala Ser Gln Phe Glu Thr Ser Ala Ala Lys Leu Lys Arg Lys
   35 40 45
   Tyr Trp Trp Lys Asn Leu Lys
   50 55
<210> 57
   <211> 55
   <212> PRT
   <213> Artificial Sequence
<220>
   <223> H6tHAtev1V(40-94)LS M46IL54AE55QK59RA67RA72D
<400> 57
   Asp Glu Val Val Asp Ile Ile Arg Val Asn Val Asp Lys Val Ala Gln
   1               5                   10                  15
   Arg Asp Gln Arg Leu Ser Glu Leu Asp Asp Arg Arg Asp Ala Leu Gln
               20                  25                  30
   Asp Gly Ala Ser Gln Phe Glu Thr Ser Ala Ala Lys Leu Lys Arg Lys
           35                  40                  45
   Tyr Trp Trp Lys Asn Leu Lys
       50                  55
<210> 58
   <211> 55
   <212> PRT
   <213> Artificial Sequence
<220>
   <223> H6tHAtev1V(40-94)LS M46IL54AE55QK59RQ76V
<400> 58
   Asp Glu Val Val Asp Ile Ile Arg Val Asn Val Asp Lys Val Ala Gln
   1               5                   10                  15
   Arg Asp Gln Arg Leu Ser Glu Leu Asp Asp Arg Ala Asp Ala Leu Gln
               20                  25                  30
   Ala Gly Ala Ser Val Phe Glu Thr Ser Ala Ala Lys Leu Lys Arg Lys
           35                  40                  45
   Tyr Trp Trp Lys Asn Leu Lys
       50                  55
<210> 59
   <211> 75
   <212> PRT
   <213> Artificial Sequence
<220>
   <223> H6tHAtev1V(2-76)LS L54EE55LK59R
<400> 59
   Ser Ala Thr Ala Ala Thr Ala Pro Pro Ala Ala Pro Ala Gly Glu Gly
   1 5 10 15
   Gly Pro Pro Ala Pro Pro Pro Asn Leu Thr Ser Asn Arg Arg Leu Gln
   20 25 30
   Gln Thr Gln Ala Gln Val Asp Glu Val Val Asp Ile Met Arg Val Asn
   35 40 45
   Val Asp Lys Val Glu Leu Arg Asp Gln Arg Leu Ser Glu Leu Asp Asp
   50 55 60
   Arg Ala Asp Ala Leu Gln Ala Gly Ala Ser Gln
   65 70 75
<210> 60
   <211> 75
   <212> PRT
   <213> Artificial Sequence
<220>
   <223> H6tHAtev1V(2-76)LS Q34AK59R
<400> 60
   Ser Ala Thr Ala Ala Thr Ala Pro Pro Ala Ala Pro Ala Gly Glu Gly
   1 5 10 15
   Gly Pro Pro Ala Pro Pro Pro Asn Leu Thr Ser Asn Arg Arg Leu Gln
   20 25 30
   Ala Thr Gln Ala Gln Val Asp Glu Val Val Asp Ile Met Arg Val Asn
   35 40 45
   Val Asp Lys Val Leu Glu Arg Asp Gln Arg Leu Ser Glu Leu Asp Asp
   50 55 60
   Arg Ala Asp Ala Leu Gln Ala Gly Ala Ser Gln
   65 70 75
<210> 61
   <211> 75
   <212> PRT
   <213> Artificial Sequence
<220>
   <223> H6tHAtev1V(2-76)LS V43AK59R
<400> 61
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Ala Asp Ile Met Arg Val Asn
           35                  40                  45
   Val Asp Lys Val Leu Glu Arg Asp Gln Arg Leu Ser Glu Leu Asp Asp
       50                  55                  60
   Arg Ala Asp Ala Leu Gln Ala Gly Ala Ser Gln
   65                  70                  75
<210> 62
   <211> 75
   <212> PRT
   <213> Artificial Sequence
<220>
   <223> H6tHAtev1V(2-76)LS M46IL54EE55L
<400> 62
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Val Asp Ile Ile Arg Val Asn
           35                  40                  45
   Val Asp Lys Val Glu Leu Arg Asp Gln Lys Leu Ser Glu Leu Asp Asp
       50                  55                  60
   Arg Ala Asp Ala Leu Gln Ala Gly Ala Ser Gln
   65                  70                  75
<210> 63
   <211> 75
   <212> PRT
   <213> Artificial Sequence
<220>
   <223> H6tHAtev1V(2-76)LS Q34AM46I
<400> 63
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Ala Thr Gln Ala Gln Val Asp Glu Val Val Asp Ile Ile Arg Val Asn
           35                  40                  45
   Val Asp Lys Val Leu Glu Arg Asp Gln Lys Leu Ser Glu Leu Asp Asp
       50                  55                  60
   Arg Ala Asp Ala Leu Gln Ala Gly Ala Ser Gln
   65                  70                  75
<210> 64
   <211> 75
   <212> PRT
   <213> Artificial Sequence
<220>
   <223> H6tHAtev1V(2-76)LS V43AM46I
<400> 64
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Ala Asp Ile Ile Arg Val Asn
           35                  40                  45
   Val Asp Lys Val Leu Glu Arg Asp Gln Lys Leu Ser Glu Leu Asp Asp
       50                  55                  60
   Arg Ala Asp Ala Leu Gln Ala Gly Ala Ser Gln
   65                  70                  75
<210> 65
   <211> 75
   <212> PRT
   <213> Artificial Sequence
<220>
   <223> H6tHAtev1V(2-76)LS K59RA67R
<400> 65
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Val Asp Ile Met Arg Val Asn
           35                  40                  45
   Val Asp Lys Val Leu Glu Arg Asp Gln Arg Leu Ser Glu Leu Asp Asp
       50                  55                  60
   Arg Arg Asp Ala Leu Gln Ala Gly Ala Ser Gln
   65                  70                  75
<210> 66
   <211> 75
   <212> PRT
   <213> Artificial Sequence
<220>
   <223> H6tHAtev1V(2-76)LS L54AK59R
<400> 66
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Val Asp Ile Met Arg Val Asn
           35                  40                  45
   Val Asp Lys Val Ala Glu Arg Asp Gln Arg Leu Ser Glu Leu Asp Asp
       50                  55                  60
   Arg Ala Asp Ala Leu Gln Ala Gly Ala Ser Gln
   65                  70                  75
<210> 67
   <211> 75
   <212> PRT
   <213> Artificial Sequence
<220>
   <223> H6tHAtev1V(2-76)LS E55QA67R
<400> 67
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Val Asp Ile Met Arg Val Asn
           35                  40                  45
   Val Asp Lys Val Leu Gln Arg Asp Gln Lys Leu Ser Glu Leu Asp Asp
       50                  55                  60
   Arg Arg Asp Ala Leu Gln Ala Gly Ala Ser Gln
   65                  70                  75
<210> 68
   <211> 75
   <212> PRT
   <213> Artificial Sequence
<220>
   <223> H6tHAtev1V(2-76)LS L54AE55Q
<400> 68
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Val Asp Ile Met Arg Val Asn
           35                  40                  45
   Val Asp Lys Val Ala Gln Arg Asp Gln Lys Leu Ser Glu Leu Asp Asp
   50                  55                  60
   Arg Ala Asp Ala Leu Gln Ala Gly Ala Ser Gln
   65                  70                  75
<210> 69
   <211> 93
   <212> PRT
   <213> Artificial Sequence
<220>
   <223> H6tHAtev1V(2-94)LS Q76V
<400> 69
   Ser Ala Thr Ala Ala Thr Ala Pro Pro Ala Ala Pro Ala Gly Glu Gly
   1 5 10 15
   Gly Pro Pro Ala Pro Pro Pro Asn Leu Thr Ser Asn Arg Arg Leu Gln
   20 25 30
   Gln Thr Gln Ala Gln Val Asp Glu Val Val Asp Ile Met Arg Val Asn
   35 40 45
   Val Asp Lys Val Leu Glu Arg Asp Gln Lys Leu Ser Glu Leu Asp Asp
   50 55 60
   Arg Ala Asp Asn Leu Gln Ala Gly Ala Ser Val Phe Glu Thr Ser Ala
   65 70 75 80
   Ala Lys Leu Lys Arg Lys Tyr Trp Trp Lys Asn Leu Lys
   85 90
<210> 70
   <211> 93
   <212> PRT
   <213> Artificial Sequence
<220>
   <223> H6tHAtev1V(2-94)LS Q76V
<400> 70
   Ser Ala Thr Ala Ala Thr Ala Pro Pro Ala Ala Pro Ala Gly Glu Gly
   1 5 10 15
   Gly Pro Pro Ala Pro Pro Pro Asn Leu Thr Ser Asn Arg Arg Leu Gln
   20 25 30
   Gln Thr Gln Ala Gln Val Asp Glu Val Val Asp Ile Met Arg Val Asn
   35 40 45
   Val Asp Lys Val Leu Glu Arg Asp Gln Lys Leu Ser Glu Leu Asp Asp
   50 55 60
   Arg Ala Asp Asn Leu Gln Ala Gly Ala Ser Val Phe Glu Thr Ser Ala
   65 70 75 80
   Ala Lys Leu Lys Arg Lys Tyr Trp Trp Lys Asn Leu Lys
   85 90
<210> 71
   <211> 93
   <212> PRT
   <213> Artificial Sequence
<220>
   <223> H6tHAtev1V(2-94)LS M46IK59R
<400> 71
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Val Asp Ile Ile Arg Val Asn
           35                  40                  45
   Val Asp Lys Val Leu Glu Arg Asp Gln Arg Leu Ser Glu Leu Asp Asp
       50                  55                  60
   Arg Ala Asp Ala Leu Gln Ala Gly Ala Ser Gln Phe Glu Thr Ser Ala
   65                  70                  75                  80
   Ala Lys Leu Lys Arg Lys Tyr Trp Trp Lys Asn Leu Lys
                   85                  90
<210> 72
   <211> 93
   <212> PRT
   <213> Artificial Sequence
<220>
   <223> H6tHAtev1V(2-94)LS L54AE55Q
<400> 72
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Val Asp Ile Met Arg Val Asn
           35                  40                  45
   Val Asp Lys Val Ala Gln Arg Asp Gln Lys Leu Ser Glu Leu Asp Asp
       50                  55                  60
   Arg Ala Asp Ala Leu Gln Ala Gly Ala Ser Gln Phe Glu Thr Ser Ala
   65                  70                  75                  80
   Ala Lys Leu Lys Arg Lys Tyr Trp Trp Lys Asn Leu Lys
                   85                  90
<210> 73
   <211> 93
   <212> PRT
   <213> Artificial Sequence
<220>
   <223> H6tHAtev1V(2-94)LS A67R
<400> 73
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Val Asp Ile Met Arg Val Asn
           35                  40                  45
   Val Asp Lys Val Leu Glu Arg Asp Gln Lys Leu Ser Glu Leu Asp Asp
       50                  55                  60
   Arg Arg Asp Ala Leu Gln Ala Gly Ala Ser Gln Phe Glu Thr Ser Ala
   65                  70                  75                  80
   Ala Lys Leu Lys Arg Lys Tyr Trp Trp Lys Asn Leu Lys
                   85                  90
<210> 74
   <211> 93
   <212> PRT
   <213> Artificial Sequence
<220>
   <223> H6tHAtev1V(2-94)LS M46IL54AE55QK59R
<400> 74
   Ser Ala Thr Ala Ala Thr Ala Pro Pro Ala Ala Pro Ala Gly Glu Gly
   1               5                   10                  15
   Gly Pro Pro Ala Pro Pro Pro Asn Leu Thr Ser Asn Arg Arg Leu Gln
               20                  25                  30
   Gln Thr Gln Ala Gln Val Asp Glu Val Val Asp Ile Ile Arg Val Asn
           35                  40                  45
   Val Asp Lys Val Ala Gln Arg Asp Gln Arg Leu Ser Glu Leu Asp Asp
       50                  55                  60
   Arg Ala Asp Ala Leu Gln Ala Gly Ala Ser Gln Phe Glu Thr Ser Ala
   65                  70                  75                  80
   Ala Lys Leu Lys Arg Lys Tyr Trp Trp Lys Asn Leu Lys
                   85                  90
<210> 75
   <211> 206
   <212> PRT
   <213> Homo sapiens
<400> 75
<210> 76
   <211> 116
   <212> PRT
   <213> homo sapiens
<400> 76

## Description

The present teaching relates to novel methods of detecting Tetanus neurotoxin (TeNT) and C. botulinum neurotoxin activity in a test sample and to peptides or polypeptides used in said methods. The methods circumvent a previous requirement for neurotoxin serotyping based on antibodies.

Botulinum neurotoxins (BoNT) produced by the bacteria Clostridium botulinum, Clostridium butyricum and Clostridium baratii and TeNT produced by Clostridium tetani are the most poisonous bacterial protein toxins known [1]. BoNT cause the fatal disease botulism due to blockade of neurotransmitter release upon cleavage of soluble N-ethylmaleimide-sensitive factor attachment protein receptor (SNARE) proteins resulting in paralysis. The family of BoNT consists of the nine serotypes BoNT/A-H, X comprising more than 40 variants called subtypes [2, 110, 111]. Due to their low LD₅₀ (<1 ng/kg bodyweight [3]) BoNT detection requires highly sensitive methods suitable for all known BoNT variants.

The most widely established method for BoNT detection is still the lethal Mouse Bioassay (MBA) with a limit of detection of 10-100 pg/mL depending on the serotype [4-6]. However, the end point of this animal experiment is respiratory failure preceded by heavy suffering. Furthermore, the MBA requires large numbers of mice for serotyping and quantitation (> 100/sample), often displays high variations (20-40 % depending on the method used and the laboratory) and requires long readout times of up to four days. Neutralization of BoNT positive samples by preincubation with monovalent BoNT antisera prior to the MBA allows the determination of a known BoNT serotype.

The ex vivo mice phrenic nerve hemidiaphragm (MPN) assay replaces the MBA by closely reproducing in vivo respiratory failure thereby examining the full physiological pharmacodynamics of BoNT with similar sensitivity in a much shorter time (2-4 h), but clearly less animal consumption and hardly any animal suffering [7]. Analogously, preincubation of BoNT positive samples with monovalent BoNT antisera allows the determination of a known BoNT serotype by the MPN assay.

Neuronal cell-based assays for BoNT detection (NCBA) employ primary neuronal cell cultures, stem cell derived neurons and continuous cell lines like neuroblastoma cells and cover all steps of the cellular intoxication including cell surface binding, endocytosis, translocation of the LC into the cellular cytosol and enzymatic activity of the LC on SNARE substrates [8]. A very specific endpoint for BoNT activity is endogenous SNARE cleavage determined in cell lysates by Western blot [9-12] or by ELISA employing BoNT/A-cleavage sensitive antibodies [13-16], or in fixed cells by quantitative immunofluorescence methods also using BoNT/A-cleavage sensitive antibodies [12,17] or in live cells by Förster resonance energy transfer (FRET) detection of an exogenous SNARE-FRET reporter [18]. While BoNT/E causes a level of synaptic inhibition equivalent to the amount of cleaved SNAP-25, BoNT/A causes 50% inhibition of neurotransmitter release at 10-fold lower concentrations than required for 50% cleavage of SNAP-25 [19]. Therefore, measuring inhibition of neurotransmitter release e.g. by the release of pre-loaded, radioactively labelled neurotransmitter [8,20], pre-loaded fluorescent dyes [21,22] or ectopically expressed bioluminescent reporters [23] is a less specific, but more sensitive endpoint. This also accounts for the measurement of endogenous neurotransmitter release by high performance liquid chromatography (HPLC) or immunoassays, indirect measurement detecting enzymatic breakdown products or the postsynaptic currents by voltage clamping [24] or multi electrode arrays (MEAs) [25]. Prior neutralization of BoNT positive samples by monovalent BoNT antisera also allows the determination of a known BoNT serotype by the NCBA [26]. However, NCBA can only be used with highly purified BoNT samples and do not tolerate complex matrices like low pH orange juice or proteases-rich faeces etc. Their read out time ranges from 1-3 days while cell culturing and differentiation of stem cells requires several weeks, skilled personnel and is very expensive.

Immunoassays like ELISA include high sensitivity and sometimes too narrow specificity, but lack detection of active BoNT (i.e. false positive results) and require a large number of antibodies to reliably detect the more than 40 known subtypes of BoNT (i.e. high risk of false negative results) [27-29]. So far no single immunoassay has been produced to detect all BoNT subtypes in parallel.

A newly identified BoNT serotype or subtype easily causes false-negative results in established in vitro assays due to lack of appropriate reagents. Knowledge of BoNT amino acid sequence, its receptors, protein substrate and its cleavage site constitute essential knowledge for safe detection of a BoNT by in vitro assays.

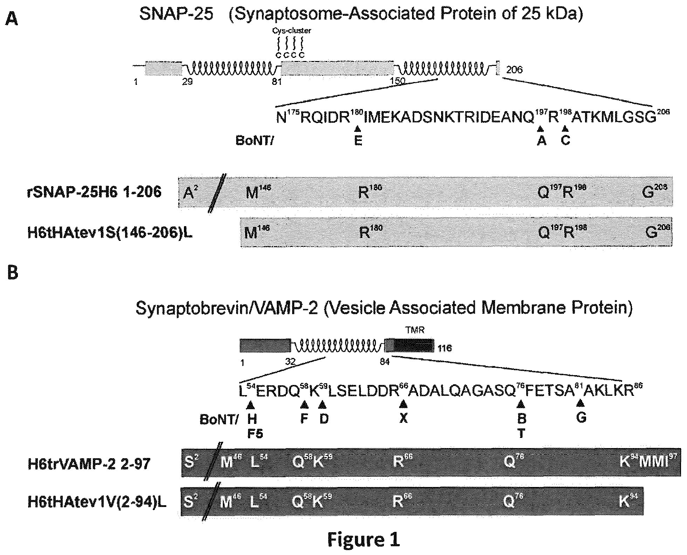

BoNT are 150 kDa AB-protein toxins comprising four domains which play individual roles in the intoxication mechanism [2,30]. Their heavy chain (HC) contains three domains and is responsible for neuronal binding, uptake and translocation of the 50 kDa light chain (LC), a Zn²⁺-dependent endoprotease [31], into the neuronal cytosol [2]. Upon reduction of the disulfide bridge connecting LC and HC the neuronal SNARE proteins are specifically hydrolysed at distinct peptide bonds. VAMP (vesicle associated membrane protein)/synaptobrevin-1/2 represent the substrate for BoNT/B, D, F, G, F5/H, X and TeNT [32-40, 111], whereas BoNT/A, C and E cleave SNAP-25 (synaptosome associated protein of 25 kDa) [41-45]. Except for BoNT/B and TeNT, which share the same cleavage site, hydrolysis occurs in unique positions (Figure 1). BoNT/C is in addition capable of hydrolyzing Syntaxin [42,46]. The LC do not recognize a consensus motif on the SNARE proteins, instead require long stretches of the substrates away from the scissile bond for optimal cleavage [36,43,47-53]. Accordingly, the SNAP-25 peptide was shown to wrap around the LC in a substrate cleft covered by the belt in the holotoxin [54]. Moreover, the mode of substrate recognition of BoNT/B, D, F and TeNT has been investigated [112].

The above described proteolytic specificity of BoNT is utilized by various in vitro endopeptidase assays which detect BoNT due to their catalytic activity by analysing SNARE cleavage products by sodiumdodecyl-polyacrylamide gel electrophoresis (SDS-PAGE), Western blot or HPLC [27]. In addition, SNARE proteins fused to fluorophores would release the fluorescent reporter upon cleavage [55,56] while SNARE peptides equipped with a chromophore and fluorophore exhibit FRET upon BoNT cleavage [18,57-63]. Depending on the SNARE-substrate offered one can distinguish between the group of serotypes cleaving VAMP (B, D, F, G, F5/H, X and TeNT) and that hydrolyzing SNAP-25 (BoNT/A, C and E), but not between serotypes within each group. Advances in mass spectrometry (MS) allowed the development of the Endopep-MS assay which combines the in vitro proteolytic assay employing short, SNARE protein based peptides whose serotype-specific cleavage products are unanimously detected by sophisticated MS equipment [64]. In combination with high-affinity antibody-mediated BoNT enrichment, 0.5 mLD₅₀ of BoNT/A and 0.1 mLD₅₀ of BoNT/B can be detected [65]. The immunological specific recognition of non-cleaved SNARE proteins or the newly generated amino acid (aa) termini, the so-called neoepitopes, provides an alternative to the elaborate and expensive Endopep-MS. Polyclonal antibodies raised against residues 44-94 of human VAMP-2 recognized only non-BoNT/B cleaved VAMP-2 [66] while polyclonal antibodies generated against aa 193-206 and aa 191-206 of SNAP-25 detected only non-BoNT/A cleaved SNAP-25 [13,67]. The first neoepitope antibodies were obtained from rabbits and specifically recognized the newly exposed N-terminus of the C-terminal BoNT/B cleavage product of VAMP-2 [68] and the newly exposed C-terminus of the N-terminal BoNT/A cleavage product of SNAP-25 [67]. Subsequently, polyclonal neoepitope antibodies against TeNT/BoNT/B-cleaved VAMP-2 [69], BoNT/C-cleaved SNAP-25 (aa 191-198) and Syntaxin (254-AVKYQSKA-261) [70] and BoNT/E-cleaved SNAP-25 (aa 173-180) [71] were used in endopeptidase immunoassays to quantify residual TeNT activity in TeNT toxoid vaccine or functionally active BoNT in pharmaceutical preparations, respectively. The first mouse monoclonal antibody (mAb) 4F3-2C1 was raised against the C-terminal BoNT/A neoepitope of rat SNAP-25 (aa 183-197), is still the only commercially available neoepitope Ab (http://www.rdabs.com/files/datasheets/MC-6053.pdf) and was used in a protein chip membrane-capture assay [72] and quantitative immunofluorescence and Western blot methods [12] to detect BoNT/A. Subsequently, further mAb against BoNT/A-cleaved SNAP-25 [14,16,73], against the N-terminal BoNT/B neoepitope of rat VAMP-2 (77-FETSAAK-83) [74], and against the C-terminal BoNT/E neoepitope of rat SNAP-25 (173-TQNRQIDR-180) [75] were generated for use in NCBA or in surface plasmon resonance based assays, respectively. However, endopeptidase immunoassays are hindered by multiple washing and binding steps, non-commercial availability of the required antibodies, possible lot-to-lot variability especially of polyclonal antibodies and unspecific recognition of peptides displaying epitopes similar to neoepitopes like e.g. in human serum albumin [76] or in complex matrices like serum [28,29].

Improved means and methods for testing biological samples for the presence of neurotoxin activity are highly desirable but not yet available. In particular, methods which avoid the current need for immunological reagents and have the potential for practical application are highly desirable. Thus, the technical problem underlying the present teaching may be seen as the provision of means and methods for replacing existing methods by complying with the aforementioned needs. The technical problem is solved by the embodiments characterised in the claims and herein below.

Accordingly, the present teaching relates to a method of detecting clostridial neurotoxin activity in a test sample, comprising (i) contacting a test sample comprising the neurotoxin with a peptide or polypeptide, said peptide or polypeptide comprising an amino acid sequence selected from the group consisting of SEQ ID NOs: 1-31, and 43-74; and (ii) detection of the presence or amount of at least one peptide or polypeptide or of at least one proteolytic cleavage product of said peptide or polypeptide; and (iii) concluding from the presence or amount of the peptide, polypeptide or cleavage product on the presence or amount of a neurotoxin in the sample, wherein the neurotoxin is selected from the group consisting of C. tetani neurotoxin (TeNT) and C. botulinum neurotoxin (BoNT) of serotypes A, B, C, D, E, F, F5, X and H (also called BoNT/H or BoNT/FA, see Peck et al. 2017).

Surprisingly the present inventors found that peptides or polypeptides comprising an amino acid sequence as shown in any one of SEQ ID NO: 1 to 31 and SEQ ID NO: 43 to 74 can be used for identifying a particular neurotoxin responsible for neurotoxin protease activity in a test sample. The aforementioned peptides and polypeptides allow detecting neurotoxin activity and, depending on the need, assigning the activity to either small groups of neurotoxin serotypes or to an individual serotype. US2003/143651 discloses a method of assigning activity to individual neurotoxin serotypes.

### Summary of the invention

The present invention is directed to the subject-matter set forth in the appended claims.

The present invention relates to a method of detecting clostridial neurotoxin activity in a test sample and assigning the activity to an individual neurotoxin serotype,
said method comprising
(i) contacting a test sample suspected of comprising said neurotoxin with a peptide or polypeptide,
(ii) detection of the presence or amount of at least one peptide or polypeptide or of at least one proteolytic cleavage product of said peptide or polypeptide; and
(iii) concluding from the presence or amount of the peptide, polypeptide or cleavage product on the presence or amount of a specific neurotoxin serotype in the sample,
wherein the detection of a cleavage product of the peptide or polypeptide comprising SEQ ID NO: 5 or SEQ ID NO: 6 or SEQ ID NO: 45 or SEQ ID NO: 46 or SEQ ID NO: 47 or SEQ ID NO: 48 or SEQ ID NO: 49 is indicative of catalytic activity of BoNT/D and serotype variants thereof.

The present invention also relates to a peptide or polypeptide comprising an amino acid sequence selected from the group consisting of SEQ ID NOs: 45-49.

The present invention also relates to a kit comprising the peptide or polypeptide of the invention.

### Summary of the teaching

The present invention is defined by the appended claims. The present disclosure provides teachings which in some respects go beyond the disclosure of the invention as such, which is defined exclusively by the appended claims. The teachings are provided to place the actual invention in a broader technical context and to illustrate possible related technical developments. Such additional technical information which does not fall within the scope of the appended claims is not part of the invention. In particular, the terms "embodiment" and "aspect" are not to be construed as necessarily referring to the claimed invention, unless the subject-matter in question falls within the scope of the claims.

The present teaching relates to a method of detecting clostridial neurotoxin activity in a test sample, comprising
(i) contacting a test sample comprising the neurotoxin with a peptide or polypeptide, wherein said peptide or polypeptide comprises an amino acid sequence according to SEQ ID NO: 75 or SEQ ID NO: 76 or a variant thereof, wherein said variant of SEQ ID NO: 75 comprises a fragment of SEQ ID NO: 75 with at least one mutation at position 179, 180, 181, 183, 196, 197, 198, or 199 of SEQ ID NO: 75 and said variant of SEQ ID NO: 76 comprises a fragment of SEQ ID NO: 76 with at least one mutation at position 34, 43, 46, 54, 55, 58, 59, 60, 66, 67, 69, 72, 76, 77, 79, 81 or 82 of SEQ ID NO: 76, wherein said fragment comprises at least 20 consecutive amino acid residues of said amino acid sequence and wherein said mutation renders the peptide or polypeptide insusceptible of proteolytic cleavage by at least one neurotoxin;
(ii) detection of the presence or amount of at least one peptide or polypeptide or of at least one proteolytic cleavage product of said peptide or polypeptide; and
(iii) concluding from the presence or amount of the peptide, polypeptide or cleavage product on the presence or amount of a neurotoxin in the sample,
wherein the neurotoxin is selected from the group consisting of C. tetani neurotoxin (TeNT) and C. botulinum neurotoxin (BoNT) of serotypes A, B, C, D, E, F, F5, H, X and variants thereof.

In one embodiment said peptide or polypeptide comprises a fragment of SEQ ID NO: 75 with at least one mutation selected from the group consisting of D179K, R180W, I181E, E183I, N196Q, Q197N, R198K, and A199R, preferably a mutation or combination of mutations selected from the group consisting of (a) D179K and A199R, (b) D179K and N196Q and Q197N and R198K, (c) R180W and I181E and E183I, (d) D179K, (e) A199R, and (f) N196Q and Q197N and R198K.

In another embodiment said peptide or polypeptide comprises a fragment of SEQ ID NO: 76 with at least one mutation selected from the group consisting of Q34A, V43A, M46I, L54A, L54E, E55Q, E55L, K59R, A67R, A69N, A72D, Q76V, and T79S, preferably a mutation or combination of mutations selected from the group consisting of (a) T79S, (b) E55Q and K59R, (c) L54A and E55Q and K59R, (d) M46I and E55Q, (e) M46I and L54A and K59R, (f) M46I and E55Q and K59R and A72D, (g) M46I and L54A and E55Q and K59R and A72D, (h) M46I and L54A and E55Q and K59R, (i) K59R, (j) M46I, (k) E55Q, (1) A72D, (m) M46I, (n) K59R, (o) L54A and K59R, (p) E55Q, (q) A69N, (r) T79S, (s) A67R, (t) A67R and T79S, (u) E55Q and K59R and A67R, (v) L54A and E55Q and K59R and A67R, (w) L54E and E55L and K59R and A67R, (x) Q34A and K59R and A67R, (y) V43A and K59R and A67R, (z) M46I and E55Q and A67R, (aa) M46I and L54E and E55L and A67R, (bb) Q34A and M46I and A67R, (cc) V43A and M46I and A67R, (dd) M46I and L54A and K59R and A67R, (ee) M46I and K59R and A67R, (ff) M46I and E55Q and K59R and A67R and A72D, (gg) M46I and L54A and E55Q and K59R and A67R and A72D, (hh) M46I and L54A and E55Q and K59R and Q76V, (ii) L54E and E55L and K59R, (jj) Q34A and K59R, (kk) V43A and K59R, (11) M46I and L54E and E55L, (mm) Q34A and M46I, (nn) V43A and M46I, (oo) K59R and A67R, (pp) L54A and K59R, (qq) E55Q and A67R, (rr) L54A and E55Q, (ss) Q76V, (tt) M46I and K59R, (uu) L54A and E55Q, (vv) A67R, and (ww) M46I and L54A and E55Q and K59R.

In another embodiment the peptide or polypeptide is selected from the group consisting of SEQ ID NOs: 1-31, and 43-76.

The present teaching also relates to a method of detecting clostridial neurotoxin activity in a test sample, comprising (i) contacting a test sample comprising the neurotoxin with a peptide or polypeptide, said peptide or polypeptide comprising an amino acid sequence selected from the group consisting of SEQ ID NOs: 1-31, and 43-74; and (ii) detection of the presence or amount of at least one peptide or polypeptide or of at least one proteolytic cleavage product of said peptide or polypeptide; and (iii) concluding from the presence or amount of the peptide, polypeptide or cleavage product on the presence or amount of a neurotoxin in the sample, wherein the neurotoxin is selected from the group consisting of C. tetani neurotoxin (TeNT) and C. botulinum neurotoxin (BoNT) of serotypes A, B, C, D, E, F, F5, X and H.

In one embodiment of the method of the teaching, the detection of (a) a cleavage product of the peptide or polypeptide comprising SEQ ID NO: 1 is indicative of catalytic activity of BoNT/A1 and serotype variants thereof;
(b) a cleavage product of the peptide or polypeptide comprising SEQ ID NO: 2 or SEQ ID NO: 3 or SEQ ID NO: 43 or SEQ ID NO: 44 is indicative of catalytic activity of BoNT/B1 and serotype variants thereof;
(c) a cleavage product of the peptide or polypeptide comprising SEQ ID NO: 4 is indicative of catalytic activity of BoNT/C and serotype variants thereof;
(d) a cleavage product of the peptide or polypeptide comprising SEQ ID NO: 5 or SEQ ID NO: 6 or SEQ ID NO: 45 or SEQ ID NO: 46 or SEQ ID NO: 47 or SEQ ID NO: 48 or SEQ ID NO: 49 is indicative of catalytic activity of BoNT/D and serotype variants thereof;
(e) a cleavage product of the peptide or polypeptide comprising SEQ ID NO: 7 or SEQ ID NO: 8 is indicative of catalytic activity of BoNT/E1 and serotype variants thereof;
(f) a cleavage product of the peptide or polypeptide comprising SEQ ID NO: 9 or SEQ ID NO: 50 or SEQ ID NO: 51 or SEQ ID NO: 52 or SEQ ID NO: 53 is indicative of catalytic activity of BoNT/F1 and serotype variants thereof;
(g) a cleavage product of the peptide or polypeptide comprising SEQ ID NO: 10 or SEQ ID NO: 54 or SEQ ID NO: 55 is indicative of catalytic activity of BoNT/F5, BoNT/H (also called BoNT/HA or BoNT/FA) and serotype variants thereof;
(h) a cleavage product of the peptide or polypeptide comprising SEQ ID NO: 11 or SEQ ID NO: 12 or SEQ ID NO: 56 or SEQ ID NO: 57 is indicative of catalytic activity of TeNT and serotype variants thereof;
(i) a cleavage product of the peptide or polypeptide comprising SEQ ID NO: 58 is indicative of catalytic activity of BoNT/X and serotype variants thereof;
(j) a cleavage product of the peptide or polypeptide comprising SEQ ID NO: 13 or SEQ ID NO: 14 is indicative of catalytic activity of BoNT/A1 and/or BoNT/C and serotype variants thereof;
(k) a cleavage product of the peptide or polypeptide comprising SEQ ID NO: 15 is indicative of catalytic activity of BoNT/A1 and/or BoNT/E1 and serotype variants thereof;
(l) a cleavage product of the peptide or polypeptide comprising SEQ ID NO: 16 is indicative of catalytic activity of BoNT/C and/or BoNT/E1 and serotype variants thereof;
(m) a cleavage product of the peptide or polypeptide comprising SEQ ID NO: 2 or SEQ ID NO: 3 is indicative of catalytic activity of BoNT/B and/or BoNT/X and serotype variants thereof;
(n) a cleavage product of the peptide or polypeptide comprising SEQ ID NO: 5 or SEQ ID NO: 6 or SEQ ID NO: 59 or SEQ ID NO: 60 or SEQ ID NO: 61 is indicative of catalytic activity of BoNT/D and/or BoNT/X and serotype variants thereof;
(o) a cleavage product of the peptide or polypeptide comprising SEQ ID NO: 9 or SEQ ID NO: 62 or SEQ ID NO: 63 or SEQ ID NO: 64 is indicative of catalytic activity of BoNT/F1 and/or BoNT/X and serotype variants thereof;
(p) a cleavage product of the peptide or polypeptide comprising SEQ ID NO: 10 is indicative of catalytic activity of BoNT/F5 and/or BoNT/H and/or BoNT/X and serotype variants thereof;
(q) a cleavage product of the peptide or polypeptide comprising SEQ ID NO: 11 or SEQ ID NO: 12 is indicative of catalytic activity of TeNT and/or BoNT/X and serotype variants thereof;
(r) a cleavage product of the peptide or polypeptide comprising SEQ ID NO: 17 or SEQ ID NO: 74 is indicative of catalytic activity of TeNT and/or BoNT/B1 and/or BoNT/X and serotype variants thereof;
(s) a cleavage product of the peptide or polypeptide comprising SEQ ID NO: 65 is indicative of catalytic activity of BoNT/D and/or BoNT/F5 and/or BoNT/H and serotype variants thereof;
(t) a cleavage product of the peptide or polypeptide comprising SEQ ID NO: 18 or SEQ ID NO: 66 is indicative of catalytic activity of BoNT/D and/or BoNT/F5 and/or BoNT/H and/or BoNT/X and serotype variants thereof;
(u) a cleavage product of the peptide or polypeptide comprising SEQ ID NO: 19 is indicative of catalytic activity of BoNT/F1 and/or BoNT/F5 and/or BoNT/H and/or BoNT/X and serotype variants thereof;
(v) a cleavage product of the peptide or polypeptide comprising SEQ ID NO: 67 is indicative of catalytic activity of BoNT/D and/or BoNT/F1 and serotype variants thereof;
(w) a cleavage product of the peptide or polypeptide comprising SEQ ID NO: 20 or SEQ ID NO: 68 is indicative of catalytic activity of BoNT/D and/or BoNT/F1 and/or BoNT/X and serotype variants thereof;
(x) a cleavage product of the peptide or polypeptide comprising SEQ ID NO: 21 or SEQ ID NO: 22 is indicative of catalytic activity of BoNT/A1 and/or BoNT/C and/or BoNT/E1 and serotype variants thereof;
(y) a cleavage product of the peptide or polypeptide comprising SEQ ID NO: 23 or SEQ ID NO: 69 is indicative of catalytic activity of BoNT/D and/or BoNT/F1 and/or BoNT/F5 and/or BoNT/H and/or BoNT/X and serotype variants thereof;
(z) a cleavage product of the peptide or polypeptide comprising SEQ ID NO: 24 is indicative of catalytic activity of TeNT and/or BoNT/D and/or BoNT/F1 and/or BoNT/F5 and/or BoNT/H and/or BoNT/X and serotype variants thereof;
(aa) a cleavage product of the peptide or polypeptide comprising SEQ ID NO: 25 is indicative of catalytic activity of TeNT and/or BoNT/B1 and/or BoNT/F1 and/or BoNT/F5 and/or BoNT/H and/or BoNT/X and serotype variants thereof;
(bb) a cleavage product of the peptide or polypeptide comprising SEQ ID NO: 26 or SEQ ID NO: 27 is indicative of catalytic activity of TeNT and/or BoNT/B1 and/or BoNT/D and/or BoNT/F5 and/or BoNT/H and/or BoNT/X and serotype variants thereof;
(cc) a cleavage product of the peptide or polypeptide comprising SEQ ID NO: 71 is indicative of catalytic activity of TeNT and/or BoNT/B 1 and/or BoNT/F5 and/or BoNT/H and/or BoNT/X and serotype variants thereof;
(dd) a cleavage product of the peptide or polypeptide comprising SEQ ID NO: 28 or SEQ ID NO: 72 is indicative of catalytic activity of TeNT and/or BoNT/B1 and/or BoNT/D and/or BoNT/F1 and/or BoNT/X and serotype variants thereof;
(ee) a cleavage product of the peptide or polypeptide comprising SEQ ID NO: 29 is indicative of catalytic activity of BoNT/B1 and/or BoNT/D and/or BoNT/F1 and/or BoNT/F5 and/or BoNT/H and/or BoNT/X and serotype variants thereof;
(ff) a cleavage product of the peptide or polypeptide comprising SEQ ID NO: 73 is indicative of catalytic activity of TeNT and/or BoNT/B1 and/or BoNT/D and/or BoNT/F1 and/or BoNT/F5 and/or BoNT/H and serotype variants thereof; and
(gg) a cleavage product of the peptide or polypeptide comprising SEQ ID NO: 30 or SEQ ID NO: 31 is indicative of catalytic activity of TeNT and/or BoNT/B1 and/or BoNT/D and/or BoNT/F1 and/or BoNT/F5 and/or BoNT/H and/or BoNT/X and serotype variants thereof.

In another embodiment,
(a) the catalytic activity of BoNT/A1, and serotype variants thereof, is established by detecting a cleavage product of a peptide or polypeptide comprising the amino acid sequence as shown in SEQ ID NO: 1;
(b) the catalytic activity of BoNT/B, and serotype variants thereof, is established by detecting a cleavage product of a peptide or polypeptide comprising the amino acid sequence as shown in SEQ ID NO: 43 or 44;
(c) the catalytic activity of BoNT/C, and serotype variants thereof, is established by detecting a cleavage product of a peptide or polypeptide comprising the amino acid sequence as shown in SEQ ID NO: 4;
(d) the catalytic activity of BoNT/D, and serotype variants thereof, is established by detecting a cleavage product of a peptide or polypeptide comprising the amino acid sequence as shown in any one of SEQ ID NOs: 45-49;
(e) the catalytic activity of BoNT/E, and serotype variants thereof, is established by detecting a cleavage product of a peptide or polypeptide comprising the amino acid sequence as shown in SEQ ID NO: 7 or 8;
(f) the catalytic activity of BoNT/F, and serotype variants thereof, is established by detecting a cleavage product of a peptide or polypeptide comprising the amino acid sequence as shown in any one of SEQ ID NOs: 50-53;
(g) the catalytic activity of BoNT/F5 and/or BoNT/H, and serotype variants thereof, is established by detecting a cleavage product of a peptide or polypeptide comprising the amino acid sequence as shown in SEQ ID NO: 54 or 55;
(h) the catalytic activity of BoNT/X, and serotype variants thereof, is established by detecting a cleavage product of a peptide or polypeptide comprising the amino acid sequence as shown in SEQ ID NO: 58; and
(i) the catalytic activity of TeNT, and serotype variants thereof, is established by detecting a cleavage product of a peptide or polypeptide comprising the amino acid sequence as shown in SEQ ID NO: 56 or 57.

In another embodiment, any of the methods described herein is performed by making use of a peptide or polypeptide comprising an amino acid sequence selected from the group consisting of SEQ ID NOs: 1-31 and 43-74. In a preferred embodiment said peptide or polypeptide comprises an amino acid sequence selected from the group consisting of SEQ ID NOs: 1, 4, 7, 8, and 43-58.

In another embodiment, the peptide or polypeptide or cleavage product is coupled to a solid support.

In another embodiment, the peptide, polypeptide or cleavage product comprises a detectable label.

In another embodiment, the detectable label is a luciferase, a green, red, cyan, yellow fluorescent protein, a peroxidase, a phosphatase, β-galactosidase, or a radioactive isotope.

In another embodiment, the peptide, polypeptide or cleavage product comprises a tag for immobilization on a solid support.

In another embodiment, the tag is a strep-tag, a halo-tag (HA), a poly-his-tag, a poly-arginine-tag, a poly-HisAsn-tag, a HAT (natural histidine affinity)-tag, a S-tag, a flag-tag, a GST (glutathione-S-transferase)-tag, or a MBP (maltose-binding-protein)-tag.

In another embodiment, said presence or amount of said peptide, polypeptide and/or cleavage product is determined from the detectable amount of cleavage product.

In another embodiment, said method comprises comparing the amount of peptide, polypeptide or cleavage product detectable in step (ii) of the method described herein above with the amount of peptide, polypeptide or cleavage product detectable in a reference sample.

The present teaching also relates to a peptide or polypeptide comprising (a) an amino acid sequence selected from the group consisting of SEQ ID NOs: 1-31 and SEQ ID NOs: 43-74, (b) an amino acid sequence variant of SEQ ID NO: 75, wherein said variant comprises a fragment of at least 20 consecutive amino acids of SEQ ID NO: 75 with at least one mutation selected from the group consisting of D179K, R180W, I181E, E183I, N196Q, Q197N, R198K, and A199R or (c) an amino acid sequence variant of SEQ ID NO: 76 wherein said variant comprises a fragment of at least 20 consecutive amino acids of SEQ ID NO: 76 with at least one mutation selected from the group consisting of Q34A, V43A, M46I, L54A, L54E, E55Q, E55L, K59R, A67R, A69N, A72D, Q76V, and T79S.

In one embodiment of the teaching, the amino acid sequence variant of SEQ ID NO: 75 is selected from the group consisting of (a) D179K and A199R, (b) D179K and N196Q and Q197N and R198K, (c) R180W and I181E and E183I, (d) D179K, (e) A199R, and (f) N196Q and Q197N and R198K.

In one embodiment of the teaching, the amino acid sequence variant of SEQ ID NO: 76 is selected from the group consisting of (a) T79S, (b) E55Q and K59R, (c) L54A and E55Q and K59R, (d) M46I and E55Q, (e) M46I and L54A and K59R, (f) M46I and E55Q and K59R and A72D, (g) M46I and L54A and E55Q and K59R and A72D, (h) M46I and L54A and E55Q and K59R, (i) K59R, (j) M46I, (k) E55Q, (1) A72D, (m) M46I, (n) K59R, (o) L54A and K59R, (p) E55Q, (q) A69N, (r) T79S, (s) A67R, (t) A67R and T79S, (u) E55Q and K59R and A67R, (v) L54A and E55Q and K59R and A67R, (w) L54E and E55L and K59R and A67R, (x) Q34A and K59R and A67R, (y) V43A and K59R and A67R, (z) M46I and E55Q and A67R, (aa) M46I and L54E and E55L and A67R, (bb) Q34A and M46I and A67R, (cc) V43A and M46I and A67R, (dd) M46I and L54A and K59R and A67R, (ee) M46I and K59R and A67R, (ff) M46I and E55Q and K59R and A67R and A72D, (gg) M46I and L54A and E55Q and K59R and A67R and A72D, (hh) M46I and L54A and E55Q and K59R and Q76V, (ii) L54E and E55L and K59R, (jj) Q34A and K59R, (kk) V43A and K59R, (ll) M46I and L54E and E55L, (mm) Q34A and M46I, (nn) V43A and M46I, (oo) K59R and A67R, (pp) L54A and K59R, (qq) E55Q and A67R, (rr) L54A and E55Q, (ss) Q76V, (tt) M46I and K59R, (uu) L54A and E55Q, (vv) A67R, and (ww) M46I and L54A and E55Q and K59R.

The present teaching also relates to a peptide or polypeptide comprising an amino acid sequence selected from the group consisting of SEQ ID NOs: 1-31 and SEQ ID NOs: 43-74, preferably from the group consisting of SEQ ID NOs: 1-12 of the sequence listing or from the group consisting of SEQ ID NOs:1, 4, 7, 8, and 43-58.

In one embodiment, the peptide or polypeptide described according to the present teaching, comprises a tag for immobilization, preferably selected from the group consisting of a halo-tag (HA), strep-tag, a poly-his-tag, a poly-arginine-tag, a poly-HisAsn-tag, a HAT-tag, a S-tag, a flag-tag, a GST-tag, a MBP-tag.

In another embodiment the peptide or polypeptide comprises a detectable label, preferably selected from the group consisting of luciferase, a green fluorescent protein, a red fluorescent protein, a cyan fluorescent protein, a yellow fluorescent protein, preferably GFP, mCherry, CFP, or YFP, or a peroxidase, a phosphatase, β-galactosidase, or a radioactive isotope.

In another embodiment, the peptide or polypeptide comprises at least one additional cleavage site for a protease, said protease being selected from the group consisting of TEV (cysteine protease from Tobacco Etch Virus), HRV (human rhinovirus) 3C protease, Thrombin, Factor Xa, SUMO-protease (ULP1/2, Senp1-7).

The present teaching also relates to the use of the peptide or polypeptide of the present teaching for specific detection of BoNT of serotype A, B, C, D, E, F, F5, H and X and variants thereof and TeNT.

Finally, the present teaching also relates to a kit comprising the peptide or polypeptide of the present teaching. Particularly preferred is a kit comprising a set of peptides or polypeptides consisting of an amino acid sequence selected from the group consisting of SEQ ID NOs: 1, 4, 7, 8, and 43-58 or a set of peptides or polypeptides consisting of an amino acid sequence according to SEQ ID NOs: 1, 4, 7, 8, and 43-58.

### Detailed description

Although the present teaching is described in detail below, it is to be understood that this teaching is not limited to the particular methodologies, protocols and reagents described herein as these may vary. It is also to be understood that the terminology used herein is for the purpose of describing particular embodiments only, and is not intended to limit the scope of the present teaching which will be limited only by the appended claims. Unless defined otherwise, all technical and scientific terms used herein have the same meanings as commonly understood by one of ordinary skill in the art.

In the following, the elements of the present teaching will be described. These elements are listed with specific embodiments, however, it should be understood that they may be combined in any manner and in any number to create additional embodiments. The variously described examples and preferred embodiments should not be construed to limit the present teaching to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

The following abbreviations are used in this specification:
aa: amino acid; BoNT, botulinum neurotoxin; BSA: bovine serum albumin; DTE: dithioerythritol; DTT: Dithiothreitol; EC₅₀, median effective concentration; ELISA: enzyme linked immunosorbent assay; FRET: Förster resonance energy transfer; GBS, ganglioside binding site; GFP, mCherry, CFP, YFP, green fluorescent protein, red fluorescent protein, cyan fluorescent protein, yellow fluorescent protein; GST, glutathion-S-transferase; h, human; HA, halo-tag; HAT: natural histidine affinity tag; HC, heavy chain; H_{C}, carboxyl-terminal half of HC; HcA, HcE etc., H_{C} fragment of BoNT serotype A, E etc, respectively; H_{CN} and Hcc, 25 kDa halves of H_{C}; H_{N}, amino-terminal half of HC; H6, hexahistidine-tag; 6xHN, (HisAsn)6 tag; HA, HaloTag; HPLC, high performance liquid chromatography; HRV, human rhinovirus 3C protease; LC, light chain; LD₅₀: median lethal dose; LD, luminal domain; mAb: monoclonal antibody; MBA/MLB, mouse lethality bioassay; MPN assay, mice phrenic nerve hemidiaphragm assay; MS: Mass spectrometry; MW, molecular weight; NCBA: neuronal cell based assay; NL: NanoLuc^{®} Luciferase; S, C-terminal Streptag; SDS-PAGE, sodiumdodecyl-polyacrylamide gel electrophoresis; SEC, size exclusion chromatography; SNAP-25: synaptosomal associated protein of 25 kDa; SNARE, soluble N-ethylmaleimide-sensitive factor attachment protein receptor; SV, synaptic vesicle; rSV2A-C, rat synaptic vesicle glycoprotein 2 isoform A, B or C; Syt, synaptotagmin; synaptobrevin, Syb; Syntaxin, Stx; TeNT: Tetanus Neurotoxin; TEV, cysteine protease from Tobacco Etch Virus; TMD, transmembrane domain; VAMP, vesicle associated membrane protein.

The practice of the present teaching will employ, unless otherwise indicated, conventional methods of chemistry, biochemistry, cell biology, immunology, and recombinant DNA techniques which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps although in some embodiments such other member, integer or step or group of members, integers or steps may be excluded, i.e. the subject-matter consists in the inclusion of a stated member, integer or step or group of members, integers or steps. The terms "a" and "an" and "the" and similar reference used in the context of describing the teaching (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as"), provided herein is intended merely to better illustrate the teaching and does not pose a limitation on the scope of the teaching. No language in the specification should be construed as indicating any non-claimed element essential to the practice of the teaching.

Several documents are cited throughout the text of this specification. Nothing herein is to be construed as an admission that the teaching is not entitled to antedate such disclosure by virtue of prior teaching.

The present teaching relates to a method of detecting clostridial neurotoxin activity in a test sample, comprising (i) contacting a test sample comprising the neurotoxin with a peptide or polypeptide, said peptide or polypeptide comprising an amino acid sequence selected from the group consisting of SEQ ID NOs: 1-31, and 43-74; and (ii) detection of the presence or amount of at least one peptide or polypeptide or of at least one proteolytic cleavage product of said peptide or polypeptide; and (iii) concluding from the presence or amount of the peptide, polypeptide or cleavage product on the presence or amount of a neurotoxin in the sample, wherein the neurotoxin is selected from the group consisting of C. tetani neurotoxin (TeNT) and C. botulinum neurotoxin (BoNT) of serotypes A, B, C, D, E, F, F5, X and H.

The present teaching also relates to a method of detecting clostridial neurotoxin activity in a test sample, comprising (i) contacting a test sample comprising the neurotoxin with a peptide or polypeptide, wherein said peptide or polypeptide comprises an amino acid sequence according to SEQ ID NO: 75 or SEQ ID NO: 76 or a variant thereof, wherein said variant of SEQ ID NO: 75 comprises a fragment of SEQ ID NO: 75 with at least one mutation at position 179, 180, 181, 183, 196, 197, 198, or 199 of SEQ ID NO: 75 and said variant of SEQ ID NO: 76 comprises a fragment of SEQ ID NO: 76 with at least one mutation at position 34, 43, 46, 54, 55, 58, 59, 60, 66, 67, 69, 72, 76, 77, 79, 81 or 82 of SEQ ID NO: 76, wherein said fragment comprises at least 20 consecutive amino acid residues of said amino acid sequence and wherein said mutation renders the peptide or polypeptide insusceptible of proteolytic cleavage by at least one neurotoxin; (ii) detection of the presence or amount of at least one peptide or polypeptide or of at least one proteolytic cleavage product of said peptide or polypeptide; and (iii) concluding from the presence or amount of the peptide, polypeptide or cleavage product on the presence or amount of a neurotoxin in the sample, wherein the neurotoxin is selected from the group consisting of C. tetani neurotoxin (TeNT) and C. botulinum neurotoxin (BoNT) of serotypes A, B, C, D, E, F, F5, H, X and variants thereof.

The term "variant" refers to peptides and polypeptides which differ in amino acid sequence from the reference amino acid sequence. A variant may differ by being an N-terminal and/or C-terminal deletion mutant of the reference sequence and/or by having at least one mutation in the amino acid sequence when compared to the reference sequence. Preferably the mutation is an amino acid substitution. The amino acid substitution may be for example a conservative amino acid substitution, a non-conservative amino acid substitution or a chemical modification of at least one amino acid residue, preferably a modification which is not observed in a eukaryotic or prokaryotic cell.

As used herein. the term "at least 20 consecutive amino acid residues" includes fragments of for example at least 20 amino acid residues, at least 30 amino acid residues, at least 40 amino acid residues, at least 50 amino acid residues, at least 60 amino acid residues, at least 80 amino acid residues. Such fragments may comprise the full amino acid sequence of the reference sequence or comprise an N-terminal and/or C-terminal deletion of said reference sequence. The amino acid sequence of SEQ ID NOs: 75 and 76 are examples of reference sequences. A deletion may comprise up to 10, 20, 30, 40, or 50 N-terminal and/ or C-terminal amino acid residues,

The term "consecutive amino acid residues" refers to a sequence of amino acids which is identical to the reference sequence. Regarding the reference sequence of SEQ ID NO: 75, the term also includes fragments of said sequence with one or more mutations at positions 179, 180, 181, 183, 196, 197, 198, or 199 of SEQ ID NO: 75. Regarding the reference sequence of SEQ ID NO: 76, the term also includes fragments of said sequence one or more mutations at positions 34, 43, 46, 54, 55, 58, 59, 60, 66, 67, 69, 72, 76, 77, 79, 81 or 82 of SEQ ID NO: 76.

As used herein, the term "detecting clostridial neurotoxin activity in a test sample" means demonstrating that the test sample comprises a protein having proteolytic activity that can be assigned to a neurotoxin of clostridial origin. Examples of neurotoxin of clostridial origin comprise neurotoxins such as BoNT/A1, BoNT/A2, BoNT/A3, BoNT/A3, BoNT/A4, BoNT/A5, BoNT/A6, BoNT/A7, BoNT/A8, BoNT/B1, BoNT/B2, BoNT/B3, BoNT/B4bv, BoNT/B5nP, BoNT/B6, BoNT/B7, BoNT/B8, BoNT/B9, BoNT/C, BoNT/CD, BoNT/D, BoNT/DC, BoNT/E1, BoNT/E2, BoNT/E3, BoNT/E4, BoNT/E5, BoNT/E6, BoNT/E7, BoNT/E8, BoNT/E10, BoNT/E11, BoNT/E12, BoNT/F1, BoNT/F2, BoNT/F3, BoNT/F4, BoNT/F5, BoNT/F6, BoNT/F7, BoNT/F8, BoNT/F9, BoNT/H (also called BoNT/H and BoNT/FA), BoNT/G , BoNT/X and TeNT.

As used herein, the term "contacting a test sample comprising the neurotoxin with a peptide or polypeptide" means establishing conditions allowing a functional interaction between the neurotoxin and the peptide or polypeptide. Functional conditions are conditions allowing for the neurotoxin to be proteolytic ally active. In one embodiment, the peptide or polypeptide may be coupled to a solid support and the test sample is a liquid sample comprising the neurotoxin, wherein the liquid sample is applied to the solid support.

As used herein, the term "peptide" refers to an oligopeptide of up to 30 amino acids, whereas the term "polypeptide" refers to an amino acid chain of more than 31 amino acids. These terms also refer to peptides and polypeptides, respectively, which contain a modification including those required for coupling of the peptide or polypeptide to the solid support. Examples of modified amino acids comprise D-amino acids, selenocysteine, pyrrolysine, hydroxyproline, O-phosphoserine, O-phosphotyrosine, γ-carboxyglutamate, canavanine, azetidin-2-carbonic acid, ornithine, norleucine, norvaline, and homonorleucine. The "peptide or polypeptide" is the substrate of a clostridial neurotoxin, if it can be recognized and proteolytically cleaved by the neurotoxin. A peptide or polypeptide comprising an amino acid sequence selected from the group consisting of a particular amino acid sequence may be a peptide or polypeptide *consisting of* or *comprising* said amino acid sequence. Whereas the term "peptide or polypeptide" refers to the substrate of a neurotoxin, the term "cleavage product" refers to the product arising from the proteolytic activity of the neurotoxin. A cleavage product can be distinguished from the "peptide or polypeptide" by lacking at least one peptide bond.

The term "peptide or polypeptide" also comprises detectably labelled peptides and polypeptides and peptides and polypeptides coupled to a solid support.

In one embodiment, the method described herein comprises the additional step of concluding from the particular neurotoxin identified in step (iii) on the presence of the particular Clostridium strain producing clostridial neurotoxin. Said strain is characterized by producing at least one of the following neurotoxins selected from the group consisting of BoNT/A1, BoNT/A2, BoNT/A3, BoNT/A3, BoNT/A4, BoNT/A5, BoNT/A6, BoNT/A7, BoNT/A8, BoNT/B1, BoNT/B2, BoNT/B3, BoNT/B4bv, BoNT/B5nP, BoNT/B6, BoNT/B7, BoNT/B8, BoNT/B9, BoNT/C, BoNT/CD, BoNT/D, BoNT/DC, BoNT/E1, BoNT/E2, BoNT/E3, BoNT/E4, BoNT/E5, BoNT/E6, BoNT/E7, BoNT/E8, BoNT/E10, BoNT/E11, BoNT/E12, BoNT/F1, BoNT/F2, BoNT/F3, BoNT/F4, BoNT/F5, BoNT/F6, BoNT/F7, BoNT/F8, BoNT/F9, BoNT/H (also called BoNT/H and BoNT/FA), BoNT/X and TeNT.

As used herein, the term "test sample" refers to a sample comprising a biological sample to be tested, whereas the term "biological sample" refers to a specimen obtained from any source, comprising or suspected of comprising C. botulinum, C. butyricum, C. baratii or C. tetani or neurotoxin of C. botulinum, C. butyricum, C. baratii or C. tetani.

In one embodiment, the method described herein comprises prior to step (i) a step of obtaining a biological sample. The biological sample may be obtained from food such as milk, juices, water, meat, sausage, canned food, fish, sea food, honey, environmental samples such as soil, dust, air, and clinical samples such as tissue samples, blood, serum, feces.

In another embodiment, the biological sample or the test sample may be treated for example in order to stabilize the components of the biological sample or in order to establish the reaction conditions of the method described herein.

Such treatment may aim at lysing cells present in the sample. Accordingly, the method described herein may also comprise a step of adding to the biological sample or test sample a detergent. Preferably, the detergent is Polyoxyethylen(20)-sorbitan-monolaurat such as Tween-20^{®}, preferably added to a final concentration in the test sample of 0.5%.

Such treatment may aim at stabilizing residual proteins of the sample or by providing conditions suitable for neurotoxin activity. Accordingly, the method described herein may also comprise a step of adding to the biological sample or test sample a salt such as NaCl, ZnCl₂, KCl, NaSO₄, Na-glutamate, K-glutamate, CaCl₂, a reducing agent such as DTT (dithiothreitol), beta-mercaptoethanol, dithioerythritol (DTE), TCEP (tris(2-carboxyethyl)phosphine) or a buffer such as HEPES (4-(2-hydroxyethyl)-1-piperazineethanesulfonic acid ), MES (2-(N-morpholino)ethanesulfonic acid), glycine/hydrochloric acid, potassium hydrogen phthalate/hydrochloric acid, citric acid/sodium citrate, sodium acetate/acetic acid, potassium hydrogen phthalate/sodium hydroxide, disodium hydrogen phthalate/sodium dihydrogen orthophosphate, dipotassium hydrogen phthalate/potassium dihydrogen orthophospate, potassium dihydrogen orthophosphate/sodium hydroxide, barbitone sodium/hydrochloric acid, tris(hydroxylmethyl) aminomethane/hydrochloric acid, sodium tetraborate/hydrochloric acid, glycine/sodium hydroxide, sodium carbonate/sodium hydrogen carbonate, sodium tetraborate/sodium hydroxide, sodium bicarbonate/sodium hydroxide, sodium hydrogen orthophosphate/sodium hydroxide, or potassium chloride/sodium hydroxide.

ZnCl₂ may preferably be added to a final concentration in the test sample of up to 5 µM, up to 10 µM, up to 50 µM, up to 100 µM, up to 150 µM, up to 200 µM, up to 250 µM or up to 500 µM. Preferably, the test sample comprises at least 250 µM of ZnCl₂.

DTT may preferably be added to a final concentration in the test sample of up to 1 mM, up to 2 mM, up to 5 mM, up to 10 mM, up to 15 mM up to 20mM, up to 25 mM, up to 50 mM or up to 100 mM. Preferably, the test sample comprises at least 20 mM of DTT.

HEPES pH 7.2 may preferably be added to a final concentration in the test sample of up to 1 mM, up to 2 mM, up to 5 mM, up to 10 mM, up to 15 mM, up to 20 mM, up to 50 mM or up to 100 mM. Preferably, the test sample comprises at least 50 mM of HEPES.

The method described herein may also comprise a step of adding a protease inhibitor not chelating Zn²⁺. Preferably, the protease inhibitor is selected from the group consisting of antipain-dihydrochloride, antithrombin III, aprotinin, APMSF (4-Amidino-phenyl) methanesulfonyl fluoride, bestatin, Calpain Inhibitor-I/-II, chymostatin, dabigatran, danaparoid, 3,4-dichloroisocoumarin, E-64, hirudin, leupeptin, a2-macroglobulin, Pefabloc SC, pepstatin, phosphormidon, PMSF (Phenylmethylsulfonyl fluoride), rivaroxaban, TIMP-2 (Tissue Inhibitor of Metalloproteinase 2), TLCK/HCI (L-1-Chloro-3-[4-tosylamido]-7-amino-2-heptanone-HCI), TPCK (L-1-Chloro-3-[4-tosylamido]-4-phenyl-2-butanone), Trypsin Inhibitors from chicken egg white and soybean.

As used herein, the term "detection of the presence or amount of at least one peptide or polypeptide or ... cleavage product" means assessing or monitoring the presence or amount of educt, product or both. It will be apparent to those of skilled in the art that, upon exposure of the peptide or polypeptide to the neurotoxin, i.e. after contacting the test sample with the peptide or polypeptide and depending on the particular neurotoxin activity, the peptide or polypeptide will be cleaved. Neurotoxin activity can be assessed by detecting the amount of educt, the amount of product or both, wherein said amount is preferably detected over time. The term "educt" refers to the peptide or polypeptide prior to any proteolytic cleavage by the neurotoxin, the term "product" refers to the cleaved product or cleavage product arising from the proteolytic cleavage of the peptide or polypeptide. The term "cleaved product" or "cleavage product" comprises the fragment arising from the amino acid sequence which is located in the uncleaved peptide or polypeptide N-terminal to the neurotoxin cleavage site and the fragment arising from the amino acid sequence which is located in the uncleaved peptide or polypeptide C-terminal to the neurotoxin cleavage site or both fragments. In other words, the term "cleaved product" or "cleavage product" covers an N-terminal cleavage product of the peptide or polypeptide, a C-terminal cleavage product of the peptide or polypeptide or both.

The amounts can be detected by any suitable methods, including SDS-PAGE, HPLC. A preferred method comprises measuring fluorescence or bioluminescence. In a preferred embodiment, neurotoxin activity results in the release of a labelled N- or C-terminal cleavage product of the peptide or polypeptide, wherein the released cleavage product is preferably labelled with a fluorophore or luminophore. Another preferred embodiment comprises measuring educts and/or products in a so-called Endopep-MS assay [64, 65].

The method described herein encompasses embodiments comprising a labelled peptide or polypeptide, which is coupled to a solid support via an amino acid residue located N-terminal to the cleavage site of a neurotoxin. In a preferred embodiment, said peptide or polypeptide is labelled at an amino acid residue located C-terminal to the cleavage site. The method described herein also encompasses embodiments comprising a labelled peptide or polypeptide, which is coupled to a solid support via an amino acid residue located C-terminal to the cleavage site of a neurotoxin. In a preferred embodiment, said latter peptide or polypeptide is labelled at an amino acid residue located N-terminal to the cleavage site. Since the neurotoxin activity of the aforementioned embodiments will result in release of a labelled fragment, the presence or amount of cleavage product may be detected by measuring the amount of detectable label in the supernatant of the solid support.

A cleavage product of the present teaching comprises at least one amino acid residues. In other words, it may consist of a single amino acid residue or of up to 2, up to 3, up to 4, up to 5, up to 6, up to 7, up to 8, up to 9 or up to 10, up to 16, up to 17, up to 18, up to 19, up to 22, up to 36, up to 40 or more amino acid residues. Longer cleavage products consist of up to 15, up to 20, up to 30, up to 40, up to 50, up to 51, up to 54, up to 57, up to 58, up to 59, up to 60, up to 76, up to 180, up to 197, up to 198 or up to 200 amino acid residues. Preferably, the cleavage product is detectably labelled.

The method of detecting neurotoxin activity described herein may also comprise a step of calculating a ratio of the amount of product over the amount of educt.

The term "concluding from the presence or amount of the peptide, polypeptide or cleavage product on the presence or amount of a neurotoxin" means drawing conclusions from detected amounts. Preferably, an increase in the amount of product, a decrease in the amount educt, or an increase of the ratio of amount of product over amount of educt are indicative for neurotoxin activity.

As used herein, the term "a neurotoxin of serotype A" comprises BoNT/A1 according to SEQ ID NO:32 and serotype variants thereof, the term "a neurotoxin of serotype B" comprises BoNT/B1 according to SEQ ID NO:33 and serotype variants thereof, the term "a neurotoxin of serotype C" comprises BoNT/C according to SEQ ID NO:34 and serotype variants thereof, the term "a neurotoxin of serotype D" comprises BoNT/D according to SEQ ID NO:35 and serotype variants thereof, the term "a neurotoxin of serotype E" comprises BoNT/E1 according to SEQ ID NO:36 and serotype variants thereof, the term "a neurotoxin of serotype F" comprises BoNT/F1 according to SEQ ID NO:37, the term "a neurotoxin of serotype F5" comprises BoNT/F5 according to SEQ ID NO:38 and serotype variants thereof and the term "a neurotoxin of serotype G" comprises BoNT/G according to SEQ ID NO:39 and serotype variants thereof and the term "a neurotoxin of serotype H" comprises BoNT/H according to SEQ ID NO:40 and serotype variants thereof and the term "a neurotoxin of serotype X" comprises BoNT/X according to SEQ ID NO:42 and serotype variants thereof and the term "Tetanus neurotoxin" comprises TeNT according to SEQ ID NO:41 and serotype variants thereof. The term "serotype variant" is described herein below.

In one embodiment the detection of (a) a cleavage product of the peptide or polypeptide comprising SEQ ID NO: 1 is indicative of catalytic activity of BoNT/A1 and serotype variants thereof;
(b) a cleavage product of the peptide or polypeptide comprising SEQ ID NO: 2 or SEQ ID NO: 3 or SEQ ID NO: 43 or SEQ ID NO: 44 is indicative of catalytic activity of BoNT/B1 and serotype variants thereof;
(c) a cleavage product of the peptide or polypeptide comprising SEQ ID NO: 4 is indicative of catalytic activity of BoNT/C and serotype variants thereof;
(d) a cleavage product of the peptide or polypeptide comprising SEQ ID NO: 5 or SEQ ID NO: 6 or SEQ ID NO: 45 or SEQ ID NO: 46 or SEQ ID NO: 47 or SEQ ID NO: 48 or SEQ ID NO: 49 is indicative of catalytic activity of BoNT/D and serotype variants thereof;
(e) a cleavage product of the peptide or polypeptide comprising SEQ ID NO: 7 or SEQ ID NO: 8 is indicative of catalytic activity of BoNT/E1 and serotype variants thereof;
(f) a cleavage product of the peptide or polypeptide comprising SEQ ID NO: 9 or SEQ ID NO: 50 or SEQ ID NO: 51 or SEQ ID NO: 52 or SEQ ID NO: 53 is indicative of catalytic activity of BoNT/F1 and serotype variants thereof;
(g) a cleavage product of the peptide or polypeptide comprising SEQ ID NO: 10 or SEQ ID NO: 54 or SEQ ID NO: 55 is indicative of catalytic activity of BoNT/F5, BoNT/H (also called BoNT/HA or BoNT/FA) and serotype variants thereof;
(h) a cleavage product of the peptide or polypeptide comprising SEQ ID NO: 11 or SEQ ID NO: 12 or SEQ ID NO: 56 or SEQ ID NO: 57 is indicative of catalytic activity of TeNT and serotype variants thereof;
(i) a cleavage product of the peptide or polypeptide comprising SEQ ID NO: 58 is indicative of catalytic activity of BoNT/X and serotype variants thereof;
(j) a cleavage product of the peptide or polypeptide comprising SEQ ID NO: 13 or SEQ ID NO: 14 is indicative of catalytic activity of BoNT/A1 and/or BoNT/C and serotype variants thereof;
(k) a cleavage product of the peptide or polypeptide comprising SEQ ID NO: 15 is indicative of catalytic activity of BoNT/A1 and/or BoNT/E1 and serotype variants thereof;
(l) a cleavage product of the peptide or polypeptide comprising SEQ ID NO: 16 is indicative of catalytic activity of BoNT/C and/or BoNT/E1 and serotype variants thereof;
(m) a cleavage product of the peptide or polypeptide comprising SEQ ID NO: 2 or SEQ ID NO: 3 is indicative of catalytic activity of BoNT/B and/or BoNT/X and serotype variants thereof;
(n) a cleavage product of the peptide or polypeptide comprising SEQ ID NO: 5 or SEQ ID NO: 6 or SEQ ID NO: 59 or SEQ ID NO: 60 or SEQ ID NO: 61 is indicative of catalytic activity of BoNT/D and/or BoNT/X and serotype variants thereof;
(o) a cleavage product of the peptide or polypeptide comprising SEQ ID NO: 9 or SEQ ID NO: 62 or SEQ ID NO: 63 or SEQ ID NO: 64 is indicative of catalytic activity of BoNT/F1 and/or BoNT/X and serotype variants thereof;
(p) a cleavage product of the peptide or polypeptide comprising SEQ ID NO: 10 is indicative of catalytic activity of BoNT/F5 and/or BoNT/H and/or BoNT/X and serotype variants thereof;
(q) a cleavage product of the peptide or polypeptide comprising SEQ ID NO: 11 or SEQ ID NO: 12 is indicative of catalytic activity of TeNT and/or BoNT/X and serotype variants thereof;
(r) a cleavage product of the peptide or polypeptide comprising SEQ ID NO: 17 or SEQ ID NO: 74 is indicative of catalytic activity of TeNT and/or BoNT/B 1 and/or BoNT/X and serotype variants thereof;
(s) a cleavage product of the peptide or polypeptide comprising SEQ ID NO: 65 is indicative of catalytic activity of BoNT/D and/or BoNT/F5 and/or BoNT/H and serotype variants thereof;
(t) a cleavage product of the peptide or polypeptide comprising SEQ ID NO: 18 or SEQ ID NO: 66 is indicative of catalytic activity of BoNT/D and/or BoNT/F5 and/or BoNT/H and/or BoNT/X and serotype variants thereof;
(u) a cleavage product of the peptide or polypeptide comprising SEQ ID NO: 19 is indicative of catalytic activity of BoNT/F1 and/or BoNT/F5 and/or BoNT/H and/or BoNT/X and serotype variants thereof;
(v) a cleavage product of the peptide or polypeptide comprising SEQ ID NO: 67 is indicative of catalytic activity of BoNT/D and/or BoNT/F1 and serotype variants thereof;
(w) a cleavage product of the peptide or polypeptide comprising SEQ ID NO: 20 or SEQ ID NO: 68 is indicative of catalytic activity of BoNT/D and/or BoNT/F1 and/or BoNT/X and serotype variants thereof;
(x) a cleavage product of the peptide or polypeptide comprising SEQ ID NO: 21 or SEQ ID NO: 22 is indicative of catalytic activity of BoNT/A1 and/or BoNT/C and/or BoNT/E1 and serotype variants thereof;
(y) a cleavage product of the peptide or polypeptide comprising SEQ ID NO: 23 or SEQ ID NO: 69 is indicative of catalytic activity of BoNT/D and/or BoNT/F1 and/or BoNT/F5 and/or BoNT/H and/or BoNT/X and serotype variants thereof;
(z) a cleavage product of the peptide or polypeptide comprising SEQ ID NO: 24 is indicative of catalytic activity of TeNT and/or BoNT/D and/or BoNT/F1 and/or BoNT/F5 and/or BoNT/H and/or BoNT/X and serotype variants thereof;
(aa) a cleavage product of the peptide or polypeptide comprising SEQ ID NO: 25 is indicative of catalytic activity of TeNT and/or BoNT/B1 and/or BoNT/F1 and/or BoNT/F5 and/or BoNT/H and/or BoNT/X and serotype variants thereof;
(bb) a cleavage product of the peptide or polypeptide comprising SEQ ID NO: 26 or SEQ ID NO: 27 is indicative of catalytic activity of TeNT and/or BoNT/B1 and/or BoNT/D and/or BoNT/F5 and/or BoNT/H and/or BoNT/X and serotype variants thereof;
(cc) a cleavage product of the peptide or polypeptide comprising SEQ ID NO: 71 is indicative of catalytic activity of TeNT and/or BoNT/B 1 and/or BoNT/F5 and/or BoNT/H and/or BoNT/X and serotype variants thereof;
(dd) a cleavage product of the peptide or polypeptide comprising SEQ ID NO: 28 or SEQ ID NO: 72 is indicative of catalytic activity of TeNT and/or BoNT/B1 and/or BoNT/D and/or BoNT/F1 and/or BoNT/X and serotype variants thereof;
(ee) a cleavage product of the peptide or polypeptide comprising SEQ ID NO: 29 is indicative of catalytic activity of BoNT/B1 and/or BoNT/D and/or BoNT/F1 and/or BoNT/F5 and/or BoNT/H and/or BoNT/X and serotype variants thereof;
(ff) a cleavage product of the peptide or polypeptide comprising SEQ ID NO: 73 is indicative of catalytic activity of TeNT and/or BoNT/B1 and/or BoNT/D and/or BoNT/F1 and/or BoNT/F5 and/or BoNT/H and serotype variants thereof; and
(gg) a cleavage product of the peptide or polypeptide comprising SEQ ID NO: 30 or SEQ ID NO: 31 is indicative of catalytic activity of TeNT and/or BoNT/B1 and/or BoNT/D and/or BoNT/F1 and/or BoNT/F5 and/or BoNT/H and/or BoNT/X and serotype variants thereof.

In a preferred embodiment, said method of the present teaching comprises a step of mass spectrometry or is an Endopep-MS assay.

In another embodiment the method of the present teaching is performed by making use of a peptide or polypeptide that can only be proteolytically cleaved by the protease of a single serotype so that the observation of a cleavage product is indicative of the catalytic activity of said protease. In this embodiment, (a) the catalytic activity of BoNT/A1, and serotype variants thereof, is established by detecting a cleavage product of a peptide or polypeptide comprising the amino acid sequence as shown in SEQ ID NO: 1; (b) the catalytic activity of BoNT/B, and serotype variants thereof, is established by detecting a cleavage product of a peptide or polypeptide comprising the amino acid sequence as shown in SEQ ID NO: 43 or 44; (c) the catalytic activity of BoNT/C, and serotype variants thereof, is established by detecting a cleavage product of a peptide or polypeptide comprising the amino acid sequence as shown in SEQ ID NO: 4; (d) the catalytic activity of BoNT/D, and serotype variants thereof, is established by detecting a cleavage product of a peptide or polypeptide comprising the amino acid sequence as shown in any one of SEQ ID NOs: 45-49; (e) the catalytic activity of BoNT/E, and serotype variants thereof, is established by detecting a cleavage product of a peptide or polypeptide comprising the amino acid sequence as shown in SEQ ID NO: 7 or 8; (f) the catalytic activity of BoNT/F, and serotype variants thereof, is established by detecting a cleavage product of a peptide or polypeptide comprising the amino acid sequence as shown in any one of SEQ ID NOs: 50-53; (g) the catalytic activity of BoNT/F5 and/or BoNT/H, and serotype variants thereof, is established by detecting a cleavage product of a peptide or polypeptide comprising the amino acid sequence as shown in SEQ ID NO: 54 or 55; (h) the catalytic activity of BoNT/X, and serotype variants thereof, is established by detecting a cleavage product of a peptide or polypeptide comprising the amino acid sequence as shown in SEQ ID NO: 58; and (i) the catalytic activity of TeNT, and serotype variants thereof, is established by detecting a cleavage product of a peptide or polypeptide comprising the amino acid sequence as shown in SEQ ID NO: 56 or 57.

As used herein, the term "indicative of catalytic activity of BoNT/A1" means that any of the cleavage products observed have been generated by the proteolytic activity of BoNT/A1. The term "indicative of catalytic activity of BoNT/B" means that any of the cleavage products observed have been generated by the proteolytic activity of BoNT/B. The term "indicative of catalytic activity of BoNT/C" means that any of the cleavage products observed have been generated by the proteolytic activity of BoNT/C. The term "indicative of catalytic activity of BoNT/D" means that any of the cleavage products observed have been generated by the proteolytic activity of BoNT/D. The term "indicative of catalytic activity of BoNT/E" means that any of the cleavage products observed have been generated by the proteolytic activity of BoNT/E. The term "indicative of catalytic activity of BoNT/F" means that any of the cleavage products observed have been generated by the proteolytic activity of BoNT/F. The term "indicative of catalytic activity of BoNT/F1" means that any of the cleavage products observed have been generated by the proteolytic activity of BoNT/F5. the term "indicative of catalytic activity of BoNT/F5" means that any of the cleavage products observed have been generated by the proteolytic activity of BoNT/F5. The term "indicative of catalytic activity of BoNT/H" means that any of the cleavage products observed have been generated by the proteolytic activity of BoNT/H. The term "indicative of catalytic activity of BoNT/X" means that any of the cleavage products observed have been generated by the proteolytic activity of BoNT/X. The term "indicative of catalytic activity of TeNT" means that any of the cleavage products observed have been generated by the proteolytic activity of TeNT.

In a preferred embodiment, the term "indicative of catalytic activity of BoNT/A1"also means that the peptide or polypeptide comprising SEQ ID NO: 1 of the sequence listing cannot be cleaved by any of BoNT/B1, BoNT/B2, BoNT/B3, BoNT/B4bv, BoNT/B5nP, BoNT/B6, BoNT/B7, BoNT/B8, BoNT/B9, BoNT/C, BoNT/CD, BoNT/D, BoNT/DC, BoNT/E1, BoNT/E2, BoNT/E3, BoNT/E4, BoNT/E5, BoNT/E6, BoNT/E7, BoNT/E8, BoNT/E9, BoNT/E10, BoNT/E11, BoNT/E12, BoNT/F1, BoNT/F2, BoNT/F3, BoNT/F4, BoNT/F5, BoNT/F6, BoNT/F7, BoNT/F8, BoNT/F9, BoNT/H (also called BoNT/H and BoNT/FA), BoNT/G, BoNT/X and TeNT or the sequence variants of the aforementioned neurotoxins.

In a preferred embodiment, the term "indicative of catalytic activity of BoNT/B 1" means that the peptide or polypeptide comprising SEQ ID NO: 43 or 44 of the sequence listing cannot be cleaved by any of BoNT/A1, BoNT/A2, BoNT/A3, BoNT/A4, BoNT/A5, BoNT/A6, BoNT/A7, BoNT/A8, BoNT/C, BoNT/CD, BoNT/D, BoNT/DC, BoNT/E1, BoNT/E2, BoNT/E3, BoNT/E4, BoNT/E5, BoNT/E6, BoNT/E7, BoNT/E8, , BoNT/E9 BoNT/E 10, BoNT/E11, BoNT/E12, BoNT/F1, BoNT/F2, BoNT/F3, BoNT/F4, BoNT/F5, BoNT/F6, BoNT/F7, BoNT/F8, BoNT/F9, BoNT/H (also called BoNT/HA and BoNT/FA) , BoNT/G, BoNT/X and TeNT or the sequence variants of the aforementioned neurotoxins.

In a preferred embodiment, the term "indicative of catalytic activity of BoNT/C" means that the peptide or polypeptide comprising SEQ ID NO: 4 of the sequence listing cannot be cleaved by any of BoNT/A1, BoNT/A2, BoNT/A3, BoNT/A4, BoNT/A5, BoNT/A6, BoNT/A7, BoNT/A8, BoNT/B1, BoNT/B2, BoNT/B3, BoNT/B4bv, BoNT/B5nP, BoNT/B6, BoNT/B7, BoNT/B8, BoNT/B9, BoNT/D, BoNT/DC, BoNT/E1, BoNT/E2, BoNT/E3, BoNT/E4, BoNT/E5, BoNT/E6, BoNT/E7, BoNT/E8, BoNT/E9, BoNT/E10, BoNT/E11, BoNT/E12, BoNT/F1, BoNT/F2, BoNT/F3, BoNT/F4, BoNT/F5, BoNT/F6, BoNT/F7, BoNT/F8, BoNT/F9, BoNT/H (also called BoNT/HA and BoNT/FA), BoNT/G, BoNT/X and TeNT or the sequence variants of the aforementioned neurotoxins.

In a preferred embodiment, the term "indicative of catalytic activity of BoNT/D" means that the peptide or polypeptide comprising any one of SEQ ID NOs: SEQ ID NOs: 45-49 of the sequence listing cannot be cleaved by any of BoNT/A1, BoNT/A2, BoNT/A3, BoNT/A4, BoNT/A5, BoNT/A6, BoNT/A7, BoNT/A8, BoNT/B1, BoNT/B2, BoNT/B3, BoNT/B4bv, BoNT/B5nP, BoNT/B6, BoNT/B7, BoNT/B8, BoNT/B9, BoNT/C, BoNT/CD, BoNT/E1, BoNT/E2, BoNT/E3, BoNT/E4, BoNT/E5, BoNT/E6, BoNT/E7, BoNT/E8, BoNT/E9, BoNT/E10, BoNT/E11, BoNT/E12, BoNT/F1, BoNT/F2, BoNT/F3, BoNT/F4, BoNT/F5, BoNT/F6, BoNT/F7, BoNT/F8, BoNT/F9, BoNT/H (also called BoNT/HA and BoNT/FA), BoNT/G, BoNT/X and TeNT or the sequence variants of the aforementioned neurotoxins.

In a preferred embodiment, the term "indicative of catalytic activity of BoNT/E1" means that the peptide or polypeptide comprising SEQ ID NO: 7 or 8 of the sequence listing cannot be cleaved by any of BoNT/A1, BoNT/A2, BoNT/A3, BoNT/A4, BoNT/A5, BoNT/A6, BoNT/A7, BoNT/A8, BoNT/B1, BoNT/B2, BoNT/B3, BoNT/B4bv, BoNT/B5nP, BoNT/B6, BoNT/B7, BoNT/B8, BoNT/B9, BoNT/C, BoNT/CD, BoNT/D, BoNT/DC, BoNT/F1, BoNT/F2, BoNT/F3, BoNT/F4, BoNT/F5, BoNT/F6, BoNT/F7, BoNT/F8, BoNT/F9, BoNT/H (also called BoNT/HA and BoNT/FA), BoNT/G, BoNT/X and TeNT or the sequence variants of the aforementioned neurotoxins.

In a preferred embodiment, the term "indicative of catalytic activity of BoNT/F1" means that the peptide or polypeptide comprising any one of SEQ ID NOs: 50-53 of the sequence listing cannot be cleaved by any of BoNT/A1, BoNT/A2, BoNT/A3, BoNT/A4, BoNT/A5, BoNT/A6, BoNT/A7, BoNT/A8, BoNT/B1, BoNT/B2, BoNT/B3, BoNT/B4bv, BoNT/B5nP, BoNT/B6, BoNT/B7, BoNT/B8, BoNT/B9, BoNT/C, BoNT/CD, BoNT/D, BoNT/DC, BoNT/E1, BoNT/E2, BoNT/E3, BoNT/E4, BoNT/E5, BoNT/E6, BoNT/E7, BoNT/E8, BoNT/E9, BoNT/E10, BoNT/E11, BoNT/E12, BoNT/F5, BoNT/H (also called BoNT/HA and BoNT/FA), BoNT/G, BoNT/X and TeNT or the sequence variants of the aforementioned neurotoxins.

In a preferred embodiment, the term "indicative of catalytic activity of BoNT/F5, BoNT/H" means that the peptide or polypeptide comprising SEQ ID NO: 54 or 55 of the sequence listing cannot be cleaved by any of BoNT/A1, BoNT/A2, BoNT/A3, BoNT/A4, BoNT/A5, BoNT/A6, BoNT/A7, BoNT/A8, BoNT/B1, BoNT/B2, BoNT/B3, BoNT/B4bv, BoNT/B5nP, BoNT/B6, BoNT/B7, BoNT/B8, BoNT/B9, BoNT/C, BoNT/CD, BoNT/D, BoNT/DC, BoNT/E1, BoNT/E2, BoNT/E3, BoNT/E4, BoNT/E5, BoNT/E6, BoNT/E7, BoNT/E8, BoNT/E9 BoNT/E10, BoNT/E11, BoNT/E12, BoNT/F1, BoNT/F2, BoNT/F3, BoNT/F4, BoNT/F6, BoNT/F7, BoNT/F8, BoNT/F9, BoNT/G, BoNT/X and TeNT or the sequence variants of the aforementioned neurotoxins.

In a preferred embodiment, the term "indicative of catalytic activity of TeNT" means that the peptide or polypeptide comprising SEQ ID NO: 56 or 57 of the sequence listing cannot be cleaved by any of BoNT/A1, BoNT/A2, BoNT/A3, BoNT/A4, BoNT/A5, BoNT/A6, BoNT/A7, BoNT/A8, BoNT/B1, BoNT/B2, BoNT/B3, BoNT/B4bv, BoNT/B5nP, BoNT/B6, BoNT/B7, BoNT/B8, BoNT/B9, BoNT/C, BoNT/CD, BoNT/D, BoNT/DC, BoNT/E1, BoNT/E2, BoNT/E3, BoNT/E4, BoNT/E5, BoNT/E6, BoNT/E7, BoNT/E8, BoNT/E9, BoNT/E10, BoNT/E11, BoNT/E12, BoNT/F1, BoNT/F2, BoNT/F3, BoNT/F4, BoNT/F5, BoNT/F6, BoNT/F7, BoNT/F8, BoNT/F9, BoNT/G, BoNT/X and BoNT/H (also called BoNT/HA and BoNT/FA) or the sequence variants of the aforementioned neurotoxins.

In a preferred embodiment, the term "indicative of catalytic activity of BoNT/X" means that the peptide or polypeptide comprising SEQ ID NO: 58 of the sequence listing cannot be cleaved by any of BoNT/A1, BoNT/A2, BoNT/A3, BoNT/A4, BoNT/A5, BoNT/A6, BoNT/A7, BoNT/A8, BoNT/B1, BoNT/B2, BoNT/B3, BoNT/B4bv, BoNT/B5nP, BoNT/B6, BoNT/B7, BoNT/B8, BoNT/B9, BoNT/C, BoNT/CD, BoNT/D, BoNT/DC, BoNT/E1, BoNT/E2, BoNT/E3, BoNT/E4, BoNT/E5, BoNT/E6, BoNT/E7, BoNT/E8, BoNT/E9, BoNT/E10, BoNT/E11, BoNT/E12, BoNT/F1, BoNT/F2, BoNT/F3, BoNT/F4, BoNT/F5, BoNT/F6, BoNT/F7, BoNT/F8, BoNT/F9, BoNT/G, BoNT/H (also called BoNT/HA and BoNT/FA), TeNT or the sequence variants of the aforementioned neurotoxins.

As used herein, the term "serotype variant" comprises any variant of the neurotoxin within the same serotype. For example, a serotype variant of BoNT/A1 comprises any of BoNT/A2, BoNT/A3, BoNT/A4, BoNT/A5, BoNT/A6, BoNT/A7 and BoNT/A8. Preferably a serotype variant of BoNT/A1 is a variant BoNT polypeptide having an amino acid sequence identity of at least 50%, at least 60%, at least 70%, at least 80% or at least 85% in comparison to the amino acid sequence of BoNT/A1.

A serotype variant of BoNT/B1 comprises any BoNT/B2, BoNT/B3, BoNT/B4bv, BoNT/B5nP, BoNT/B6, BoNT/B7, BoNT/B8 and BoNT/B9. Preferably a serotype variant of BoNT/B1 is a variant BoNT polypeptide having an amino acid sequence identity of at least 50%, at least 60%, at least 70%, at least 80% or at least 85% in comparison to the amino acid sequence of BoNT/B 1.

A serotype variant of BoNT/E1 comprises any of BoNT/E2, BoNT/E3, BoNT/E4, BoNT/E5, BoNT/E6, BoNT/E7, BoNT/E8, BoNT/E9, BoNT/E10, BoNT/E11 and BoNT/E12. Preferably a serotype variant of BoNT/E1 is a variant BoNT polypeptide having an amino acid sequence identity of at least 50%, at least 60%, at least 70%, at least 80% or at least 85% in comparison to the amino acid sequence of BoNT/E1.

A serotype variant of BoNT/F1 comprises any of BoNT/F2, BoNT/F3, BoNT/F4, BoNT/F5, BoNT/F6, BoNT/F7, BoNT/F8 and BoNT/F9. Preferably a serotype variant of BoNT/F1 is a variant BoNT polypeptide having an amino acid sequence identity of at least 50%, at least 60%, at least 70%, at least 80% or at least 85% in comparison to the amino acid sequence of BoNT/F1.

A serotype variant of BoNT/C is a variant BoNT polypeptide having an amino acid sequence identity of at least 50%, at least 60%, at least 70%, at least 80% or at least 85% in comparison to the amino acid sequence of BoNT/C. A serotype variant of BoNT/D is a variant BoNT polypeptide having an amino acid sequence identity of at least 50%, at least 60%, at least 70%, at least 80% or at least 85% in comparison to the amino acid sequence of BoNT/D. A serotype variant of BoNT/H is a variant BoNT polypeptide having an amino acid sequence identity of at least 50%, at least 60%, at least 70%, at least 80% or at least 85% in comparison to the amino acid sequence of BoNT/H. A serotype variant of BoNT/G is a variant BoNT polypeptide having an amino acid sequence identity of at least 50%, at least 60%, at least 70%, at least 80% or at least 85% in comparison to the amino acid sequence of BoNT/G. A serotype variant of BoNT/X is a variant BoNT polypeptide having an amino acid sequence identity of at least 50%, at least 60%, at least 70%, at least 80% or at least 85% in comparison to the amino acid sequence of BoNT/X. A serotype variant of TeNT is a variant TeNT polypeptide having an amino acid sequence identity of at least 50%, at least 60%, at least 70%, at least 80% or at least 85% in comparison to the amino acid sequence of TeNT.

In an alternative embodiment, the term "serotype variant" as used herein is characterized by the capability of cleaving the identical peptide bond of the peptide or polypeptide or yielding the identical cleavage products.

In one embodiment, the peptide or polypeptide or cleavage product is coupled to a solid support. The peptide, polypeptide and/or cleavage product can be detectably labelled. The label can be attached within the peptide or polypeptide at an N-terminal or C-terminal amino acid residue with respect to the cleavage site or at an internal amino acid residue. The detectable label can be attached to an amino acid residue located N-terminal to a neurotoxin cleavage site or the detectable label can be attached to an amino acid residue located C-terminal said cleavage site. The present teaching, however, also encompasses peptides and polypeptides which are labelled at least one amino acid residue located N-terminal to the neurotoxin cleavage site and at least one amino acid residue located C-terminal to said cleavage site. In addition, the peptide or polypeptide described herein can be attached to a solid support. The peptide or polypeptide may be attached to a solid support via an amino acid residue located N-terminal to a neurotoxin cleavage site. However, the peptide or polypeptide may also be attached to a solid support via an amino acid residue located C-terminal to a neurotoxin cleavage site.

In other embodiments of the present teaching, the peptide or polypeptide described herein is attached to a solid support via an amino acid residue located N-terminal to a neurotoxin cleavage site and a detectable label is attached to an amino acid residue located C-terminal to said cleavage site. In another embodiment, the peptide or polypeptide is attached to a solid support via an amino acid residue located C-terminal to a neurotoxin cleavage site and a detectable label is attached to an amino acid residue located N-terminal to said cleavage site.

In one embodiment, the detectable label is a luciferase, a green, red, cyan, yellow fluorescent protein, in particular a superfolder green fluorescent protein (sfGFP) [77], mCherry [78], CFP, YFP [79] or a horseradish peroxidase [80], an alkaline phosphatase[81], β-galactosidase [82,83], or a radioactive isotope.

In another embodiment, the peptide, polypeptide or cleavage product comprises a tag for immobilization on a solid support. The tag for immobilization can be attached via an amino acid residue located N-terminal to a neurotoxin cleavage site or C-terminal to a neurotoxin cleavage site. Examples of tags comprise a strep-tag, a halo-tag (HA), a poly-his-tag, a poly-arginine-tag, a poly-HisAsn-tag, a HAT (natural histidine affinity)-tag, a S-tag, a flag-tag, a GST-tag, and a maltose-binding-protein-tag and derivatives of the aforementioned tags [84,85].

In one embodiment of the present teachings method, the presence or amount of peptide, polypeptide and/or cleavage product is determined from the detectable amount of cleavage product. The cleavage product may be the fragment arising from the amino acid sequence which is located in the uncleaved peptide or polypeptide N-terminal to the neurotoxin cleavage site and the fragment arising from the amino acid sequence which is located in the uncleaved peptide or polypeptide C-terminal to the neurotoxin cleavage site or both fragments. Preferably said fragment is labelled. If both fragments are labelled, it is preferred that the label of the fragments is different.

In another embodiment, the method described herein above comprises a step of comparing the amount of peptide, polypeptide or cleavage product detectable in step (ii) with the amount of peptide, polypeptide or cleavage product detectable in a reference sample. The reference sample preferably comprises the reference material of BoNT/A1, BoNT/B 1, BoNT/C, BoNT/D, BoNT/E1, BoNT/F1, BoNT/F5, BoNT/H, BoNT/X or of TeNT [86].

The present teaching also relates to a peptide or polypeptide comprising (a) an amino acid sequence selected from the group consisting of SEQ ID NOs: 1-31 and SEQ ID NOs: 43-74, (b) an amino acid sequence variant of SEQ ID NO: 75, wherein said variant comprises a fragment of at least 20 consecutive amino acids of SEQ ID NO: 75 with at least one mutation selected from the group consisting of D179K, R180W, I181E, E183I, N196Q, Q197N, R198K, and A199R or (c) an amino acid sequence variant of SEQ ID NO: 76 wherein said variant comprises a fragment of at least 20 consecutive amino acids of SEQ ID NO: 76 with at least one mutation selected from the group consisting of Q34A, V43A, M46I, L54A, L54E, E55Q, E55L, K59R, A67R, A69N, A72D, Q76V, and T79S.

As used herein, the term "mutation" refers to an amino acid modification of at least one amino acid residue of a reference sequence.

In one embodiment the peptide or polypeptide of the teaching comprises an amino acid sequence variant of SEQ ID NO: 75, wherein said variant comprises a fragment of at least 20 consecutive amino acids of SEQ ID NO: 75 with a least one mutation selected from the group consisting of (a) D179K and A199R, (b) D179K and N196Q and Q197N and R198K, (c) R180W and I181E and E183I, (d) D179K, (e) A199R, and (f) N196Q and Q197N and R198K.

In one embodiment the peptide or polypeptide of the teaching comprises an amino acid sequence variant of SEQ ID NO: 76, wherein said variant comprises a fragment of at least 20 consecutive amino acids of SEQ ID NO: 76 with a least one mutation selected from the group consisting of (a) T79S, (b) E55Q and K59R, (c) L54A and E55Q and K59R, (d) M46I and E55Q, (e) M46I and L54A and K59R, (f) M46I and E55Q and K59R and A72D, (g) M46I and L54A and E55Q and K59R and A72D, (h) M46I and L54A and E55Q and K59R, (i) K59R, (j) M46I, (k) E55Q, (1) A72D, (m) M46I, (n) K59R, (o) L54A and K59R, (p) E55Q, (q) A69N, (r) T79S, (s) A67R, (t) A67R and T79S, (u) E55Q and K59R and A67R, (v) L54A and E55Q and K59R and A67R, (w) L54E and E55L and K59R and A67R, (x) Q34A and K59R and A67R, (y) V43A and K59R and A67R, (z) M46I and E55Q and A67R, (aa) M46I and L54E and E55L and A67R, (bb) Q34A and M46I and A67R, (cc) V43A and M46I and A67R, (dd) M46I and L54A and K59R and A67R, (ee) M46I and K59R and A67R, (ff) M46I and E55Q and K59R and A67R and A72D, (gg) M46I and L54A and E55Q and K59R and A67R and A72D, (hh) M46I and L54A and E55Q and K59R and Q76V, (ii) L54E and E55L and K59R, (jj) Q34A and K59R, (kk) V43A and K59R, (11) M46I and L54E and E55L, (mm) Q34A and M46I, (nn) V43A and M46I, (oo) K59R and A67R, (pp) L54A and K59R, (qq) E55Q and A67R, (rr) L54A and E55Q, (ss) Q76V, (tt) M46I and K59R, (uu) L54A and E55Q, (vv) A67R, and (ww) M46I and L54A and E55Q and K59R.

The term "variant which comprises a fragment of at least 20 consecutive amino acids" refers to peptides and polypeptides of the teaching which comprise at least 20 amino acid residues of the reference sequence of SEQ ID NO: 75 or 76 with at least one mutation as identified above. Peptides and polypeptides of the teaching, which comprise a fragment of at least 20 consecutive amino acid residues of the reference sequence of SEQ ID NO: 75 or 76 include peptides and polypeptides which are variants of the full-length amino acid sequence of SEQ ID NOs: 75 and 76 with at least one mutation as identified above.

The present teaching also relates to a peptide or polypeptide comprising an amino acid sequence selected from the group consisting of SEQ ID NOs: 1-31 or SEQ ID NOs: 43-74.

The present teaching also relates to peptides or polypeptides comprising or consisting of the amino acid sequence of Synaptobrevin/VAMP-2 according to SEQ ID NO: 76 or of a fragment thereof. A fragment comprises at least 10, at least 20, at least 30, at least 40 at least 50 amino acid sequences. In a preferred embodiment, said fragment comprises up to 20, up to 30, up to 40, up to 50, up to 60, up to 70, up to 80, up to 90 amino acid sequences of Synaptobrevin/VAMP-2. A fragment is preferably selected from the group consisting of amino acids 1-94, 2-94, 1-76, 2-76, 40-94, and 60-94. Preferably, said peptides and polypeptides comprise at least one mutation at a position of Synaptobrevin/VAMP-2 selected from the group consisting of 34, 43, 46, 54, 55, 58, 59, 60, 66, 67, 69, 72, 76, 77, 79, 81, 82. In a preferred embodiment, the mutation at said position is Q34A, V43A, M46I, L54A, E55Q, K59R, A67R, A69N, A72D, Q76V, and T79S. In another preferred embodiment the peptide or polypeptide comprises a mutation or combination of mutations selected from the group consisting of T79S, E55Q and K59R, L54A and E55Q and K59R, M46I and E55Q, M46I and L54A and K59R, M46I and E55Q and K59R and A72D, M46I and L54A and E55Q and K59R and A72D, M46I and L54A and E55Q and K59R, K59R, M46I, E55Q, A72D, M46I, K59R, L54A K59R, E55Q, A69N, T79S, A67R, A67R and T79S, E55Q and K59R and A67R, L54A and E55Q and K59R and A67R, L54E and E55L and K59R and A67R, Q34A and K59R and A67R, V43A and K59R and A67R, M46I and E55Q and A67R, M46I and L54E and E55L and A67R, Q34A and M46I and A67R, V43A and M46I and A67R, M46I and L54A and K59R and A67R, M46I and K59R and A67R, M46I and E55Q and K59R and A67R and A72D , M46I and L54A and E55Q and K59R and A67R and A72D, M46I and L54A and E55Q and K59R and Q76V, L54E and E55L and K59R, Q34A and K59R, V43A and K59R, M46I and L54E and E55L, Q34A and M46I, V43A and M46I, K59R and A67R, L54A and K59R, E55Q and A67R, L54A and E55Q, Q76V, M46I and K59R, L54A and E55Q, A67R, M46I and L54A and E55Q and K59R.

According to the teaching of the present application, the amino acid sequence of Synaptobrevin/VAMP-2 at position 66 can also be replaced with a mutation selected from the group consisting of R66A, R66I, R66L, R66V and R66T which renders the peptide or polypeptide less susceptible to cleavage by BoNT/X. Wherein less susceptible is preferably a cleavage reduction of at least 50%, at least 60%, at least 70%, at least 80%, or at least 90%. For example, R66A is typically associated with a 100 fold reduced cleavage. In other words, the aforementioned mutations reduce cleavage of up to 100 fold or more.

According to the teaching of the present application, the above mutation of Synaptobrevin/VAMP-2 at position 67 can also be replaced with a mutation selected from the group consisting of A67K, A67E, A67D, A67Q, A67 N, A67F and A67W which renders the peptide or polypeptide less susceptible to cleavage by BoNT/X.

The present teaching also relates to peptides or polypeptides comprising or consisting of the amino acid sequence of SNAP-25 according to SEQ ID NO: 75 or of a fragment thereof. A fragment comprises at least 10, at least 20, at least 30, at least 40 at least 50 amino acid sequences. In a preferred embodiment, said fragment comprises up to 20, up to 30, up to 40, up to 50, up to 60, up to 70, up to 80, up to 90 amino acid sequences of SNAP-25. A fragment is preferably selected from the group consisting of amino acids 1-197, 2-197, 1-206, 2-206, 140-197, 146-206 of SNAP-25. Preferably, said peptides and polypeptides comprise at least one mutation at a position of SNAP-25 selected from the group consisting of 179, 180, 181, 183, 196, 197, 198, 199 of SNAP-25. Preferably said mutation is D179K, R180W, I181E, E183I, N196Q, Q197N, R198K, and A199R. In another preferred embodiment the peptide or polypeptide comprises a mutation or combination of mutations selected from the group consisting of D179K and A199R, D179K and N196Q and Q197N and R198K, R180W and I181E and E183I, D179K, A199R, N196Q and Q197N and R198K.

In one embodiment, the peptide or polypeptide comprises a tag for immobilization, preferably selected from the group consisting of a halo-tag (HA), strep-tag, a poly-his-tag, a poly-arginine-tag, a poly-HisAsn-tag, a HAT (natural histidine affinity)-tag, a S-tag, a flag-tag, a GST-tag, a maltose-binding-protein-tag.

In another embodiment, the peptide or polypeptide described herein comprises a detectable label, preferably selected from the group consisting of luciferase, a green, red, cyan, yellow fluorescent protein (GFP, mCherry, CFP, YFP) or a peroxidase, a phosphatase, β-galactosidase, radioactive isotope.

In another embodiment, the peptide or polypeptide described herein comprises at least one additional cleavage site for a protease, said protease being selected from the group consisting of TEV, Human rhinovirus 3C protease, Thrombin, Faktor Xa, SUMO-protease (ULP1/2, Senp1-7).

The present teaching also relates to the use of the peptide or polypeptide described herein above for specific detection of BoNT of serotype A, B, C, D, E, F, F5, H, X and variants thereof and TeNT.

Finally, the present teaching also relates to a kit comprising the peptide or polypeptide described herein above. In a preferred embodiment, the kit comprises at least one peptide or polypeptide.

The figures show:
**Figure 1****:** Overview of SNARE proteins SNAP-25 (A) and synaptobrevin/VAMP-2 (B) and derived generic peptidic substrates which are fused between an N-terminal His6- and Halotag (H6tHAtev) and a C-terminal reporter protein like luciferase (L). Serotype specific cleavage sites are indicated by black arrows.

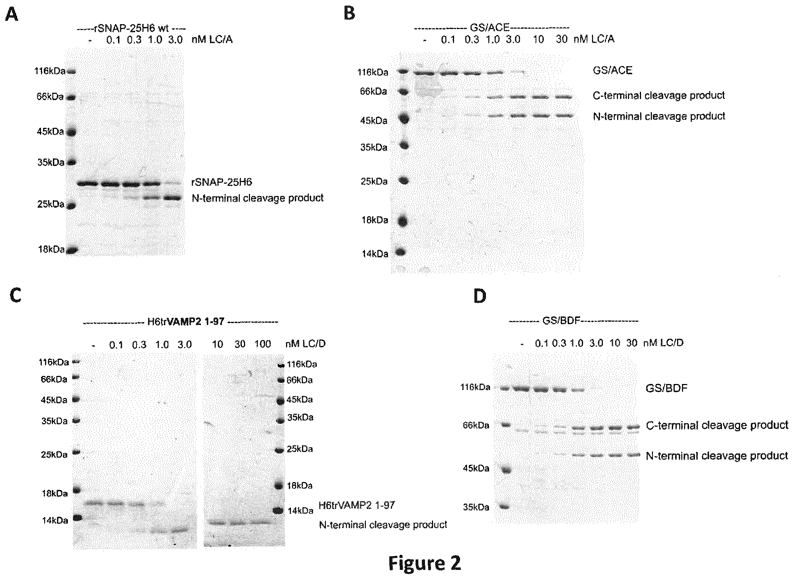

**Figure 2****:** Endopeptidase assay of rSNAP-25H6 wt (A) and H6tHA1S(146-206)L (B) with LC/A and H6trVAMP-2 2-97 (C) and H6tHAtev1V(2-94)LS (D) with LC/D for 1 h at 37°C in PBS pH 7.4. Analysis by SDS-PAGE, detection by Coomassie blue staining.

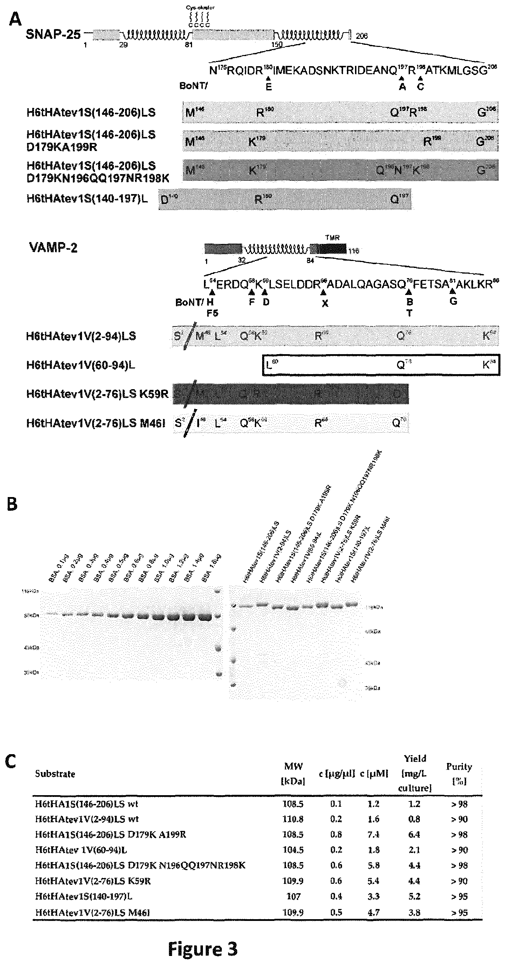

**Figure 3****:** Generic and serotype-specific substrates (GS/X, SSS/X). (A) Generic substrates H6tHAtev1S(146-206)LS (top) cleavable by BoNT/A, C and E and H6tHAtev1V(2-94)L (bottom) cleavable by BoNT/B, D, F, H, X and TeNT. H6tHAtev1S(146-206)LS D179KA199R is the serotype-specific substrate for BoNT/A (SSS/A), H6tHAtev1S(146-206)LS D179KN196QQ197NR198K is preferably cleaved by BoNT/C (SSS/C) and H6tHAtev1S(140-197)L is preferably cleaved by BoNT/E (SSS/E). Analogous, H6tHAtev1V(60-94)L (SSS/B) is hydrolyzed only by BoNT/B and X, H6tHAtev1V(2-76)LS K59R (SSS/D) is cleaved by BoNT/D, F5/H and X, while H6tHAtev1V(2-76)LS M46I (SSS/F) is cleaved by BoNT/F, F5/H and X. (B) 12.5% SDS-PAGE analysis of generic and serotype-specific substrates, detection by Coomassie blue staining. (C) MW, yield, concentration and purity of GS/X and the six SSS/X.

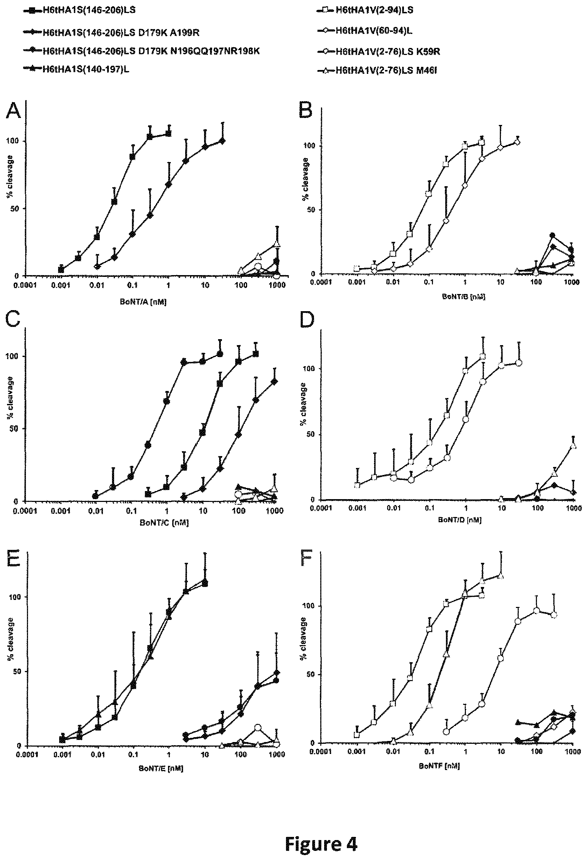

**Figure 4****:** Determination of EC₅₀ for BoNT/A-F for the six serotype specific substrates (SSS/X) and the generic substrates H6tHA1S(146-206)LS and H6tHA1V(2-94)LS. Assay in JDZ-buffer for 1 h of incubation at 37°C (mean ± SD, n = 3-4).

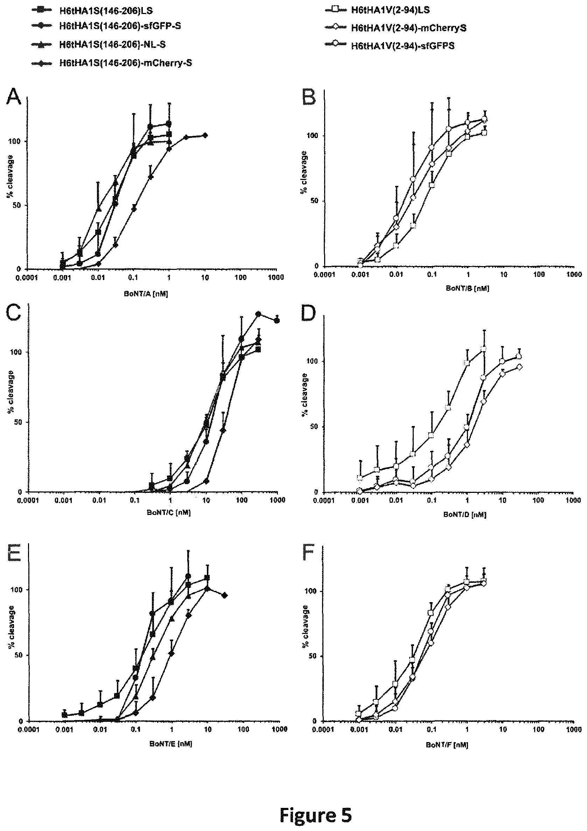

**Figure 5****:** Analysis of cleavage the generic substrate peptide H6tHA1S(146-206) or H6tHA1V(2-94), respectively, with three to four different detector molecules luciferase (L), NanoLuc^{®} Luciferase (NL), superfolder green-fluorescent Protein (sfGFP) and mCherry by different concentrations of BoNT/A-F in JDZ-buffer after 1 h of incubation at 37°C (A-F). (G) Determination of the EC₅₀ of the generic substrate peptides with different reporter molecules for BoNT/A-F.

The following Examples illustrate the teaching.

### EXAMPLES

### Example 1: Methods

### Plasmid construction

The genes encoding LC/A1 1-429, LC/B1 1-436, LC/C 1-436, LC/D 1-436, LC/E1 1-411 and LC/F1 1-428, LC/G 1-435 and LC/T 1-438 fused to a C-terminal 6xHN-tag were inserted into the vector pPROTet.E133 (CLONTECH Laboratories, Inc.). The DNA fragments encoding LC/C-6xHN and LC/D-6xHN were also inserted into a pQe3 vector via EcoR I/Pst I sites.

The plasmid pET-HAtev2SL was generated by inserting the fragment encoding the halo-tag (HA) and TEV cleavage site from pHA2SL into the plasmid pET-2SL [87]. Optionally, a StrepTag (S) was fused to the C-terminus of H6tHAtev2SL resulting in the plasmid pH6tHAtev2SLS. Restriction sites Kpn I and BamH I were used to insert different DNA fragments encoding the various SNAP-25 and VAMP-2 based peptides between TEV and luc. Single site mutations were introduced via PCR applying the GeneTailor^{™} site-directed mutagenesis system (Life Technologies, Darmstadt, Germany) and suitable primers (Eurofins, Ebersberg, Germany) using pH6tHAtev1SL(S) or pH6tHAtev1VL(S) as template DNA. PCR products encoding the different reporter molecules sfGFP, mCherry and NanoLuc^{®} Luciferase were inserted via a BamH I and Nhe I restriction site.

Nucleotide sequences of all newly generated constructs were verified by DNA sequencing (GATC Biotech, Konstanz, Germany).

### Expression and purification of recombinant proteins

Recombinant LC/X-6xHN were expressed utilizing the E. coli strain TG1 in 2YT medium supplemented with chloramphenicol (Cm, 34 mg/L) during 16 h of induction at 22°C in the presence of anhydrotetracyclin (aTC, 100 ng/mL) or in case of LC/C-6xHN and LC/D-6xHN encoded in a pQe3 plasmid in E. coli M15 pRep4 strain as described previously [88]. LC/X-6xHN were isolated employing Co²⁺-Talon matrix (Takara Bio Europe S.A.S., Saint-Germain-en-Laye, France) and eluted in 50 mM Tris-HCl, pH 8.0, 150 mM NaCl, 250 mM imidazole. Desired eluates were subjected to HPLC in 20 mM HEPES, pH 7.2, 150 mM K-glutamate buffer (Superdex-200 16/60 column or HiPrep 16/10 Q FF, GE Healthcare, Germany) or dialyzed against 20 mM HEPES, pH 7.2, 150 mM K-glutamate, frozen in liquid nitrogen and kept at -70°C.

The generation of recombinant, activated full-length BoNT/A-F, BoNT/G, BoNT/X and TeNT was described previously [86,89,90,111].

Recombinant rat SNAP-25 aa 1-206 fused to a C-terminal His6tag (rSNAP-25H6) was expressed in E. coli M15 pRep4 strain as described previously [43]. The plasmid pET15b-VAMP-2 [52] encoding rat VAMP-2 aa 1-97 fused to an N-terminal thrombin cleavable His6tag (H6trVAMP-2 1-97) was expressed in E. coli BL21DE3 strain upon induction by IPTG. Expression of all substrate constructs H6tHAtevXY(S) (X=SNARE peptide, Y=detector molecule) was done following a modified protocol of Stevens et al. [87]. The harvested cells were resuspended in 0.1 M Tris/HCl pH 7.6, 0.15 M NaCl, 5 mM MgCl₂ supplemented with 1 mg/mL lysozyme. After centrifugation the clear cell lysate was applied to a Co²⁺-Talon matrix (Takara Bio Europe S.A.S., Saint-Germain-en-Laye, France). The matrix was washed first and finally with 50 mM Tris/HCl pH 7.6, 150 mM NaCl, 5 mM Imidazole, in between with washing buffer containing 1 M NaCl instead. The protein of interest was eluted by washing buffer supplemented with 100 mM imidazole. Desired eluates were pooled and subjected to gel filtration (Superdex-200 16/60 column, GE Healthcare, Germany) in PBS pH 7.4. The substrates comprising a C-terminal StrepTag were subsequently purified on Strep-Tactin^{®} Superflow^{®} matrix (IBA) according to the manufacturer's instructions and eluted in 0.1 M Tris/HCl pH 7.6, 0.15 m NaCl supplemented with 10 mM desthiobiotin. The desired eluates were dialyzed against PBS pH 7.4 or JDZ-buffer. All recombinant proteins were shock-frozen in liquid nitrogen, and kept at - 70°C.

Protein concentrations were determined subsequent to SDS-PAGE and Coomassie blue staining by densitometry using a LAS-3000 imaging system (Fuji Photo Film), the AIDA 3.51 software (Raytest, Straubenhardt, Germany) and BSA (100-1600 ng) as reference protein.

### Endopeptidase assay

The endopeptidase assays were performed in PBS pH 7.4 (for LC) or JDZ-buffer (50 mM HEPES pH 7, 20 mM DTT, 250 µM ZnCl₂, 0.5 % Tween-20, for BoNT) unless otherwise stated in a total volume of 20 µl. The substrates (0.45 µM) and serially diluted LC or BoNT were incubated for 1 h at 37 °C. The reactions were stopped by the addition of 7 µl 4x Laemmli buffer. Samples were denatured at 99 °C for 2 min and then subjected to 10 % or 12.5 % SDS-PAGE. Protein bands were detected by Coomassie blue staining and subsequently quantified by densitometry using the software TINA (version 2.09f, Raytest, Straubenhardt, Germany).

### Example 2: Development of substrates for detecting BoNT/A, C and E

The SNARE protein SNAP-25 is exclusively cleaved by BoNT/A, C and E at unique peptide bonds. BoNT/A hydrolyses at Q197-R198 [41,43,91] whereas BoNT/C proteolyses SNAP-25 at the neighboring peptide bond R198-A199 [45] and BoNT/E further upstream between R180-1181 [41,43]. In the design of a generic substrate suitable for these three BoNT, a minimal substrate should include the SNARE motif S4 (residues 145-155 of SNAP-25) which is thought to be important for cleavage [53,92]. Subsequently, it was proposed that SNAP-25 146-202 is the minimum essential peptide for BoNT/A cleavage while BoNT/C needs the SNAP-25 peptide 93-202 [49,51]. We inserted the peptide 146-206 of SNAP-25 into the modified construct to serve as generic substrate for BoNT/A, C and E to yield H6tHAtev1S(146-206)L(S) in high yield and >99% purity (Figure 1 & 3).

To identify negative effects of the large N-terminal H6tHAtev-tag, the C-terminal reporter molecule luciferase and truncations of the SNAP-25 peptide on substrate hydrolysis the newly generated H6tHAtev1S(146-206)L substrate was compared with the full-length wild-type SNAP-25 (1-206) substrate fused only C-terminal to a small His6Tag (rSNAP25-H6) [88] (Figure 1). In an endopeptidase assay H6tHAtev1S(146-206)L was incubated each with LC/A, LC/C and LC/E in PBS pH 7.4 for 1 hour at 37°C. The EC₅₀ values were determined for both substrate constructs by SDS-PAGE (Figure 2). As shown in Table 1, fusion of tags and reporter protein as well as truncations of SNAP-25 did not impair cleavage by LC/A or LC/E, but increased EC₅₀ values two-fold. Only LC/C shows a very high EC₅₀ value for the H6tHAtev1S(146-206)L substrate, but it is known that SNAP-25 is a poor *in vitro* substrate for LC/C, as can be seen by the much higher EC₅₀ values compared to those of LC/A and LC/E. We also analyzed the cleavage of H6tHAtev1S(146-206)LS by the full-length BoNT/A, C and E in an optimized cleavage buffer (JDZ-buffer, 50 mM HEPES pH 7.2, 20 mM DTT, 200 µM ZnCl₂, 0.5 % Tween-20) (Figure 4). Here, the EC₅₀ values of BoNT/A, C and E were determined as 0.03 nM, 10 nM and 0.2 nM, respectively (Table 3). The cleavage rate of full-length BoNT in the optimized JDZ-buffer was clearly increased compared to LC in PBS buffer. All in all, H6tHAtev1S(146-206)L(S) can be used as a generic substrate to detect low concentrations of BoNT/A, BoNT/C and BoNT/E.

**Table 1: Endopeptidase assay of H6tHAtev1S(146-206)L compared to rSNAP-25H6 wt by LC/A, LC/C and LC/E in PBS pH 7.4, for 1 h at 37°C (mean ± SD, n = 3).**

| Substrate | ECso [nM] | | |
|---|---|---|---|
| | LC/A | LC/C | LC/E |
| **rSNAP-25H6 wt** | 1.67 ±0.58 | 2100 ± 100 | 0.40 ± 0.17 |
| **H6tHAtev1S(146-206)L** | 0.87 ±0.23 | >3000 | 0.17 ± 0.06 |

### Example 3: Development of substrates for detecting BoNT/B, D, F, F5/H, X and TeNT

BoNT/B, D, F, F5/H and G cleave VAMP-2 at different peptide bonds (BoNT/B: Q76-F77 [32], BoNT/D: K59-L60 [36], BoNT/F: Q58-K59 [33], BoNT/G: A81-A82 [37], BoNT/F5 and BoNT/H: L54-E55 [39,40], BoNT/X: R66-A67 [111]). Only TeNT cleaves VAMP-2 at the same site as BoNT/B (Q76-F77 [32,38]) (Figure 1).

It is known that BoNT/B needs at least 12 residues on both the N-terminal and C-terminal side of its cleavage site [93]. Schmidt et al. showed that the VAMP-2 peptide 35-75 is a suitable substrate for BoNT/D and BoNT/F [94] while BoNT/F7 produced by C. baratii requires residues 27-70 for optimal cleavage [95]. To cover also the substrate requirements of BoNT/G we choose a peptide covering aa 2-94 of VAMP-2 as the generic substrate for BoNT/B, D, F, F5/H, G, X and TeNT which was inserted into the modified construct mentioned above yielding H6tHAtev1V(2-94)L(S) in high yield and >95% purity (Figure 1 & 3).

**Table 2: Endopeptidase assay of H6tHAtev1V(2-94)L(S) compared to H6trVAMP-2 2-97 in PBS pH 7.4, for 1 h at 37°C (mean ± SD; n.d., not determined).**

| Substrate | ECso [nM] | | | | | |
|---|---|---|---|---|---|---|
| | LC/B | LC/D | LC/F | LC/F5 | LC/G | LC/T |
| **H6trVAMP-2 2-97 (n=1)** | 60 | 1 | 2 | n.d. | >10,000 | 10 |
| **H6tHAtev1V(2-94)L (n=2-3)** | 160 ± 69 | 1.3 ±0.35 | 6.0 ± 265 | n.d. | >10,000 | n.d. |
| **H6tHAtev1V(2-94)LS (n=2-3)** | 80 ± 26 | 0.6 ±0.07 | 2.3 ±0.35 | 30 ± 0 | >10,000 | 83 ± 6 |

Cleavage of H6tHAtev1V(2-94)L(S) by LC/B, D, F, F5, G, and LC/T was compared with the cleavage of an N-terminal His6-tagged VAMP-2 lacking the C-terminal transmembrane domain (TMD) (H6trVAMP-2 21-97) in PBS pH 7.4. The EC₅₀ of LC/B and LC/F for H6tHAtev1V(2-94)LS was factor 1.5-3 higher than for H6trVAMP-2 2-97 while the EC₅₀ for LC/D was equal for both substrates. LC/F5 cleaved H6tHAtev1V(2-94)LS more efficient than LC/B and LC/T. The EC₅₀ value for the LC/G was above the maximum concentration of LC/G. One can speculate that absence of TMD made VAMP-2 uncleavable by LC/G. The cleavage of H6tHAtev1V(2-94)LS by LC/T was decreased by factor 10 which might be attributed to the N-terminal halo-tag interfering with substrate recognition by LC/T. The order of LC activity in PBS employing H6trVAMP-2 2-97 as well as H6tHAtev1V(2-94)L is LC/D > LC/F > LC/F5 > LC/T > > LC/B >>> LC/G.

In conclusion, albeit cleavage of H6tHAtev1V(2-94)L(S) by LC/G is not detectable the most relevant serotypes LC/B, D, F, F5 and LC/T hydrolyze H6tHAtev1V(2-94)L(S) similarly to the H6trVAMP-2 2-97. We also analysed the cleavage of H6tHAtev1V(2-94)L(S) by the activated, full-length BoNT/B, BoNT/D and BoNT/F in the optimized JDZ-buffer (Figure 4). The EC₅₀ values for BoNT/B, D and F are 0.07 nM, 0.2 nM and 0.03 nM (Table 3) and thus clearly in the picomolar concentration range which is required for sensitive detection methods. In different buffer conditions (PBS pH 7.4 supplemented with 20 µM ZnCl₂,0.5 % Tween-20 and 5 mM DTT), the cleavage of H6tHAtev1V(2-94)L(S) yielded EC₅₀ values of 2.5 nM, 30 nM, 20 nM and 0.2 nM for BoNT/F1, LC/F5, BoNT/H and BoNT/X, respectively (Table 4).

### Example 4: Serotype specific substrates (SSS) for BoNT/A, C and E

Apart from highly sensitive detection of BoNT employing the generic substrates mentioned above, it is desirable to identify the serotype of BoNT to execute e.g. optimal medical treatment. The availability, characterisation and shelf life of immunological reagents for serotyping as well as the performance of the respective assays often leaves room for improvement. Therefore, we designed peptides that can only be cleaved by a single BoNT and called them serotype X specific substrates (SSS/X). To obtain hydrolysis by only one serotype, e.g. BoNT/A, the H6tHAtev1S(146-206)L has to be rendered insensitive to BoNT/C and BoNT/E by introducing point mutations and/or truncations either in the respective SNARE recognition motif or in the neighborhood of the scissile peptide bond.

To obtain SSS/A, H6tHAtev1S(146-206)L already comprising the minimum essential peptide for BoNT/A cleavage had to be made uncleavable by BoNT/C and E. Truncating the 146-206 peptide to remove the BoNT/E cleavage site at R180-I181 is not possible since BoNT/A would lose its SNARE motif. It was previously shown that the mutations D179A and D179V at the BoNT/E P2 site drastically reduces catalysis by BoNT/E [96], but the consequences on BoNT/A cleavage was not tested. Furthermore, D179 is considered as forming an important salt bridge with K224 of BoNT/E [97]. Consequently, we tried to destroy this salt bridge by charge reversal and tested the mutation D179K to prevent BoNT/E cleavage. While the cleavage rate of H6tHAtev1S(146-206)L D179K by LC/A was marginally reduced (3-fold) and unaltered for LC/C, the ECso of LC/E increased -30,000-fold making D179K a suitable mutation to prevent BoNT/E cleavage. For BoNT/C, it has been shown that its P1' residue A199 which also serves as P2' for BoNT/A renders SNAP-25 uncleavable by BoNT/C if mutated to arginine, respectively [98]. However, the effect of mutation A199R on BoNT/A and E cleavage has not investigated. On the other hand, a synthetic 15-mer peptide comprising the mutation A199L was not cleavable by BoNT/A [50] highlighting the critical importance of a small, aliphatic residue at position 199 for BoNT/A cleavage. We demonstrate that SNAP-25 A199R is equally well cleaved as SNAP-25 wild-type by BoNT/E and only 5-fold worse by BoNT/A. Subsequently, we inserted the mutation A199R in combination with D179K into H6tHAtev1S(146-206)L to abolish the cleavage by BoNT/C and E. The resulting substrate H6tHAtev1S(146-206)LS D179KA199R (Figure 3) turned out to be insensitive to BoNT/C and E while still being cleavable by BoNT/A (Figure 4). Compared to the generic substrate (H6tHAtev1S(146-206)LS) the ECso of BoNT/A for SSS/A (H6tHAtev1S(146-206)LS D179KA199R, Figure 4) was increased to 0.4 nM while the ECso of BoNT/C and E for SSS/A was increased to 200 nM and 1000 nM, respectively (Table 3). Thus, only 500-fold and 2500-fold higher concentrations of BoNT/C and E than BoNT/A would yield similar cleavage of SSS/A. The VAMP-2-cleaving serotypes do not exhibit any detectable cleavage of SSS/A at concentrations up to 1 µM. In conclusion, the SSS/A is able to specifically detect BoNT/A in concentrations from 0.01 nM to 10 nM.

Analogously to SSS/A the SSS/C was designed. Cleavage by BoNT/E is prevented by mutation D179K. For BoNT/A, Vaidyanathan et al. demonstrated that mutant SNAP-25 R198K retained full susceptibility to BoNT/C while cleavage by BoNT/A was reduced by factor 500 [51]. Previously, the R198K mutation caused resistance of a synthetic 15-mer peptide towards BoNT/A. Furthermore, cleavage of the synthetic 15-mer by BoNT/A is strongly and slightly reduced due to mutation N196Q and Q197N, respectively [50]. Combining R198K and Q197A yielded a double mutant with 38,000-fold reduced EC₅₀ of BoNT/A [99]. A recent study showed that the single mutation N196Q improved the cleavage of a synthetic 15-mer peptide by BoNT/C slightly, the mutation Q197N improved the cleavage by BoNT/C 5-fold and the mutation R198K 2.6-fold [100]. However, the corresponding triple mutant N196QQ197NR198K optimized for BoNT/C cleavage was not analyzed for cleavage by BoNT/A. Based on this data we generated the substrate H6tHAtev1S(146-206)LS D179KN196QQ197NR198K to serve as SSS/C (Figure 3) which should not be cleaved by BoNT/A and BoNT/E. Interestingly, SSS/C was cleaved better than the generic peptide H6tHAtev1S(146-206)LS (Figure 4). Its EC₅₀ value decreased 16-fold (0.6 nM, Table 3). BoNT/A does not cleave the SSS/C at concentrations of up to 1 µM. Cleavage of SSS/C by BoNT/E occurred with an EC₅₀ of ~1 µM, a ~1,700-fold higher concentration than by BoNT/C. The VAMP-2-cleaving BoNTs do not cleave the SSS/C at concentrations up to 1 µM. In conclusion, the SSS/C is able to specifically detect BoNT/C in concentrations from 0.03 nM to 3 nM (Figure 4).

BoNT/E requires the SNARE motif S4 spanning from aa 145-155 [53] for optimal cleavage of SNAP-25 [92]. The scissile bond for BoNT/E resides 17 and 18 residues upstream of those of BoNT/A and C, respectively. A C-terminal truncation clipping off the cleavage sites of BoNT/A and C should prevent cleavage by them. Accordingly, the SSS/E was generated by truncating SNAP-25 at the N- and C-terminus, resulting in the SNAP-25 based peptide 140-197 and the SSS/E construct H6tHAtev1S(140-197)L (Figure 3). SSS/E was cleaved by BoNT/E with an EC₅₀ of 0.2 nM which is identical to that of the generic substrate (Figure 4, Table 3). Cleavage of SSS/E by BoNT/A and BoNT/C was not detectable at concentrations up to 1 µM. Likewise, the cleavage of SSS/E by the VAMP-2-cleaving serotypes is absent up to concentrations of 1 µM (Figure 4). In conclusion, the SSS/E is able to specifically detect BoNT/E in concentrations from 0.003 nM to 1 µM.

### Example 5: Serotype specific substrates for BoNT/B, D, F, F5/H, X and TeNT

Additionally to the generic substrate for the detection of the VAMP-2-cleaving BoNT (BoNT/B, D, F, F5/H, X) and TeNT, serotype-specific-substrates (SSS/X) susceptible only for a single serotype should be designed. Optimal proteolytic activity by BoNT/B was only observed with VAMP-2 based peptide substrates containing greater than 12 amino acid residues N-terminally and C-terminally of the cleavage site Q76-F77 [93]. The 35-residue VAMP-2 peptide 60-94 is cleaved by BoNT/B at the same rate as the larger VAMP-2 peptide 33-94 representing its helical domain [101]. The N-terminal truncation is intended to prevent the hydrolysis by BoNT/D, F and F5/H, which cleave VAMP-2 upstream of residue 60 and by TeNT, proteolysing the identical peptide bond as BoNT/B, but exhibiting the most demanding specificity by requiring the entire helical domain of VAMP-2 from aa 33-94 for optimal cleavage rates [48]. In view of that, SSS/B was obtained by inserting aa 60-94 of VAMP-2 into the modified construct yielding H6tHAtev1V(60-94)L (Figure 3). The BoNT/B ECso for SSS/B was determined as 0.6 nM (Table 3) and thus 10-fold higher as for the generic substrate H6tHAtev1V(2-94)LS comprising the full luminal domain of VAMP-2. The SSS/B was not cleavable by BoNT/A, C, D, E, F5/H and TeNT at concentrations up to 1 µM while BoNT/F caused minor hydrolysis above 0.3 µM. In conclusion, the SSS/B is able to specifically detect BoNT/B in concentrations from 0.03 nM to 0.3 µM.

Contrary to SSS/B, the VAMP-2 peptide for BoNT/D as well as for BoNT/F were truncated at the C-Terminus to prevent cleavage by BoNT/B and TeNT, resulting in H6tHAtev1V(2-76)LS(Figure 3). The ECso for LC/D, BoNT/F, LC/F5, BoNT/H and BoNT/X were determined as 6 nM, 3 nM, 30 nM, 21 nM and 1 nM, respectively, which is similar to the generic substrate H6tHAtev1V(2-94)LS. This result is congruent with data showing that the VAMP-2 peptide aa 35-75 is a suitable substrate for BoNT/D and F [94]. To obtain BoNT/D or BoNT/F specific substrates, additional mutations had to be inserted into H6tHAtev1V(2-76)LS to prevent cleavage by BoNT/F or BoNT/D, respectively. In the case of SSS/D, residue K59 was mutated to arginine due to data of Sikorra et al. who observed no cleavage of the VAMP-2 K59R mutant by BoNT/F, but 85 % cleavage by BoNT/D [102], and of Chen et al. who described 100-fold decreased cleavage of VAMP-2 K59R by BoNT/F [103]. Cleavage of substrate H6tHAtev1V(2-76)LS K59R as SSS/D was analyzed for BoNT/A-F in JDZ-buffer (Figure 4). The cleavage of SSS/D by BoNT/D was only marginal decreased by factor 3 compared to its generic substrate. The ECso values for cleavage by BoNT/D and F were determined as 0.6 nM and 6 nM (Table 3), a 10-fold difference between BoNT/D and F. BoNT/B as well as BoNT/A, C and E do not cleave the SSS/D at concentrations up to 1 µM. In conclusion, the SSS/D is able to specifically detect BoNT/D in concentrations from 0.01 nM to 1 nM.

VAMP-1 of human beings, chimpanzees, bonobos and certain rat species like Sprague-dawley is not cleavable by BoNT/D because of the mutation M48I [36,104,105]. Furthermore, Yamasaki et al. demonstrated that rat VAMP-2 M46I mutant was ~3,500-fold less susceptible to cleavage by BoNT/D, but only 10-fold less sensitive to BoNT/F cleavage [36]. Accordingly, the homologous mutation M46I was inserted in the SSS for BoNT/F. The substrate H6tHAtev1V(2-76)LS M46I to serve as SSS/F was generated and analyzed for BoNT cleavage (Figure 3). BoNT/F cleaves SSS/F with an EC₅₀ of 0.2 nM, which is 6-fold worse than the cleavage of the generic substrate H6tHAtev1V(2-94)LS (Table 3), but resembles the data obtained for rat VAMP-2 M46I. Likewise, BoNT/D displays an EC₅₀ of >1 µM, more than 5000-fold increased vs. the generic substrate H6tHAtev1V(2-94)LS (Figure 4) thereby confirming results of Yamasaki et al. [36]. Up to 1 µM of the other BoNT serotypes did not yield detectable cleavage of SSS/F. In conclusion, the SSS/F is able to specifically detect BoNT/F in concentrations from 0.03 nM to 0.3 µM.

Insertion of mutation A67R at the cleavage site of BoNT/X renders H6tHAtev1V(2-94)LS A67R insensitive to BoNT/X (Table 4). Also mutation A67K, A67E, A67D, A67Q, A67 N, A67F and A67W render H6tHAtev1V(2-94)LS less susceptible to cleavage by BoNT/X. Similarly, other H6tHAtev1V(2-94)LS-derived peptides are rendered insensitive towards BoNT/X hydrolysis by mutation A67R. Also insertion of mutation R66A at the cleavage site of BoNT/X renders H6tHAtev1V(2-94)LS 100x less susceptible to cleavage by BoNT/X. Similarly mutation R66I, R66L, R66V, R66T render H6tHAtev1V(2-94)LS less susceptible to cleavage by BoNT/X.

**Table 3: EC₅₀ of BoNT/A-F for the six serotype specific substrates (SSS/X) graphically determined from diagrams in Figure 4 (n.c. = not cleavable at concentrations up to 1 µM BoNT).**

| **EC₅₀ [nM]** | | **SSS/** | | | | | | **GS/X** | **n** |
|---|---|---|---|---|---|---|---|---|---|
| | | **A** | **B** | **C** | **D** | **E** | **F** | | |
| | **A1** | **0.4** | n.c. | n.c. | n.c. | n.c. | n.c. | **0.03** | 3-7 |
| | **B1** | n.c. | **0.6** | n.c. | n.c. | n.c. | n.c. | **0.07** | 3-7 |
| **BoNT/** | **C** | 200 | n.c. | **0.6** | n.c. | n.c. | n.c. | **10.00** | 3-7 |
| | **D** | n.c. | n.c. | n.c. | **0.6** | n.c. | >1000 | **0.20** | 3-8 |
| | **E1** | 1000 | n.c. | 1000 | n.c. | **0.2** | n.c. | **0.20** | 3-8 |
| | **F1** | n.c. | n.c. | n.c. | 6 | n.c. | **0.2** | **0.03** | 3-6 |

**Table 4: Endopeptidase assay of different VAMP-based substrates by BoNT/H, BoNT/F1, LC/F5 and BoNT/X in PBS pH 7.4 supplemented with 20 µM ZnCl₂,0.5 % Tween-20 and 5 mM DTT, for 1 h at 37°C (mean ± SD, n = 1-3; n.d., not determined; n.c. = not cleavable at concentrations up to 1 µM BoNT).**

| **Substrate** | **EC₅₀ [nM]** | | | |
|---|---|---|---|---|
| | BoNT/H | BoNT/F1 | LC/F5 | BoNT/X |
| **H6tHAtev1V(2-94)LS** | 20 ± 0 | 2.5 ± 0.7 | 30 ± 0 | 0.2 ± 0.14 |
| **H6tHAtev1V(2-76)LS** | 21 ± 0 | 3.0 ± 0 | 30 ± 0 | 1.0 ± 0 |
| **H6tHAtev1V(60-94)L** | n.c. | n.c. | n.c. | n.d. |
| **H6tHAtev1V(2-94)LS A67R** | 10 ± 0 | 3.0 ± 0 | n.d. | n.c. |

### Example 6: Analysis of various reporter proteins fused to SSS/X

The generic and serotype-specific substrates were C-terminally fused to the firefly (Photinus pyralis) luciferase (luc, L; 60 kDa) acting as reporter molecule. Upon BoNT-specific cleavage of the peptide the released luc is transferred and bioluminescence is measured upon addition of luciferin, ATP and oxygen and Mg²⁺ [87]. We also fused three other reporter molecules to the generic substrates: i) NanoLuc^{®} Luciferase (NL) from the deep sea shrimp Oplophorus gracilirostris [106], ii) superfolder green fluorescent protein (sfGFP)[77] and iii) mCherry [107].

The NanoLuc is a small (19.1 kDa), ATP-independent luciferase that utilizes a coelenterazine substrate (furimazine) to produce bioluminescence [108]. It is much smaller while brighter than firefly luciferase and also exhibits greater physical stability.

In contrast, sfGFP does not catalyze a biochemical reaction, it just acts as fluorophore with an excitation wavelength of 485 nm and an emission wavelength of 510 nm. In comparison to GFP, sfGFP contains the 'cycle-3' mutations F99S, M153T, V163A, the 'enhanced GFP' mutations F64L and S65T as well as the six novel mutations S30R, Y39N, N105T, Y145F, I171V and A206V. sfGFP shows greater resistance to chemical denaturants and improved folding kinetics [77].

mCherry belongs to a series of mFruit fluorophores [107], mutants derived from the tetrameric Discosoma sp. red fluorescent protein, DsRed [109]. The excitation and emission maxima of the mCherry protein are 587 nm and 610 nm, respectively, circumventing background fluorescence around 500 nm often observed in complex matrix samples. mCherry is monomeric, displays complete maturation yielding higher brightness (extinction coefficient x quantum yield), is more tolerant of N-terminal fusion proteins and much more photostable than DsRed [78].

The reporter molecules were fused to the generic substrates H6tHA1S(146-206) and H6tHA1V(2-94), respectively. Susceptibility of cleavage of H6tHA1S(146-206)LS, H6tHA1S(146-206)-sfGFP-S, H6tHA1S(146-206)-NL-S and H6tHA1S(146-206)-mCherry-S by BoNT/A, C and E was analyzed. Analogously, H6tHA1V(2-94)LS, H6tHA1V(2-94)-sfGFP-S and H6tHA1V(2-94)-mCherry-S were checked for cleavage by BoNT/B, D and F (Figure 5). BoNT/A cleavage of H6tHA1S(146-206)-NL-S is most efficient with an EC₅₀ of 0.01 nM followed by H6tHA1S(146-206)LS, H6tHA1S(146-206)-sfGFP-S and H6tHA1S(146-206)-mCherry-S, the latter displaying factor 3 to 10 higher EC₅₀ values than luc. With the exception of H6tHA1S(146-206)-mCherry-S, BoNT/E and C cleave all substrates similarly efficient. The cleavage of H6tHA1V(2-94)LS, H6tHA1V(2-94)-sfGFP-S and H6tHA1V(2-94)-mCherry-S by BoNT/B and F, respectively, deviates only by factor 2-4. In contrast, the fluorophores sfGFP and mCherry increase the EC₅₀ of BoNT/D 10-fold compared to the H6tHA1V(2-94)LS (Figure 5; Table 5).

All in all, mCherry seems to slightly impair the cleavage by all six BoNTs, especially in the case of BoNT/A, C and E. In contrast, the difference in EC₅₀ between the reporter molecules luc, NanoLuc or sfGFP is negligible compared to technical advantages provided by reporter molecules other than luc.

**Table 5: Determination of the EC50 of the generic substrate peptides with different reporter molecules for BoNT/A-F.**

| **EC50 [nM]** | | **gS/X** + **detector** Y | | | |
|---|---|---|---|---|---|
| | | **luc** | **NL** | **sfGFP** | **mCherry** |
| BoNT | **A** | 0.03 | 0.01 | 0.03 | 0.10 |
| | **B** | 0.07 | - | 0.02 | 0.03 |
| | **C** | 10.00 | 10.00 | 20.00 | 40.00 |
| | **D** | 0.20 | - | 1.00 | 2.00 |
| | **E** | 0.20 | 0.20 | 0.10 | 0.60 |
| | **F** | 0.03 | - | 0.06 | 0.06 |

**Table 6: Peptides and polypeptides of the present teaching**

| SEQ ID No | Name | Serotype-specific (SSS/X) | AA-Sequence (modified AA in bold) | Modification |
|---|---|---|---|---|
| 1 | H6tHAtev1S(146-206)LS D179KA199R | A | MDENLEQVSGIIGNLRHMALDMGNEIDTQNRQIKRIMEKADSNKTRIDEANQRRTKMLGSG | D179K A199R |
| 2 | H6tHAtev 1V(60-94)L | B, X | LSELDDRADALQAGASQFETSAAKLKRKYWWKNLK | - |
| 3 | H6tHAtev 1V(60-94)L T79S | B, X | LSELDDRADALQAGASQFESSAAKLKRKYWWKNLK | T79S |
| 4 | H6tHA1S(146-206)LS D179KN196QQ197NR 198K | C | MDENLEQVSGIIGNLRHMALDMGNEIDTQNRQIKRIMEKADSNKTRIDEA**QNK**ATKMLGSG | D179K N196Q Q197N R198K |
| 5 | H6tHAtev1V(2-76)LS E55QK59R | D, X | | E55Q K59R |
| 6 | H6tHAtev1V(2-76)LS L54AE55QK59R | D, X | | L54A E55Q K59R |
| 7 | H6tHAtev1S(140-197)L | E | DARENEMDENLEQVSGIIGNLRHMALDMGNEIDTQNRQIDRIMEKADSNKTRIDEANQ | - |
| 8 | H6tHAtev1S(2-197)L | E | | - |
| 9 | H6tHAtev1V(2-76)LS M46IE55Q | F, X | | M46I E55Q |
| 10 | H6tHAtev1V(2-76)LS M46IL54AK59R | F5/H, X | | M46I L54A K59R |
| 11 | H6tHAtev1V(40-94)LS M46IE55QK59RA72D | X, TeNT | DEVVDI**I**RVNVDKVL**Q**RDQ**R**LSELDDRADALQ**D**GASQFETSAAKLKRKYWWKNLK | M46I E55Q K59R A72D |
| 12 | H6tHAtev1V(40-94)LS M46IL54AE55QK59RA 72D | X, TeNT | DEVVDI**I**RVNVDKV**AQ**RDQ**R**LSELDDRADALQ**D**GASQFETSAAKLKRKYWWKNLK | M46I L54A E55Q K59R A72D |
| 13 | H6tHA1S(146-206)LS R180WI181EE183I | A,C | MDENLEQVSGIIGNLRHMALDMGNEIDTQNRQID**WE**MIKADSNKTRIDEANQRATKMLGSG | R180WI181EE1831 |
| 14 | H6tHA1S(146-206)LS D179K | A,C | MDENLEQVSGIIGNLRHMALDMGNEIDTQNRQIKRIMEKADSNKTRIDEANQRATKMLGSG | D179K |
| 15 | H6tHAtev1S(146-206)LS A199R | A, E | MDENLEQVSGIIGNLRHMALDMGNEIDTQNRQIDRIMEKADSNKTRIDEANQR**R**TKMLGSG | A199R |
| 16 | H6tHAtev1S(146-206)LS N196QQ197NR198K | C,E | MDENLEQVSGIIGNLRHMALDMGNEIDTQNRQIDRIMEKADSNKTRIDEA**QNK**ATKMLGSG | N196Q Q197N R198K |
| 17 | H6tHAtev1V(40-94)LS M46IL54AE55QK59R | B, X, TeNT | DEVVDI**I**RVNVDKV**AQ**RDQ**R**LSELDDRADALQAGASQFETSAAKLKRKYWWKNLK | M46I L54A E55Q K59R |
| 18 | H6tHAtev1V(2-76)LS K59R | D, F5/H, X | | K59R |
| 19 | H6tHAtev1V(2-76)LS M46I | F, F5/H, X | | M46I |
| 20 | H6tHAtev1V(2-76)LS E55Q | D, F, X | | E55Q |
| 21 | H6tHAtev1S(146-206)LS | A, C, E | MDENLEQVSGIIGNLRHMALDMGNEIDTQNRQIDRIMEKADSNKTRIDEANQRATKMLGSG | - |
| 22 | H6tHAtev1S(2-206)L(S) | A, C, E | | - |
| 23 | H6tHA1V(2-76)LS | D, F, F5/H, X | | - |
| 24 | H6tHAtev1V(2-94)LS A72D | D, F, F5/H, X, TeNT | | A72D |
| 25 | H6tHAtev1V(2-94)LS M46I | B, F, F5/H, X, TeNT | | M46I |
| 26 | H6tHAtev1V(2-94)LS K59R | B, D, F5/H, X, TeNT | | K59R |
| 27 | H6tHAtev1V(2-94)LS L54AK59R | B, D, F5/H, X, TeNT | | L54A K59R |
| 28 | H6tHAtev1V(2-94)LS E55Q | B, D, F, X, TeNT | | E55Q |
| 29 | H6tHAtev1V(2-94)LS A69N | B, D, F, F5/H, X | | A69N |
| 30 | H6tHAtev1V(2-94)LS | B, D, F, F5/H, X, TeNT | | - |
| 31 | H6tHAtev1V(2-94)LS T79S | B, D, F, F5/H, X, TeNT | | T79S |
| 43 | H6tHAtev 1V(60-94)L A67R | B | LSELDDR**R**DALQAGASQFETSAAKLKRKYWWKNLK | A67R |
| 44 | H6tHAtev 1V(60-94)L A67RT79S | B | LSELDDR**R**DALQAGASQFE**S**SAAKLKRKYWWKNLK | A67R T79S |
| 45 | H6tHAtev1V(2-76)LS E55QK59RA67R | D | | E55Q K59R A67R |
| 46 | H6tHAtev1V(2-76)LS L54AE55QK59RA67R | D | | L54A E55Q K59R A67R |
| 47 | H6tHAtev1V(2-76)LS L54EE55LK59RA67R | D | | L54E E55L K59R A67R |
| 48 | H6tHAtev1V(2-76)LS Q34AK59RA67R | D | | Q34A K59R A67R |
| 49 | H6tHAtev1V(2-76)LS V43AK59RA67R | D | | V43A K59R A67R |
| 50 | H6tHAtev1V(2-76)LS M46IE55QA67R | F | | M46I E55Q A67R |
| 51 | H6tHAtev1V(2-76)LS M46IL54EE55LA67R | F | | M46I L54E E55L A67R |
| 52 | H6tHAtev1V(2-76)LS Q34AM46IA67R | F | | Q34A M46I A67R |
| 53 | H6tHAtev1V(2-76)LS V43AM46IA67R | F | | V43A M46I A67R |
| 54 | H6tHAtev1V(2-76)LS M46IL54AK59RA67R | F5/H | | M46I L54A K59R A67R |
| 55 | H6tHAtev1V(2-76)LS M46IK59RA67R | F5/H | | M46I K59R A67R |
| 56 | H6tHAtev1V(40-94)LS M46I E55QK59RA67RA 72D | TeNT | DEVVDIIRVNVDKVL**Q**RDQ**R**LSELDDR**R**DALQ**D**GASQFETSAAKLKRKYWWKNLK | M46I E55Q K59R A67R A72D |
| 57 | H6tHAtev1V(40-94)LS M46IL54AE55QK59RA 67RA72D | TeNT | DEVVDIIRVNVDKV**AQ**RDQ**R**LSELDDRRDALQDGASQFETSAAKLKRKYWWKNLK | M46I L54A E55Q K59R A67R A72D |
| 58 | H6tHAtev1V(40-94)LS M46IL54AE55QK59RQ 76V | X | DEVVDIIRVNVDKV**AQ**RDQ**R**LSELDDRADALQAGASVFETSAAKLKRKYWWKNLK | M46I L54A E55Q K59R Q76V |
| 59 | H6tHAtev1V(2-76)LS L54EE55LK59R | D, X | | L54E E55L K59R |
| 60 | H6tHAtev1V(2-76)LS Q34AK59R | D, X | | Q34A K59R |
| 61 | H6tHAtev1V(2-76)LS V43AK59R | D, X | | V43A K59R |
| 62 | H6tHAtev1V(2-76)LS M46IL54EE55L | F, X | | M46I L54E E55L |
| 63 | H6tHAtev1V(2-76)LS Q34AM46I | F, X | | Q34A M46I |
| 64 | H6tHAtev1V(2-76)LS V43AM46I | F, X | | V43A M46I |
| 65 | H6tHAtev1V(2-76)LS K59RA67R | D, F5/H | | K59R A67R |
| 66 | H6tHAtev1V(2-76)LS L54AK59R | D, F5/H, X | | L54A K59R |
| 67 | H6tHAtev1V(2-76)LS E55QA67R | D, F | | E55Q A67R |
| 68 | H6tHAtev1V(2-76)LS L54AE55Q | D, F, X | | L54A E55Q |
| 69 | H6tHAtev1V(2-94)LS Q76V | D, F, F5/H, X | | Q76V |
| 71 | H6tHAtev1V(2-94)LS M46IK59R | B, F5/H, X, TeNT | | M46I K59R |
| 72 | H6tHAtev1V(2-94)LS L54AE55Q | B, D, F, X, TeNT | | L54A E55Q |
| 73 | H6tHAtev1V(2-94)LS A67R | B, D, F, F5/H, TeNT | | A67R |
| 74 | H6tHAtev1V(2-94)LS M46IL54AE55QK59R | B, X, TeNT | | M46I L54A E55Q K59R |
| | | Genbank ID | | |
| 32 | BoNT/A1 | AAA23262 | | |
| | | | | |
| 33 | BoNT/B1 | AB232927 | | |
| 34 | BoNT/C | CAA37780 | | |
| | | | | |
| 35 | BoNT/D | CAA38175 | | |
| 36 | BoNT/E1 | CAA43999 | | |
| | | | | |
| 37 | BoNT/F1 | CAA57358 | | |
| 38 | BoNT/F5 | ADA79559 | | |
| 39 | BoNT/G | Q60393 | | |
| | | | | |
| 40 | BoNT/H | KGO15617 | | |
| 41 | TeNT | CAA28033 | | |
| | | | | |
| 42 | BoNT/X | WP_045538 952 | | |
| 75 | SNAP-25 | AAH18249. 1 | | |
| 76 | VAMP-2 | NP_055047 .2 | | |

### List of references:

1. Lamanna, C. Toxicity of bacterial exotoxins by the oral route. Science 1960, 131, 1100-1101.
2. Rummel, A. The long journey of botulinum neurotoxins into the synapse. Toxicon 2015, 107, 9-24.
3. Gill, D.M. Bacterial toxins: a table of lethal amounts. Microbiol Rev 1982, 46, 86-94.
4. Ohishi, I.; Sakaguchi, G. Oral toxicities of Clostridium botulinum type C and D toxins of different molecular sizes. Infect Immun 1980, 28, 303-309.
5. Sugiyama, H. Clostridium botulinum neurotoxin. Microbiol Rev 1980, 44, 419-448.
6. Sharma, S.K.; Ferreira, J.L.; Eblen, B.S.; Whiting, R.C. Detection of type A, B, E, and F Clostridium botulinum neurotoxins in foods by using an amplified enzyme-linked immunosorbent assay with digoxigenin-labeled antibodies. Appl Environ Microbiol 2006, 72, 1231-1238.
7. Bigalke, H.; Rummel, A. Botulinum Neurotoxins: Qualitative and Quantitative Analysis Using the Mouse Phrenic Nerve Hemidiaphragm Assay (MPN). Toxins 2015, 7, 4895-4905.
8. Pellett, S. Progress in cell based assays for botulinum neurotoxin detection. Curr Top Microbiol Immunol 2013, 364, 257-285.
9. McNutt, P.; Celver, J.; Hamilton, T.; Mesngon, M. Embryonic stem cell-derived neurons are a novel, highly sensitive tissue culture platform for botulinum research. Biochem Biophys Res Commun 2011, 405, 85-90.
10. Pellett, S.; Du, Z.W.; Pier, C.L.; Tepp, W.H.; Zhang, S.C.; Johnson, E.A. Sensitive and quantitative detection of botulinum neurotoxin in neurons derived from mouse embryonic stem cells. Biochem Biophys Res Commun 2011, 404, 388-392.
11. Whitemarsh, R.C.; Strathman, M.J.; Chase, L.G.; Stankewicz, C.; Tepp, W.H.; Johnson, E.A.; Pellett, S. Novel application of human neurons derived from induced pluripotent stem cells for highly sensitive botulinum neurotoxin detection. Toxicol Sci 2012, 126, 426-435.
12. Höltje, M.; Schulze, S.; Strotmeier, J.; Mahrhold, S.; Richter, K.; Binz, T.; Bigalke, H.; Ahnert-Hilger, G.; Rummel, A. Exchanging the minimal cell binding fragments of tetanus neurotoxin in botulinum neurotoxin A and B impacts their toxicity at the neuromuscular junction and central neurons. Toxicon 2013, 75, 108-121.
13. Nuss, J.E.; Ruthel, G.; Tressler, L.E.; Wanner, L.M.; Torres-Melendez, E.; Hale, M.L.; Bavari, S. Development of cell-based assays to measure botulinum neurotoxin serotype A activity using cleavage-sensitive antibodies. J Biomol Screen 2010, 15, 42-51.
14. Mander, G.; Bruenn, C.; Jatzke, C.; Eisele, K.-H.; Taylor, H.V.; Pellett, S.; Johnson, E.A.; Fink, K. Potency assay for botulinum neurotoxin type A based on neuronal cells as a replacement for the mouse bioassay. Toxicon 2015, 93, Supplement, S41-S42.
15. Fernandez-Salas, E.; Wang, J.; Molina, Y.; Nelson, J.B.; Jacky, B.P.; Aoki, K.R. Botulinum neurotoxin serotype A specific cell-based potency assay to replace the mouse bioassay. PLoS One 2012, 7, e49516.
16. Rheaume, C.; Cai, B.B.; Wang, J.; Fernandez-Salas, E.; Aoki, K.R.; Francis, J.; Broide, R.S. A Highly Specific Monoclonal Antibody for Botulinum Neurotoxin Type A-Cleaved SNAP25. Toxins 2015, 7, 2354-2370.
17. Kiris, E.; Nuss, J.E.; Burnett, J.C.; Kota, K.P.; Koh, D.C.; Wanner, L.M.; Torres-Melendez, E.; Gussio, R.; Tessarollo, L.; Bavari, S. Embryonic stem cell-derived motoneurons provide a highly sensitive cell culture model for botulinum neurotoxin studies, with implications for high-throughput drug discovery. Stem Cell Res 2011, 6, 195-205.
18. Dong, M.; Tepp, W.H.; Johnson, E.A.; Chapman, E.R. Using fluorescent sensors to detect botulinum neurotoxin activity in vitro and in living cells. Proc Natl Acad Sci U S A 2004, 101, 14701-14706.
19. Keller, J.E.; Cai, F.; Neale, E.A. Uptake of botulinum neurotoxin into cultured neurons. Biochemistry 2004, 43, 526-532.
20. Bigalke, H.; Dimpfel, W.; Habermann, E. Suppression of 3H-acetylcholine release from primary nerve cell cultures by tetanus and botulinum-A toxin. Naunyn Schmiedebergs Arch Pharmacol 1978, 303, 133-138.
21. Tegenge, M.A.; Bohnel, H.; Gessler, F.; Bicker, G. Neurotransmitter Vesicle Release from Human Model Neurons (NT2) is Sensitive to Botulinum Toxin A. Cell Mol Neurobiol 2012**.**
22. Rasetti-Escargueil, C.; Machado, C.B.; Preneta-Blanc, R.; Fleck, R.A.; Sesardic, D. Enhanced sensitivity to Botulinum type A neurotoxin of human neuroblastoma SH-SY5Y cells after differentiation into mature neuronal cells. The Botulinum Journal 2011, 2, 30-48.
23. Pathe-Neuschäfer-Rube, A.; Neuschäfer-Rube, F.; Genz, L.; Püschel, G.P. Botulinum Neurotoxin Dose-Dependently Inhibits Release of Neurosecretory Vesicle-Targeted Luciferase from Neuronal Cells. Altex 2015, 32, 297-306.
24. Akaike, N.; Ito, Y.; Shin, M.C.; Nonaka, K.; Torii, Y.; Harakawa, T.; Ginnaga, A.; Kozaki, S.; Kaji, R. Effects of A2 type botulinum toxin on spontaneous miniature and evoked transmitter release from the rat spinal excitatory and inhibitory synapses. Toxicon 2010, 56, 1315-1326.
25. Scarlatos, A.; Cadotte, A.J.; DeMarse, T.B.; Welt, B.A. Cortical networks grown on microelectrode arrays as a biosensor for botulinum toxin. J Food Sci 2008, 73, E129-136.
26. Pellett, S.; Tepp, W.H.; Clancy, C.M.; Borodic, G.E.; Johnson, E.A. A neuronal cell-based botulinum neurotoxin assay for highly sensitive and specific detection of neutralizing serum antibodies. FEBS letters 2007**.**
27. Dorner, M.B.; Schulz, K.M.; Kull, S.; Dorner, B.G. Complexity of botulinum neurotoxins: challenges for detection technology. Curr Top Microbiol Immunol 2013, 364, 219-255.
28. Worbs, S.; Fiebig, U.; Zeleny, R.; Schimmel, H.; Rummel, A.; Luginbühl, W.; Dorner, B. Qualitative and Quantitative Detection of Botulinum Neurotoxins from Complex Matrices: Results of the First International Proficiency Test. Toxins 2015, 7, 4857.
29. Simon, S.; Fiebig, U.; Liu, Y.; Tierney, R.; Dano, J.; Worbs, S.; Endermann, T.; Nevers, M.C.; Volland, H.; Sesardic, D., et al. Recommended Immunological Strategies to Screen for Botulinum Neurotoxin-Containing Samples. Toxins 2015, 7, 5011-5034.
30. Lacy, D.B.; Tepp, W.; Cohen, A.C.; DasGupta, B.R.; Stevens, R.C. Crystal structure of botulinum neurotoxin type A and implications for toxicity. Nat Struct Biol 1998, 5, 898-902.
31. Schiavo, G.; Rossetto, O.; Santucci, A.; DasGupta, B.R.; Montecucco, C. Botulinum neurotoxins are zinc proteins. The Journal of biological chemistry 1992, 267, 23479-23483.
32. Schiavo, G.; Benfenati, F.; Poulain, B.; Rossetto, O.; Polverino de Laureto, P.; DasGupta, B.R.; Montecucco, C. Tetanus and botulinum-B neurotoxins block neurotransmitter release by proteolytic cleavage of synaptobrevin. Nature 1992, 359, 832-835.
33. Schiavo, G.; Shone, C.C.; Rossetto, O.; Alexander, F.C.; Montecucco, C. Botulinum neurotoxin serotype F is a zinc endopeptidase specific for VAMP/synaptobrevin. The Journal of biological chemistry 1993, 268, 11516-11519.
34. Schiavo, G.; Rossetto, O.; Catsicas, S.; Polverino de Laureto, P.; DasGupta, B.R.; Benfenati, F.; Montecucco, C. Identification of the nerve terminal targets of botulinum neurotoxin serotypes A, D, and E. The Journal of biological chemistry 1993, 268, 23784-23787.
35. Schiavo, G.; Malizio, C.; Trimble, W.S.; Polverino de Laureto, P.; Milan, G.; Sugiyama, H.; Johnson, E.A.; Montecucco, C. Botulinum G neurotoxin cleaves VAMP/synaptobrevin at a single Ala-Ala peptide bond. The Journal of biological chemistry 1994, 269, 20213-20216.
36. Yamasaki, S.; Baumeister, A.; Binz, T.; Blasi, J.; Link, E.; Cornille, F.; Roques, B.; Fykse, E.M.; Südhof, T.C.; Jahn, R., et al. Cleavage of members of the synaptobrevin/VAMP family by types D and F botulinal neurotoxins and tetanus toxin. The Journal of biological chemistry 1994, 269, 12764-12772.
37. Yamasaki, S.; Binz, T.; Hayashi, T.; Szabo, E.; Yamasaki, N.; Eklund, M.; Jahn, R.; Niemann, H. Botulinum neurotoxin type G proteolyses the Ala81-Ala82 bond of rat synaptobrevin 2. Biochem Biophys Res Commun 1994, 200, 829-835.
38. Link, E.; Edelmann, L.; Chou, J.H.; Binz, T.; Yamasaki, S.; Eisel, U.; Baumert, M.; Sudhof, T.C.; Niemann, H.; Jahn, R. Tetanus toxin action: inhibition of neurotransmitter release linked to synaptobrevin proteolysis. Biochem Biophys Res Commun 1992, 189, 1017-1023.
39. Kalb, S.R.; Baudys, J.; Webb, R.P.; Wright, P.; Smith, T.J.; Smith, L.A.; Fernandez, R.; Raphael, B.H.; Maslanka, S.E.; Pirkle, J.L., et al. Discovery of a novel enzymatic cleavage site for botulinum neurotoxin F5. FEBS letters 2012, 586, 109-115.
40. Kalb, S.R.; Baudys, J.; Raphael, B.H.; Dykes, J.K.; Luquez, C.; Maslanka, S.E.; Barr, J.R. Functional Characterization of Botulinum Neurotoxin Serotype H as a Hybrid of Known Serotypes F and A (BoNT F/A). Anal Chem 2015, 87, 3911-3917.
41. Schiavo, G.; Santucci, A.; Dasgupta, B.R.; Mehta, P.P.; Jontes, J.; Benfenati, F.; Wilson, M.C.; Montecucco, C. Botulinum neurotoxins serotypes A and E cleave SNAP-25 at distinct COOH-terminal peptide bonds. FEBS letters 1993, 335, 99-103.
42. Blasi, J.; Chapman, E.R.; Yamasaki, S.; Binz, T.; Niemann, H.; Jahn, R. Botulinum neurotoxin C1 blocks neurotransmitter release by means of cleaving HPC-1/syntaxin. Embo J 1993, 12, 4821-4828.
43. Binz, T.; Blasi, J.; Yamasaki, S.; Baumeister, A.; Link, E.; Südhof, T.C.; Jahn, R.; Niemann, H. Proteolysis of SNAP-25 by types E and A botulinal neurotoxins. The Journal of biological chemistry 1994, 269, 1617-1620.
44. Foran, P.; Lawrence, G.W.; Shone, C.C.; Foster, K.A.; Dolly, J.O. Botulinum neurotoxin C1 cleaves both syntaxin and SNAP-25 in intact and permeabilized chromaffin cells: correlation with its blockade of catecholamine release. Biochemistry 1996, 35, 2630-2636.
45. Williamson, L.C.; Halpern, J.L.; Montecucco, C.; Brown, J.E.; Neale, E.A. Clostridial neurotoxins and substrate proteolysis in intact neurons: botulinum neurotoxin C acts on synaptosomal-associated protein of 25 kDa. The Journal of biological chemistry 1996, 271, 7694-7699.
46. Schiavo, G.; Shone, C.C.; Bennett, M.K.; Scheller, R.H.; Montecucco, C. Botulinum neurotoxin type C cleaves a single Lys-Ala bond within the carboxyl-terminal region of syntaxins. The Journal of biological chemistry 1995, 270, 10566-10570.
47. Cornille, F.; Martin, L.; Lenoir, C.; Cussac, D.; Roques, B.P.; Fournie-Zaluski, M.C. Cooperative exosite-dependent cleavage of synaptobrevin by tetanus toxin light chain. The Journal of biological chemistry 1997, 272, 3459-3464.
48. Foran, P.; Shone, C.C.; Dolly, J.O. Differences in the protease activities of tetanus and botulinum B toxins revealed by the cleavage of vesicle-associated membrane protein and various sized fragments. Biochemistry 1994, 33, 15365-15374.
49. Schmidt, J.J.; Bostian, K.A. Proteolysis of synthetic peptides by type A botulinum neurotoxin. J Protein Chem 1995, 14, 703-708.
50. Schmidt, J.J.; Bostian, K.A. Endoproteinase activity of type A botulinum neurotoxin: substrate requirements and activation by serum albumin. J Protein Chem 1997, 16, 19-26.
51. Vaidyanathan, V.V.; Yoshino, K.; Jahnz, M.; Dorries, C.; Bade, S.; Nauenburg, S.; Niemann, H.; Binz, T. Proteolysis of SNAP-25 isoforms by botulinum neurotoxin types A, C, and E: domains and amino acid residues controlling the formation of enzymesubstrate complexes and cleavage. J Neurochem 1999, 72, 327-337.
52. Sikorra, S.; Henke, T.; Swaminathan, S.; Galli, T.; Binz, T. Identification of the amino acid residues rendering TI-VAMP insensitive toward botulinum neurotoxin B. J Mol Biol 2006, 357, 574-582.
53. Rossetto, O.; Schiavo, G.; Montecucco, C.; Poulain, B.; Deloye, F.; Lozzi, L.; Shone, C.C. SNARE motif and neurotoxins. Nature 1994, 372, 415-416.
54. Breidenbach, M.A.; Brunger, A.T. Substrate recognition strategy for botulinum neurotoxin serotype A. Nature 2004, 432, 925-929.
55. Williams, D.; Steward, L.E.; Gilmore, M.A.; Okawa, Y.; Webber, J.A.; Aoki, K.R. GFP-SNAP-25 fluorescence release assay of BoNT proteolytic activity. Neurotox. Res 2006, 9, 239.
56. Hines, H.B.; Kim, A.D.; Stafford, R.G.; Badie, S.S.; Brueggeman, E.E.; Newman, D.J.; Schmidt, J.J. Use of a recombinant fluorescent substrate with cleavage sites for all botulinum neurotoxins in high-throughput screening of natural product extracts for inhibitors of serotypes A, B, and E. Appl Environ Microbiol 2008, 74, 653-659.
57. Shine, N.; Eaton, L.; Crawford, K. In A continuous fluorimetric assay for high-throughput screening for botulinum toxin type A inhibitors, NAUNYN-SCHMIEDEBERGS ARCHIVES OF PHARMACOLOGY, 2002; SPRINGER-VERLAG 175 FIFTH AVE, NEW YORK, NY 10010 USA: pp R40-R40.
58. Anne, C.; Cornille, F.; Lenoir, C.; Roques, B.P. High-throughput fluorogenic assay for determination of botulinum type B neurotoxin protease activity. Anal Biochem 2001, 291, 253-261.
59. Poras, H.; Ouimet, T.; Orng, S.V.; Fournie-Zaluski, M.C.; Popoff, M.R.; Roques, B.P. Detection and Quantification of Botulinum Neurotoxin Type a by a Novel Rapid in Vitro Fluorigenic Assay. Appl Environ Microbiol 2009**.**
60. Ross, J.A.; Gilmore, M.A.; Williams, D.; Aoki, K.R.; Steward, L.E.; Jameson, D.M. Characterization of Förster resonance energy transfer in a botulinum neurotoxin protease assay. Anal Biochem 2011, 413, 43-49.
61. Gilmore, M.A.; Williams, D.; Okawa, Y.; Holguin, B.; James, N.G.; Ross, J.A.; Roger Aoki, K.; Jameson, D.M.; Steward, L.E. Depolarization after resonance energy transfer (DARET): A sensitive fluorescence-based assay for botulinum neurotoxin protease activity. Analytical Biochemistry 2011, 413, 36-42.
62. Bagramyan, K.; Barash, J.R.; Arnon, S.S.; Kalkum, M. Attomolar detection of botulinum toxin type A in complex biological matrices. PLoS One 2008, 3, e2041.
63. Bagramyan, K.; Kaplan, B.E.; Cheng, L.W.; Strotmeier, J.; Rummel, A.; Kalkum, M. Substrates and controls for the quantitative detection of active botulinum neurotoxin in protease-containing samples. Anal Chem 2013, 85, 5569-5576.
64. Barr, J.R.; Moura, H.; Boyer, A.E.; Woolfitt, A.R.; Kalb, S.R.; Pavlopoulos, A.; McWilliams, L.G.; Schmidt, J.G.; Martinez, R.A.; Ashley, D.L. Botulinum neurotoxin detection and differentiation by mass spectrometry. Emerg Infect Dis 2005, 11, 1578-1583.
65. Kalb, S.R.; Boyer, A.E.; Barr, J.R. Mass Spectrometric Detection of Bacterial Protein Toxins and Their Enzymatic Activity. Toxins 2015, 7, 3497-3511.
66. Keller, J.E.; Nowakowski, J.L.; Filbert, M.G.; Adler, M. Rapid microplate assay for monitoring botulinum neurotoxin B catalytic activity. J Appl Toxicol 1999, 19 Suppl 1, S13-17.
67. Ekong, T.A.; Feavers, I.M.; Sesardic, D. Recombinant SNAP-25 is an effective substrate for Clostridium botulinum type A toxin endopeptidase activity in vitro. Microbiology 1997, 143 ( Pt 10), 3337-3347.
68. Hallis, B.; James, B.A.; Shone, C.C. Development of novel assays for botulinum type A and B neurotoxins based on their endopeptidase activities. J Clin Microbiol 1996, 34, 1934-1938.
69. Kegel, B.; Bonifas, U.; Silberbach, K.; Kramer, B.; Weisser, K. In vitro determination of specific toxicity in tetanus vaccines. Dev Biol (Basel) 2002, 111, 27-33.
70. Jones, R.G.; Liu, Y.; Sesardic, D. New highly specific botulinum type C1 endopeptidase immunoassays utilising SNAP25 or Syntaxin substrates. J Immunol Methods 2009, 343, 21-27.
71. Jones, R.G.; Ochiai, M.; Liu, Y.; Ekong, T.; Sesardic, D. Development of improved SNAP25 endopeptidase immuno-assays for botulinum type A and E toxins. J Immunol Methods 2008, 329, 92-101.
72. Marconi, S.; Ferracci, G.; Berthomieu, M.; Kozaki, S.; Miquelis, R.; Boucraut, J.; Seagar, M.; Leveque, C. A protein chip membrane-capture assay for botulinum neurotoxin activity. Toxicol Appl Pharmacol 2008, 233, 439-446.
73. Leveque, C.; Ferracci, G.; Maulet, Y.; Grand-Masson, C.; Blanchard, M.P.; Seagar, M.; El Far, O. A substrate sensor chip to assay the enzymatic activity of Botulinum neurotoxin A. Biosens Bioelectron 2013, 49, 276-281.
74. Ferracci, G.; Marconi, S.; Mazuet, C.; Jover, E.; Blanchard, M.P.; Seagar, M.; Popoff, M.; Leveque, C. A label-free biosensor assay for botulinum neurotoxin B in food and human serum. Anal Biochem 2011, 410, 281-288.
75. Leveque, C.; Ferracci, G.; Maulet, Y.; Mazuet, C.; Popoff, M.R.; Blanchard, M.P.; Seagar, M.; El Far, O. An optical biosensor assay for rapid dual detection of Botulinum neurotoxins A and E. Sci Rep 2015, 5, 17953.
76. Jones, R.G.; Liu, Y.; Halls, C.; Thorpe, S.J.; Longstaff, C.; Matejtschuk, P.; Sesardic, D. Release of proteolytic activity following reduction in therapeutic human serum albumin containing products: detection with a new neoepitope endopeptidase immunoassay. J Pharm Biomed Anal 2011, 54, 74-80.
77. Pedelacq, J.D.; Cabantous, S.; Tran, T.; Terwilliger, T.C.; Waldo, G.S. Engineering and characterization of a superfolder green fluorescent protein. Nat Biotechnol 2006, 24, 79-88.
78. Shaner, N.C.; Lin, M.Z.; McKeown, M.R.; Steinbach, P.A.; Hazelwood, K.L.; Davidson, M.W.; Tsien, R.Y. Improving the photostability of bright monomeric orange and red fluorescent proteins. Nat Methods 2008, 5, 545-551.
79. Shaner, N.C.; Patterson, G.H.; Davidson, M.W. Advances in fluorescent protein technology. J Cell Sci 2007, 120, 4247-4260.
80. Blum, L.J.; Plaza, J.M.; Coulet, P.R. Chemiluminescent Analyte Microdetection Based on the Luminol-H2 O 2 Reaction Using Peroxidase Immobilized on New Synthetic Membranes. Analytical Letters 1987, 20, 317-326.
81. Maeda, M.; Tsuji, A. [Chemiluminescent assay for biological substances using lucigenin]. Yakugaku zasshi : Journal of the Pharmaceutical Society of Japan 1997, 117, 864-874.
82. Quiel, A.; Jurgen, B.; Piechotta, G.; Le Foll, A.P.; Ziebandt, A.K.; Kohler, C.; Koster, D.; Engelmann, S.; Erck, C.; Hintsche, R., et al. Electrical protein array chips for the detection of staphylococcal virulence factors. Appl Microbiol Biotechnol 2010, 85, 1619-1627.
83. Quiel, A.; Jurgen, B.; Greinacher, A.; Lassen, S.; Worl, R.; Witt, S.; Schweder, T. Sensitive detection of idiotypic platelet-reactive alloantibodies by an electrical protein chip. Biosens Bioelectron 2012, 36, 207-211.
84. Terpe, K. Overview of tag protein fusions: from molecular and biochemical fundamentals to commercial systems. Appl Microbiol Biotechnol 2003, 60, 523-533.
85. Los, G.V.; Wood, K. The HaloTag: a novel technology for cell imaging and protein analysis. Methods Mol Biol 2007, 356, 195-208.
86. Weisemann, J.; Krez, N.; Fiebig, U.; Worbs, S.; Skiba, M.; Endermann, T.; Dorner, M.; Bergström, T.; Munoz, A.; Zegers, I., et al. Generation and Characterization of Six Recombinant Botulinum Neurotoxins as Reference Material to Serve in an International Proficiency Test. Toxins 2015, 7, 4861.
87. Stevens, G.B.; Silver, D.A.; Zgaga-Griesz, A.; Bessler, W.G.; Vashist, S.K.; Patel, P.; Achazi, K.; Strotmeier, J.; Worbs, S.; Dorner, M.B., et al. Bioluminescence assay for the highly sensitive detection of botulinum neurotoxin A activity. The Analyst 2013, 138, 6154-6162.
88. Binz, T.; Bade, S.; Rummel, A.; Kollewe, A.; Alves, J. Arg(362) and Tyr(365) of the botulinum neurotoxin type a light chain are involved in transition state stabilization. Biochemistry 2002, 41, 1717-1723.
89. Rummel, A.; Bade, S.; Alves, J.; Bigalke, H.; Binz, T. Two carbohydrate binding sites in the HCC-domain of tetanus neurotoxin are required for toxicity. J Mol Biol 2003, 326, 835-847.
90. Rummel, A.; Eichner, T.; Weil, T.; Karnath, T.; Gutcaits, A.; Mahrhold, S.; Sandhoff, K.; Proia, R.L.; Acharya, K.R.; Bigalke, H., et al. Identification of the protein receptor binding site of botulinum neurotoxins B and G proves the double-receptor concept. Proc Natl Acad Sci U S A 2007, 104, 359-364.
91. Blasi, J.; Chapman, E.R.; Link, E.; Binz, T.; Yamasaki, S.; De Camilli, P.; Sudhof, T.C.; Niemann, H.; Jahn, R. Botulinum neurotoxin A selectively cleaves the synaptic protein SNAP-25. Nature 1993, 365, 160-163.
92. Washbourne, P.; Pellizzari, R.; Baldini, G.; Wilson, M.C.; Montecucco, C. Botulinum neurotoxin types A and E require the SNARE motif in SNAP-25 for proteolysis. FEBS letters 1997, 418, 1-5.
93. Shone, C.C.; Quinn, C.P.; Wait, R.; Hallis, B.; Fooks, S.G.; Hambleton, P. Proteolytic cleavage of synthetic fragments of vesicle-associated membrane protein, isoform-2 by botulinum type B neurotoxin. Eur J Biochem 1993, 217, 965-971.
94. Schmidt, J.J.; Stafford, R.G.; Millard, C.B. High-throughput assays for botulinum neurotoxin proteolytic activity: serotypes A, B, D, and F. Anal Biochem 2001, 296, 130-137.
95. Kalb, S.R.; Baudys, J.; Egan, C.; Smith, T.J.; Smith, L.A.; Pirkle, J.L.; Barr, J.R. Different substrate recognition requirements for cleavage of synaptobrevin-2 by Clostridium baratii and Clostridium botulinum type F neurotoxins. Appl Environ Microbiol 2011, 77, 1301-1308.
96. Chen, S.; Barbieri, J.T. Unique substrate recognition by botulinum neurotoxins serotypes A and E. The Journal of biological chemistry 2006, 281, 10906-10911.
97. Chen, S.; Barbieri, J.T. Multiple pocket recognition of SNAP25 by botulinum neurotoxin serotype E. The Journal of biological chemistry 2007, 282, 25540-25547.
98. Jin, R.; Sikorra, S.; Stegmann, C.M.; Pich, A.; Binz, T.; Brunger, A.T. Structural and Biochemical Studies of Botulinum Neurotoxin Serotype C1 Light Chain Protease: Implications for Dual Substrate Specificity(,). Biochemistry 2007, 46, 10685-10693.
99. O'Sullivan, G.A.; Mohammed, N.; Foran, P.G.; Lawrence, G.W.; Oliver Dolly, J. Rescue of exocytosis in botulinum toxin A-poisoned chromaffin cells by expression of cleavage-resistant SNAP-25. Identification of the minimal essential C-terminal residues. The Journal of biological chemistry 1999, 274, 36897-36904.
100. Wang, D.; Krilich, J.; Baudys, J.; Barr, J.R.; Kalb, S.R. Enhanced detection of type C botulinum neurotoxin by the Endopep-MS assay through optimization of peptide substrates. Bioorg Med Chem 2015**.**
101. Shone, C.C.; Roberts, A.K. Peptide substrate specificity and properties of the zincendopeptidase activity of botulinum type B neurotoxin. Eur J Biochem 1994, 225, 263-270.
102. Sikorra, S.; Henke, T.; Galli, T.; Binz, T. Substrate Recognition Mechanism of VAMP/Synaptobrevin-cleaving Clostridial Neurotoxins. The Journal of biological chemistry 2008, 283, 21145-21152.
103. Chen, S.; Wan, H.Y. Molecular mechanisms of substrate recognition and specificity of botulinum neurotoxin serotype F. Biochem J 2011, 433, 277-284.
104. Yamamoto, H.; Ida, T.; Tsutsuki, H.; Mori, M.; Matsumoto, T.; Kohda, T.; Mukamoto, M.; Goshima, N.; Kozaki, S.; Ihara, H. Specificity of botulinum protease for human VAMP family proteins. Microbiol Immunol 2012, 56, 245-253.
105. Peng, L.; Adler, M.; Demogines, A.; Borrell, A.; Liu, H.; Tao, L.; Tepp, W.H.; Zhang, S.C.; Johnson, E.A.; Sawyer, S.L., et al. Widespread sequence variations in VAMP1 across vertebrates suggest a potential selective pressure from botulinum neurotoxins. PLoS Pathog 2014, 10, e1004177.
106. Hall, M.P.; Unch, J.; Binkowski, B.F.; Valley, M.P.; Butler, B.L.; Wood, M.G.; Otto, P.; Zimmerman, K.; Vidugiris, G.; Machleidt, T., et al. Engineered luciferase reporter from a deep sea shrimp utilizing a novel imidazopyrazinone substrate. ACS Chem Biol 2012, 7, 1848-1857.
107. Shaner, N.C.; Campbell, R.E.; Steinbach, P.A.; Giepmans, B.N.; Palmer, A.E.; Tsien, R.Y. Improved monomeric red, orange and yellow fluorescent proteins derived from Discosoma sp. red fluorescent protein. Nat Biotechnol 2004, 22, 1567-1572.
108. Inouye, S.; Watanabe, K.; Nakamura, H.; Shimomura, O. Secretional luciferase of the luminous shrimp Oplophorus gracilirostris: cDNA cloning of a novel imidazopyrazinone luciferase(1). FEBS letters 2000, 481, 19-25.
109. Matz, M.V.; Fradkov, A.F.; Labas, Y.A.; Savitsky, A.P.; Zaraisky, A.G.; Markelov, M.L.; Lukyanov, S.A. Fluorescent proteins from nonbioluminescent Anthozoa species. Nat Biotechnol 1999, 17, 969-973.
110. Peck MW, Smith TJ, Anniballi F, Austin JW, Bano L, Bradshaw M, Cuervo P, Cheng LW, Derman Y, Dorner BG, Fisher A, Hill KK, Kalb SR, Korkeala H, Lindström M, Lista F, Lúquez C, Mazuet C, Pirazzini M, Popoff MR, Rossetto O, Rummel A, Sesardic D, Singh BR, Stringer SC. Toxins (Basel). 2017 Jan 18;9(1).
111. Zhang S, Masuyer G, Zhang J, Shen Y, Lundin D, Henriksson L, Miyashita SI, Martínez-Carranza M, Dong M, Stenmark P. Identification and characterization of a novel botulinum neurotoxin. Nat Commun. 2017 Aug 3;8:14130.
112. Sikorra S, Henke T, Galli T, Binz T. Substrate recognition mechanism of VAMP/ Synaptobrevin-cleaving clostridial neurotoxins. The Journal of biological chemistry 2008, 283, 21145-21152.

## Claims

1. A method of detecting clostridial neurotoxin activity in a test sample and assigning the activity to an individual neurotoxin serotype,
said method comprising
(i) contacting a test sample suspected of comprising said neurotoxin with a peptide or polypeptide comprising SEQ ID NO: 5 or SEQ ID NO: 6 or SEQ ID NO: 45 or SEQ ID NO: 46 or SEQ ID NO: 47 or SEQ ID NO: 48 or SEQ ID NO: 49,
(ii) detection of the presence or amount of at least one peptide or polypeptide or of at least one proteolytic cleavage product of said peptide or polypeptide; and
(iii) concluding from the presence or amount of the peptide, polypeptide or cleavage product on the presence or amount of a specific neurotoxin serotype in the sample, wherein the detection of a cleavage product of the peptide or polypeptide comprising SEQ ID NO: 5 or SEQ ID NO: 6 or SEQ ID NO: 45 or SEQ ID NO: 46 or SEQ ID NO: 47 or SEQ ID NO: 48 or SEQ ID NO: 49 is indicative of catalytic activity of BoNT/D and serotype variants thereof.

2. The method of claim 1, wherein the peptide or polypeptide or cleavage product is coupled to a solid support.

3. The method of claim 1 or 2, wherein the peptide, polypeptide or cleavage product comprises a detectable label, wherein the detectable label is preferably a luciferase, a green, red, cyan, yellow fluorescent protein, GFP, mCherry, CFP, YFP, a peroxidase, a phosphatase, β-galactosidase, or a radioactive isotope.

4. The method of any one of claims 1 to 3, wherein the peptide, polypeptide or cleavage product comprises a tag for immobilization on a solid support.

5. The method of claim 4, wherein said tag is a strep-tag, a halo-tag (HA), a poly-his-tag, a poly-arginine-tag, a poly-HisAsn-tag, a HAT (natural histidine affinity)-tag, a S-tag, a flag-tag, a GST (glutathione-S-transferase)-tag, or a maltose-binding-protein-tag.

6. The method of any one of claims 1 to 4, wherein said presence or amount is determined from the detectable amount of cleavage product.

7. The method of any one of claims 1 to 6, wherein said method comprises comparing the amount of peptide, polypeptide or cleavage product detectable in step (ii) with the amount of peptide, polypeptide or cleavage product detectable in a reference sample.

8. A peptide or polypeptide comprising an amino acid sequence selected from the group consisting of SEQ ID NOs: 45-49.

9. The peptide or polypeptide of claim 8, comprising a tag for immobilization, preferably selected from the group consisting of a halo-tag (HA), strep-tag, a poly-his-tag, a poly-arginine-tag, a poly-HisAsn-tag, a HAT-tag, a S-tag, a flag-tag, a GST-tag, a maltose-binding -protein-tag.

10. The peptide or polypeptide of claim 8 or 9, comprising a detectable label, preferably selected from the group consisting of luciferase, a green, red, cyan, yellow fluorescent protein, preferably GFP, mCherry, CFP, or YFP, or a peroxidase, a phosphatase, β-galactosidase, or a radioactive isotope.

11. The peptide or polypeptide of any one of claims 8 to 10, comprising at least one additional cleavage site for a protease, said protease being selected from the group consisting of TEV (tobacco etch virus), HRV (human rhinovirus) 3C protease, Thrombin, Factor Xa, SUMO-protease (ULP1/2, Senpl-7).

12. Kit comprising the peptide or polypeptide of any one of claims 8 to 11.

## Patentansprüche

1. Verfahren zum Nachweis der Aktivität von Clostridien-Neurotoxin in einer Testprobe und zur Zuordnung der Aktivität zu einem individuellen Neurotoxin-Serotyp, wobei das Verfahren umfasst
(i) Inkontaktbringen einer Testprobe, von der vermutet wird, dass sie das Neurotoxin enthält, mit einem Peptid oder Polypeptid, das SEQ ID NO: 5 oder SEQ ID NO: 6 oder SEQ ID NO: 45 oder SEQ ID NO: 46 oder SEQ ID NO: 47 oder SEQ ID NO: 48 oder SEQ ID NO: 49 umfasst,
(ii) Nachweis der Anwesenheit oder der Menge mindestens eines Peptids oder Polypeptids oder mindestens eines proteolytischen Spaltprodukts des Peptids oder Polypeptids; und
(iii) Rückschluss von der Anwesenheit oder Menge des Peptids, Polypeptids oder Spaltprodukts auf die Anwesenheit oder Menge eines spezifischen Neurotoxin-Serotyps in der Probe, wobei der Nachweis eines Spaltprodukts des Peptids oder Polypeptids, das SEQ ID NO: 5 oder SEQ ID NO: 6 oder SEQ ID NO: 45 oder SEQ ID NO: 46 oder SEQ ID NO: 47 oder SEQ ID NO: 48 oder SEQ ID NO: 49 umfasst, auf die katalytische Aktivität von BoNT/D und dessen Serotypvarianten hinweist.

2. Verfahren nach Anspruch 1, wobei das Peptid oder Polypeptid oder Spaltprodukt an einen festen Träger gekoppelt ist.

3. Verfahren nach Anspruch 1 oder 2, wobei das Peptid, Polypeptid oder Spaltprodukt eine nachweisbare Markierung umfasst, wobei die nachweisbare Markierung vorzugsweise eine Luziferase, ein grün, rot, cyan, gelb fluoreszierendes Protein, GFP, mCherry, CFP, YFP, eine Peroxidase, eine Phosphatase, β-Galactosidase oder ein radioaktives Isotop ist.

4. Verfahren nach einem der Ansprüche 1 bis 3, wobei das Peptid, Polypeptid oder Spaltprodukt einen Tag zur Immobilisierung auf einem festen Träger umfasst.

5. Verfahren nach Anspruch 4, wobei der Tag ein Strep-Tag, ein Halo-Tag (HA), ein Poly-His-Tag, ein Poly-Arginin-Tag, ein Poly-HisAsn-Tag, ein HAT (natürlicher Histidin-Affinitäts)-Tag, ein S-Tag, ein Flag-Tag, ein GST (Glutathion-S-Transferase)-Tag oder ein Maltose-bindendes Protein-Tag ist.

6. Verfahren nach einem der Ansprüche 1 bis 4, wobei die Anwesenheit oder die Menge anhand der nachweisbaren Menge des Spaltprodukts bestimmt wird.

7. Verfahren nach einem der Ansprüche 1 bis 6, wobei das Verfahren den Vergleich der in Schritt (ii) nachweisbaren Menge an Peptid, Polypeptid oder Spaltprodukt mit der in einer Referenzprobe nachweisbaren Menge an Peptid, Polypeptid oder Spaltprodukt umfasst.

8. Peptid oder Polypeptid, umfassend eine Aminosäuresequenz, ausgewählt aus der Gruppe bestehend aus SEQ ID NOs: 45-49.

9. Peptid oder Polypeptid nach Anspruch 8, umfassend einen Tag zur Immobilisierung, vorzugsweise ausgewählt aus der Gruppe bestehend aus einem Halo-Tag (HA), Strep-Tag, einem Poly-His-Tag, einem Poly-Arginin-Tag, einem Poly-HisAsn-Tag, einem HAT-Tag, einem S-Tag, einem Flag-Tag, einem GST-Tag, einem Maltose-bindenden-Protein-Tag.

10. Peptid oder Polypeptid nach Anspruch 8 oder 9, umfassend eine nachweisbare Markierung, vorzugsweise ausgewählt aus der Gruppe bestehend aus Luziferase, einem grün, rot, cyan, gelb fluoreszierenden Protein, vorzugsweise GFP, mCherry, CFP oder YFP, oder einer Peroxidase, einer Phosphatase, β-Galactosidase oder einem radioaktiven Isotop.

11. Peptid oder Polypeptid nach einem der Ansprüche 8 bis 10, umfassend mindestens eine zusätzliche Schnittstelle für eine Protease, wobei die Protease ausgewählt ist aus der Gruppe bestehend aus TEV (tobacco etch virus), HRV (human rhinovirus) 3C Protease, Thrombin, Faktor Xa, SUMO-Protease (ULP1/2, Senpl-7).

12. Kit, umfassend das Peptid oder Polypeptid nach einem der Ansprüche 8 bis 11.

## Revendications

1. Procédé de détection de l'activité d'une neurotoxine clostridiale dans un échantillon d'essai et d'attribution de l'activité à un sérotype de neurotoxine individuel,
ledit procédé comprenant
(i) la mise en contact d'un échantillon d'essai suspecté de contenir ladite neurotoxine avec un peptide ou un polypeptide comprenant la SEQ ID NO : 5 ou la SEQ ID NO : 6 ou la SEQ ID NO : 45 ou la SEQ ID NO : 46 ou la SEQ ID NO : 47 ou la SEQ ID NO : 48 ou la SEQ ID NO : 49,
(ii) la détection de la présence ou d'une quantité d'au moins un peptide ou polypeptide ou d'au moins un produit de clivage protéolytique dudit peptide ou polypeptide ; et
(iii) la détermination, à partir de la présence ou de la quantité du peptide, du polypeptide ou du produit de clivage, de la présence ou d'une quantité d'un sérotype de neurotoxine spécifique dans l'échantillon,
dans lequel la détection d'un produit de clivage du peptide ou du polypeptide comprenant la SEQ ID NO : 5 ou la SEQ ID NO : 6 ou la SEQ ID NO : 45 ou la SEQ ID NO : 46 ou la SEQ ID NO : 47 ou la SEQ ID NO : 48 ou la SEQ ID NO : 49 indique une activité catalytique de la BoNT/D et de ses variants sérotypiques.

2. Procédé selon la revendication 1, dans lequel le peptide, le polypeptide ou le produit de clivage est couplé à un support solide.

3. Procédé selon la revendication 1 ou 2, dans lequel le peptide, le polypeptide ou le produit de clivage comprend un marqueur détectable, dans lequel le marqueur détectable est de préférence une luciférase, une protéine fluorescente verte, rouge, cyan ou jaune, une protéine GFP, une protéine mCherry, une protéine CFP, une protéine YFP, une peroxydase, une phosphatase, une β-galactosidase, ou un isotope radioactif.

4. Procédé selon l'une quelconque des revendications 1 à 3, dans lequel le peptide, le polypeptide ou le produit de clivage comprend une étiquette pour une immobilisation sur un support solide.

5. Procédé selon la revendication 4, dans lequel ladite étiquette est une étiquette Strep, une étiquette Halo (HA), une étiquette Poly-his, une étiquette Poly-arginine, une étiquette Poly-HisAsn, une étiquette HAT (affinité naturelle pour l'histidine), une étiquette S, une étiquette Flag, une étiquette GST (glutathione-S-transférase) ou une étiquette de protéine de liaison au maltose.

6. Procédé selon l'une quelconque des revendications 1 à 4, dans lequel ladite présence ou la quantité est déterminée à partir de la quantité détectable de produit de clivage.

7. Procédé selon l'une quelconque des revendications 1 à 6, ledit procédé comprenant la comparaison entre la quantité de peptide, de polypeptide ou de produit de clivage détectable à l'étape (ii) et la quantité de peptide, de polypeptide ou de produit de clivage détectable dans un échantillon de référence.

8. Peptide ou polypeptide comprenant une séquence d'acides aminés choisie dans le groupe constitué des SEQ ID NO : 45 à 49.

9. Peptide ou polypeptide selon la revendication 8, comprenant une étiquette pour une immobilisation, de préférence choisie dans le groupe constitué d'une étiquette Halo (HA), d'une étiquette Strep, d'une étiquette Poly-his, d'une étiquette Poly-arginine, d'une étiquette Poly-HisAsn, d'une étiquette HAT, d'une étiquette S, d'une étiquette Flag, d'une étiquette GST, ou d'une étiquette de protéine de liaison au maltose.

10. Peptide ou polypeptide selon la revendication 8 ou 9, comprenant un marqueur détectable, de préférence choisi dans le groupe constitué par une luciférase, une protéine fluorescente verte, rouge, cyan ou jaune, de préférence une protéine GFP, une protéine mCherry, une protéine CFP, ou une protéine YFP, ou une peroxydase, une phosphatase, une β-galactosidase, ou un isotope radioactif.

11. Peptide ou polypeptide selon l'une quelconque des revendications 8 à 10, comprenant au moins un site de clivage supplémentaire pour une protéase, ladite protéase étant choisie dans le groupe constitué du TEV (virus de la gravure du tabac), de la protéase 3C du HRV (rhinovirus humain), de la thrombine, du facteur Xa et de la protéase SUMO (ULP1/2, Senp1-7).

12. Kit comprenant le peptide ou le polypeptide selon l'une quelconque des revendications 8 à 11.
